(19) Europäisches Patentamt
European Patent Office
Office européen des brevets

(11) Publication number: **0 000 636**
**A1**

# (12) EUROPEAN PATENT APPLICATION

(21) Application number: 78300148.0

(22) Date of filing: 12.07.78

(51) Int. Cl.[2]: C 07 D 499/00, C 07 D 205/08, C 07 D 417/12, A 61 K 31/43

(30) Priority: 13.07.77 GB 29487/77
08.03.78 GB 9280/78

(43) Date of publication of application: 07.02.79 Bulletin 79/3

(84) Designated contracting states: BE CH DE FR GB NL SE

(71) Applicant: Glaxo GROUP LIMITED
Clarges House 6-12 Clarges Street
London W1Y 8DH. (GB)

(72) Inventor: Beels, Christopher Michael Donald
24 Fryent Way Kingsbury
London. (GB)

(72) Inventor: Cocker, John Derek
Pednolva Nortoft Road Chalfort St Peter
Buckinghamshire. (GB)

(72) Inventor: Ramsay, Michael Vincent John
157 Kings Road South Harrow
Middlesex. (GB)

(72) Inventor: Watson, Nigel Stephen
5 Ruislip Close Greenford
Middlesex. (GB)

(74) Representative: Holmes, Michael John et al
Frank B. Dehn & Co.
Imperial House 15-19 Kingsway
London WC2B 6UZ. (GB)

(54) **Penem compounds, processes for their preparation, their use in pharmaceutical compositions and azetidinones used in their preparation.**

(57) Compounds of the formula

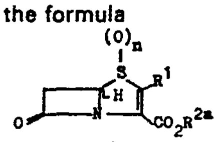

(I)

(wherein $R^1$ represents an alkyl group containing from 1 to 8 carbon atoms, optionally substituted by a hydroxy, etherified hydroxy or acylated hydroxy group; $R^{2a}$ represents a hydrogen atom or a carboxyl esterifying group; n represents 0 or 1 and salts thereof and processes for their preparation. The compounds of formula (I) are useful as antibiotics or as intermediates for the preparation or purification of antibiotics.

Intermediates of the formula

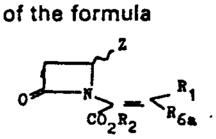

wherein Z represents a halogen atom or a group $(S)_nR_9$ or $-N R_xR_yR_z.A^{\ominus}$ are also described.

EP 0 000 636 A1

TITLE MODIFIED
see front page



Penem compounds, processes for their preparation, their use in pharmaceutical compositions and azetidinones useful in their preparation

This invention relates to novel penems having antibiotic activity, to processes for their preparation, to pharmaceutical compositions containing them and to intermediates of use in the preparation of antibiotics.

According to one feature of the present invention there are provided compounds of general formula

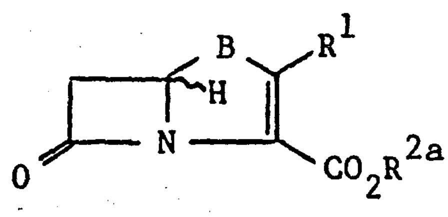

(I)

(wherein $R^1$ represents a $C_{1-8}$ alkyl group optionally substituted by a hydroxy, etherified hydroxy or acylated hydroxy group; $R^{2a}$ represents a carboxyl esterifying group or a hydrogen atom; and B represents a group >S or S→O) and salts thereof.

Compounds according to the invention may be of use as antibiotics as detailed below or may be useful as intermediates in the preparation of further active compounds or in the purification of active compounds.

The compounds according to the invention may exist as individual 5-position isomers or as mixtures thereof.

Furthermore, other chiral centres may exist in the molecule and the invention extends to all isomers of the compounds individually or in admixture.

B preferably represents the group >S.

$R^1$ may, for example, be a methyl, ethyl, n-propyl, isopropyl or butyl group; such groups may optionally be substituted as set out in more detail below.

Etherified hydroxyl substituents which may be present in $R^1$ include, for example, groups $-OR^3$ where $R^3$ is an unsubstituted or substituted hydrocarbyl group, e.g. an aliphatic, araliphatic or aromatic group or a C-attached heterocyclic group, or a silyl group. Thus, for example $R^3$ may be an unsubstituted alkyl, alkenyl or alkynyl group which may contain 1-6 carbon atoms, or such an alkyl group carrying a substituted hydroxy group; a hydroxyalkyl group having 2-6 carbon atoms; an aralkyl group which may have 1-6 carbon atoms in the alkyl portion or an aryl group, such aryl and aralkyl groups preferably being monocyclic; a cycloalkyl group, which may have 3-7 carbon atoms; a carbon-attached saturated or unsaturated 5-7 membered heterocyclic ring containing for example, an oxygen atom; or a silyl group having up to 24 carbon atoms which may carry three groups which may be the same or different selected from alkyl, alkenyl, cycloalkyl, aralkyl and aryl groups. In such silyl groups, substituents will preferably be $C_{1-4}$ alkyl groups e.g. methyl, ethyl, n-propyl, iso-propyl or <u>t</u>-butyl groups.

Substituted hydroxy groups as referred to above include acylated and etherified hydroxy groups. In

general, acylated hydroxy groups may have the formula $R^aCO_2$ where $R^a$ is a hydrocarbyl group as defined for $R^3$, relatively simple $R^a$ groups such as $C_{1-4}$ alkyl, e.g. methyl, being preferred, while etherified hydroxy groups may have the formula $R^bO$, where $R^b$ has one of the meanings given for $R^a$, simple groups such as $C_{1-4}$ alkyl, e.g. ethyl, being preferred.

Representative groups $R^3$ include methyl, ethyl, propyl, isopropyl, butyl, allyl, propargyl, hydroxyethyl, ethoxyethyl, phenyl, benzyl, phenethyl, cyclohexyl, tetrahydropyranyl and t-butyldimethylsilyl.

Suitable acylated hydroxyl substituents include, for example, groups $-OR^4$ wherein $R^4$ preferably represents an acyl group $R^c\overset{Y}{\overset{\|}{C}}-$ (wherein Y is oxygen or sulphur; and $R^c$ is a hydrogen atom, an aliphatic, araliphatic or aromatic group or a C-attached heterocyclic group, for example $C_{1-8}$ alkyl, $C_{2-8}$ alkenyl, $C_{2-8}$ alkynyl, $C_{3-12}$ cycloalkyl, $C_{4-15}$ aryl—$C_{1-6}$ alkyl or $C_{4-15}$ aryl group, which may be substituted; or an amino or mono- or di-substituted amino group). Suitable substituents on the group $R^c$ include, for example, halogen, e.g. fluorine, chlorine or bromine; hydroxy; etherified hydroxy; acyloxy; cyano; nitro; azide; carboxyl or esterified carboxyl; or amino or substituted amino.

Where in the compounds of formula (I) $R^1$ carries a carbamoyloxy group as the acylated hydroxy substituent this may, for example, be represented as $-O\overset{Y}{\overset{\|}{C}}NR^dR^e$, where Y is oxygen or sulphur and $R^d$ and $R^e$, which may be the

same or different, are hydrogen; $C_{1-5}$ alkyl; aralkyl e.g. benzyl; aryl e.g. phenyl; or a C-attached heterocyclic group e.g. tetrahydropyranyl, or $R^d$ and $R^e$ may together with the nitrogen atom to which they are attached form a heterocyclic ring preferably having 5-7 ring members, which may optionally contain another heteroatom, e.g. a nitrogen, oxygen or sulphur atom. Such groups $R^d$ and $R^e$ may themselves be substituted. Thus, the acylated hydroxy substituent $-OR^4$ may, for example, be represented as $-O\overset{Y}{\overset{\|}{C}}NHR^f$ where Y is oxygen or sulphur and $R^f$ represents a protecting group, for example an acetyl, trichloroacetyl, chlorosulphonyl or trimethylsilyl group.

Preferred groups of the formula $R^cCO$-are those wherein $R^c$ represents a hydrogen atom; a substituted or unsubstituted lower alkyl group, e.g. $C_{1-4}$, for example methyl, chloromethyl or ethyl groups; a substituted or unsubstituted aralkyl group e.g. benzyl; an aryl group e.g. phenyl; or an amino, methylamino or anilino group. Suitable substituents are defined above.

Such groups $R^3$ and $R^4$ may serve as protecting groups for the hydroxyl group during the intermediate stages of the synthesis of the compounds of formula (I). The protecting group may if desired, be removed, for example by solvolysis, e.g. by mild acid or base hydrolysis, to yield the compounds of formula (I) in which $R^1$ carries a free hydroxyl group. Examples of such protecting groups include those in which $R^3$ is a tetrahydropyranyl, 1-ethoxyethyl or silyl group or $R^4$ is a formyl or dichloroacetyl group.

When $R^{2a}$ represents a carboxyl esterifying group, it may for example be an organic group derived from an alcohol (aliphatic or araliphatic), a phenol, a silanol or a stannanol. Such an alcohol, phenol, silanol or stannanol used to esterify the carboxyl group preferably contains not more than 24 carbon atoms.

Thus, the group $R^{2a}$ may represent a straight or branched unsubstituted or substituted alkyl or alkenyl group, preferably having from 1-8 carbon atoms, for example a methyl, ethyl, propyl, isopropyl, butyl, sec-butyl, t-butyl or allyl group, optional substituents being for example, alkoxy e.g. methoxy; halogen i.e. fluorine, chlorine, bromine or iodine; cyano; acyloxy, e.g. alkanoyloxy, such as acetoxy or pivaloyloxy, or alkoxycarbonyloxy, such as ethoxycarbonyloxy; acyl e.g. p-bromobenzoyl and alkoxycarbonyl e.g. ethoxycarbonyl;

an aralkyl group having up to 20 carbon atoms especially an arylmethyl group e.g. a benzyl or substituted benzyl group, suitable substituents being halo e.g. chloro; nitro, e.g. o or p-nitro; cyano; alkoxy e.g. p-methoxy or alkyl e.g. p-methyl groups; a diphenylmethyl or triphenylmethyl group or a fur-2-ylmethyl, thien-2-ylmethyl or pyrid-4-ylmethyl group, the heterocyclic groups of which may also be substituted e.g. by a $C_{1-4}$ alkyl group, preferably methyl;

an aryl group having up to 12 carbon atoms e.g. a phenyl or substituted phenyl group, suitable substituents being halo e.g. chloro; nitro, e.g. o or p-nitro; cyano; alkoxy e.g. p-methoxy or alkyl e.g. p-methyl groups;

a cycloalkyl group containing not more than 12 carbon atoms e.g. adamantyl, cyclohexyl or cyclopentyl;

a heterocyclic group containing not more than 12 carbon atoms, the heteroatom being, for example, oxygen, as in the tetrahydropyranyl or phthalidyl group;

a stannyl group having up to 24 carbon atoms for example a stannyl group carrying three substituents which may be the same or different selected from alkyl, alkenyl, aryl, aralkyl, cycloalkyl, alkoxy, or aralkoxy groups. Such groups include methyl, ethyl, propyl, n-butyl, phenyl and benzyl groups;

or a silyl group having up to 24 carbon atoms which may carry three groups which may be the same or different selected from alkyl, alkenyl, cycloalkyl, aralkyl and aryl groups. Such groups will preferably be $C_{1-4}$ alkyl e.g. methyl, ethyl, n-propyl, iso-propyl or t-butyl groups.

Salts of compounds of formula (I) include, for example, salts of acidic compounds of the invention formed with inorganic and organic bases. Such salts include alkali metal salts, e.g. sodium, potassium and lithium salts, alkaline earth metal salts, e.g. calcium and magnesium salts, ammonium salts, and substituted ammonium salts. Salts of compounds of formula (I) also include acid addition salts of compounds of formula (I) in which the $R^1$ group contains a basic nitrogen atom. Compounds of formula (I) in which $R^{2a}$ is hydrogen and $R^1$ carries a basic nitrogen atom may exist in zwitterionic forms and such zwitterionic forms are included within the present invention.

In general acids and metabolically labile esters of compounds of formula I in which B is sulphur, and

salts of such compounds are the preferred forms for use in medicine. However, other compounds of the invention are also useful, particularly for the preparation and purification of the preferred compounds just referred to and other compounds. Thus for example, where the ester grouping is readily cleaved e.g. by hydrolysis or hydrogenolysis, without significant degradation of the rest of the molecule, the esters are especially useful as carboxyl-protected derivatives of the parent compounds. Those esters which are readily cleaved and are primarily of use in this connection include arylmethyl esters, especially the benzyl, p-nitrobenzyl, benzhydryl and trityl esters as well as the stannyl, e.g. tri-n-butylstannyl, and silyl esters. As indicated above, the ester grouping may be metabolically labile, i.e. it may be converted into the carboxylic acid during general metabolic processes, e.g. in the blood or liver. Metabolically labile esters include for example acyloxymethyl, e.g. acetoxymethyl and pivaloyloxymethyl, esters and phthalidyl esters.

Compounds of formula (I) wherein B is sulphur and $R^{2a}$ represents a hydrogen atom or a metabolically labile ester grouping, together with salts of such compounds, in general possess antibacterial activity. They have been found to be active against a range of gram-positive and gram-negative microorganisms, for example against strains of Staphylococcus aureus, Micrococcus species, Escherichia coli, Salmonella typhimurium, Shigella sonnei, Enterobacter cloacae, Klebsiella aerogenes, Proteus mirabilis, Proteus vulgaris,

Streptococcus faecalis, Serratia marcescens, Providence species and Haemophilus influenzae.

In general, the compounds are stable to the action of β-lactamases produced by pathogenic gram-positive organisms for example those produced by Staphylococcus aureus, and to β-lactamases produced by gram-negative organisms such as Enterobacter cloacae.

The compounds have the ability to inhibit β-lactamase enzymes; these include enzymes produced by gram-negative organisms, for example, those produced by strains of Enterobacter cloacae.

Certain of the compounds prepared according to the invention, including 2-methyl-, 2-ethyl-, 2-(2-methoxyethyl)- and 2-(2-acetoxyethyl)-pen-2-em-3-carboxylic acids and their salts, have shown good absorption properties in mice after administration by mouth.

In particular, 2-ethyl-, 2-n-propyl-, 2-(2-carbamoyloxyethyl)-, 2-methoxymethyl- and 2-(2-meth~~yl~~oxy-ethyl)-pen-2-em-3-carboxylic acids and their salts have shown noteworthy antibacterial activity.

Compounds of formula (I) wherein B is S and $R^{2a}$ represe a hydrogen atom or a metabolically labile ester grouping and physiologically acceptable salts ~~of compounds~~ of such compounds may thus be formulated as pharmaceutical (including veterinary) compositions which may if desired contain further β-lactam antibiotics. Such compositions will normally also contain carriers and/or excipients and may, for example, be in a form adapted for oral or parenteral administration.

Such compositions may thus, for example, take the

form of powders, tablets, capsules, lozenges, solutions and syrups suitable for oral administration, and may include, for example, starch, lactose, talc, magnesium stearate, gelatin, distilled water and suspending, dispersing, emulsifying, flavouring or colouring agents.

Active compounds of formula (I) may further be formulated in rectal compositions such as suppositories or retention enemas.

For parenteral administration, the compounds may be formulated in ampoules for reconstitution before use.

The active compounds of formula (I) will generally be administered to humans at a total daily dosage level of 50 mg to 20 g, preferably from 100 mg to 10 g, which may be in divided doses given 1-4 times per day. Dosage units will in general contain 12.5 mg to 5 g, preferably 50 mg to 1 g of active compound according to the invention.

Active compounds of formula (I) may be of use in treating a variety of diseases, in humans and animals, caused by pathogenic bacteria, such as respiratory tract or urinary tract infections.

The compounds of general formula (I) wherein B represents a group >S may, for example, be prepared by reacting a compound of the formula (II)

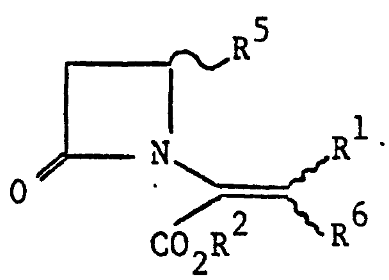

(II)

[wherein

$R^1$ is as defined above;

$R^2$ represents a carboxyl esterifying group as defined above for $R^{2a}$;

$R^5$ represents a halogen atom or a group of formula $-\overset{\oplus}{N}R^xR^yR^z$ (in which $R^x$, $R^y$ and $R^z$, which may be the same or different, each represents an aliphatic, araliphatic or aromatic group or two of $R^x$, $R^y$ and $R^z$ together with the nitrogen atom to which they are attached represent a five-, six- or seven-membered heterocyclic ring optionally containing a further heteroatom and the third of $R^x$, $R^y$ and $R^z$ is as defined above or $R^x$, $R^y$ and $R^z$ together with the nitrogen atom to which they are attached form a polycyclic heterocyclic ring system or an aromatic heterocyclic ring); and $R^6$ represents a leaving group, for example a halogen atom or an acylated or etherified hydroxy group] with hydrogen sulphide or with a hydrosulphide or sulphide salt.

If desired, a compound of formula (I) in which B represents a sulphur atom obtained by the above reaction may subsequently be oxidised, as discussed in more detail hereinafter, to yield a compound of formula (I) in which B represents a group $>S\rightarrow O$. Other optional subsequent reaction steps include deesterifying a compound of formula (I) in which $R^{2a}$ is a carboxyl esterifying group whereby a compound of formula (I) in which $R^{2a}$ represents a hydrogen atom is obtained; reaction of a free acid of formula (I) with a base or reaction of a compound of formula (I) wherein the $R^1$ group contains a basic nitrogen atom with an acid to yield a salt; and/or

modification of the $R^1$ group: all of which optional steps are discussed in more detail hereinafter.

Suitable hydrosulphide and sulphide salts include, for example, alkali metal, e.g. sodium, potassium and lithium salts; alkaline earth metal, e.g. calcium, salts; and onium salts, for example quaternary ammonium salts wherein the quaternary ammonium ion has the formula

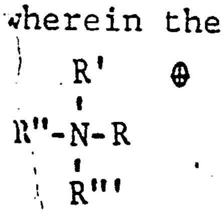

where R, R', R" and R"' which may be the same or different each represents an alkyl or aralkyl group. Examples of such salts include the benzyltrimethylammonium and tetra-n-butylammonium salts.

The presence of a base, either organic or inorganic, may be desirable to accelerate the reaction, to optimise yields and to curtail excessive acidity. It is in general preferred to use as weak a base as possible which is effective in the reaction. Suitable bases for use in the reaction include amine bases, for example, tertiary amine bases, e.g. trialkylamines, preferably those wherein the alkyl groups have from 1 to 4 carbon atoms such as triethylamine, hindered bases such as 3-azabicyclo-[3,2,2]-nonane or aromatic bases such as pyridine.

Where $R^5$ represents a halogen atom, the reaction will in general be effected at a temperature of from -150°C to +50°C, reaction temperatures of from -50°C to +10°C, particularly -15°C to +10°C, being preferred.

The reaction is desirably effected in the presence of an inert solvent, stable to the reaction conditions employed. Suitable solvents include, for example, cyclic ethers e.g. tetrahydrofuran and dioxan, halogenated

hydrocarbons, e.g. methylene chloride, esters, e.g. ethyl acetate and amide solvents, e.g. dimethylformamide. Alternatively, where the reaction is effected in the presence of a base, an excess of base may serve as solvent.

Where $R^5$ represents a group of formula $-\overset{\oplus}{N}R^xR^yR^z$, the reaction with hydrogen sulphide or a hydrosulphide or sulphide salt is preferably carried out at temperatures of from -30° to +30°C. The reaction is completed by cyclisation to form a penem of formula (I) by heating. The cyclisation reaction is preferably effected above +40°C, e.g. at +50° to +100°C.

Following the reaction with the compound of formula (II) wherein $R^5$ represents a group of formula $-\overset{\oplus}{N}R^xR^yR^z$ with the hydrogen sulphide or hydrosulphide or sulphide salt, it may be possible to isolate a thioenolate of the formula (IIa)

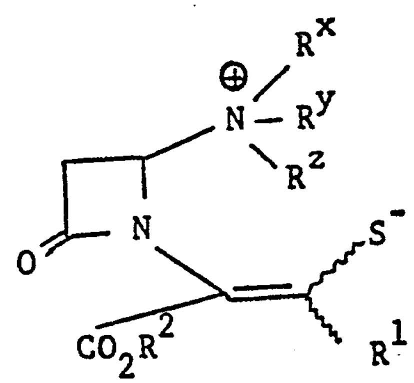

(IIa)

(wherein $R^x$, $R^y$, $R^z$, $R^1$ and $R^2$ have the above meanings). On heating, e.g. in a solvent such as ethyl acetate, the compound of formula (IIa) cyclises to the desired penem of formula (I).

Cyclisation may if desired be carried out in the presence of an acid scavenging agent, e.g. an insoluble inorganic base such as solid, anhydrous sodium, potassium or calcium carbonate.

In the compound of formula (II), when $R^5$ represents a halogen atom it is preferably a chlorine or bromine atom.

In the case that $R^5$ represents a group of formula $-\overset{\oplus}{N}R^xR^yR^z$ there will be an anion $A^{\ominus}$ associated with the compound of formula (II). In general the anion $A^{\ominus}$ will be derived from the reagent used for the introduction of the group $R^6$ e.g. a halide ion such as a chloride ion, as described hereinafter.

Where $R^5$ represents a group of formula $-\overset{\oplus}{N}R^xR^yR^z$, $R^x$, $R^y$ and $R^z$ may, for example each represent an alkyl group having up to 8 carbon atoms, e.g. a $C_{1-6}$ alkyl group preferably a $C_{1-4}$ group, an aralkyl group having up to 6 carbon atoms in the alkyl portion or an aryl group, such aryl and aralkyl groups desirably being monocyclic, e.g. benzyl and phenyl, and such alkyl groups including also cycloaliphatic e.g. $C_{3-7}$ cycloalkyl groups; or two of $R^x$, $R^y$ and $R^z$ may together with the nitrogen atom to which they are attached represent a five-, six- or seven-membered heterocyclic ring optionally containing a further oxygen or sulphur atom e.g. a N-alkyl-piperidinium or N-alkyl-morpholinium group; or $R^x$, $R^y$ and $R^z$ may, together with the nitrogen atom to which they are attached, represent a bicylic, non-aromatic heterocyclic ring system e.g. a quinuclidinium group, or an aromatic ring e.g. pyridinium. A triethylammonium group is particularly preferred.

$R^6$ is preferably the residue of an acid $R^6H$ having

a $pK_a$ (in water at 25°C) of 3.5 or less.

Where $R^6$ represents an acylated or etherified hydroxy group this may, for example, be an aliphatic, cycloaliphatic, aromatic or araliphatic carbonyloxy, sulphonyloxy or phosphinyloxy group containing 1 to 20 carbon atoms or an aliphatic ether group containing 1 to 6 carbon atoms, e.g. methoxy. Suitable sulphonyloxy groups include, for example, those wherein the aliphatic or aromatic grouping is an alkyl (e.g. $C_{1-8}$) group, which may be substituted by a halogen atom e.g. fluorine or chlorine, or an aryl (e.g. $C_{6-15}$) group which may carry alkyl, e.g. methyl, alkoxy e.g. methoxy or halogen e.g. bromine substituents. Suitable phosphinyloxy groups include, for example, groups of formula

$$\begin{array}{l} \quad O \\ \quad \| \\ -OP-R^7 \\ \quad | \\ \quad R^8 \end{array}$$

where $R^7$ and $R^8$, which may be the same or different, may each represent an alkyl, aryl or aralkyl group or may together with the phosphorus atom form a 5- or 6-membered ring. Alternatively $R^7$ and/or $R^8$ may represent groups $OR^g$ and $OR^h$ respectively where $R^g$ and $R^h$ are as defined for $R^7$ and $R^8$ or may together form a divalent 5- or 6-membered ring. An example of the latter type of phosphinyloxy group is the group

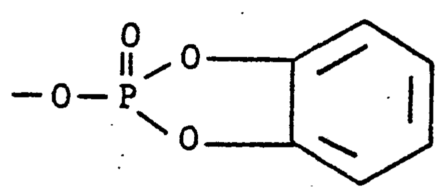

Suitable carbonyloxy groups include, for example, those wherein the aliphatic or aromatic grouping is an

alkyl (e.g. $C_{1-8}$) group optionally substituted by one or more halogen atoms or an aryl (e.g. $C_{6-15}$) group optionally substituted by, for example, one or more halogen atoms or nitro groups.

When $R^6$ represents a halogen atom, this is preferably a chlorine or bromine atom.

Preferred meanings for $R^6$ include methanesulphonyloxy, trifluoromethanesulphonyloxy, p-toluenesulphonyloxy, benzenesulphonyloxy, dimethoxyphosphinyloxy and chlorine.

The initial product of the reaction between the compound of formula (II) and hydrogen sulphide or a hydrosulphide or sulphide salt is an ester of formula (I) wherein B represents a group >S. Where a free acid of formula (I) or a salt of such an acid is required, the ester should subsequently be cleaved. Where an ester of a compound of formula (I) is required different from that originally produced, this may be obtained by deesterification followed by reesterification as described hereinbelow.

Suitable methods for deesterification are well known in the art. Thus, for example, a silyl or stannyl ester may be cleaved by mild solvolysis e.g. by reaction with water, alcohols, phenols or carboxylic acids e.g. acetic acid. Alternatively, esters which are readily subject to reduction, for example, arylmethyl esters such as benzyl, benzhydryl, trityl or more preferably, p-nitrobenzyl esters can be cleaved by reduction e.g. by hydrogenolysis, for example using a metal catalyst, e.g. a noble metal such as platinum, palladium or rhodium. The catalyst may be

supported e.g. on charcoal or kieselguhr.

In the case of p-nitrobenzyl esters, cleavage may also be effected by reduction of the nitro group (e.g. using a dissolving metal reducing agent such as zinc in acetic acid or zinc in aqueous tetrahydrofuran or acetone controlled in the pH range 3-6, preferably 4-5.5 by the addition of aqueous HCl; aluminium amalgam in a moist ether, e.g. tetrahydrofuran; or iron and ammonium chloride in an aqueous ether such as aqueous tetrahydrofuran) followed by hydrolysis either under the reduction conditions or by subsequent treatment with acid. Alternatively, a p-nitrobenzyl ester may be cleaved by alkaline hydrolysis using, for example, sodium sulphide in a solvent such as aqueous tetrahydrofuran, dimethylformamide or acetone.

Where it is desired to produce a salt of the compound of formula (I), an acid may be reacted, e.g. in an appropriate organic solvent with an appropriate base, preferably in equimolar quantities and preferably under conditions favouring precipitation of the salt. In the formation of alkali metal salts for example, e.g. sodium or potassium salts, an alkanoate is a preferred base e.g. a 2-ethyl hexanoate.

Where the compound of formula (I) obtained is a compound in which the $R^1$ group contains a basic nitrogen atom, it may, if desired, subsequently be converted into an acid addition salt by reaction with an appropriate acid. Examples of acid addition salts are those formed with hydrochloric, hydrobromic, sulphuric, phosphoric, lactic, citric, fumaric and maleic acids.

Sulphoxides of formula (I) according to the

invention may be prepared from the corresponding sulphides of formula (I) by, for example, oxidation with peracids such as peracetic, monoperphthalic, m-chloroperbenzoic or metaperiodic acid. Oxidation is preferably effected at a temperature not greater than 10°C and avoiding excess oxidising agent.

Esters of the acid of formula (I) may, for example, be prepared by reaction with an alcohol, phenol, silanol or stannanol or a reactive derivative thereof to form the desired ester. Reaction will desirably be effected under mild conditions in order to prevent rupture of the bicyclic nucleus. The use of neutral or mild acidic or basic conditions therefore, at temperatures between -70° and +35°C is preferred.

The alkyl, alkoxyalkyl and aralkyl esters may be prepared by reaction of the acid of formula (I) with the appropriate diazoalkane or diazoaralkane e.g. diazomethane or diphenyldiazomethane. The reaction will generally be effected in an ether, ester or a halohydrocarbon solvent, e.g. diethyl ether, ethyl acetate or dichloromethane. In general, reduced temperatures are preferred, for example, -15° to +15°C.

The esters derived from alcohols may also be produced by reaction of a reactive derivative of the alcohol, for example, a halide such as the chloride, bromide or iodide or a hydrocarbonsulphonyl derivative such as methanesulphonyl or p-toluenesulphonyl ester, with a salt of the acid of formula (I) e.g. an alkali or alkaline earth metal salt such as a lithium, sodium, potassium, calcium or barium salt or an amine salt,

such as a triethylammonium salt. This reaction is preferably carried out in a substituted sulphoxide or amide solvent e.g. dimethyl sulphoxide, dimethylformamide or hexamethylphosphoramide or alternatively, the esters may be prepared by reaction of the acid with the alcohol in the presence of a condensing agent such as dicyclohexylcarbodiimide.

Stannyl esters may conveniently be formed by reaction of the carboxylic acid of formula (I) or a salt thereof with reactive tetravalent tin moieties. Trialkyl tin oxides are preferred for the synthesis of tin compounds in view of their availability and low toxicity.

Silyl esters of the acid of formula (I) may be formed by reaction with a reactive tetravalent silicon moiety. Trialkylsilyl halides and mono- or bis-silylated acetamides are preferred as silylating agents. Proton acceptors e.g. weak bases such as pyridine may often be used with advantage when hydrogen halides are formed during such esterification.

Compounds of formula (I) wherein $R^1$ carries an etherified hydroxy group may be prepared by etherification of a compound of formula (I) in which $R^1$ carries a hydroxy group. Etherification may be effected, for example, by reaction of the appropriate hydroxy ester with an alcohol or phenol $R^3OH$ (in which $R^3$ is a hydrocarbyl or a C- attached heterocyclic group as defined above) or an appropriate reactive derivative thereof. The reaction is desirably effected under mild conditions in order to prevent rupture of the bicyclic nucleus. The reaction temperature is preferably in the range -70 to +70°C.

BAD ORIGINAL

According to one method, the hydroxy ester may be reacted with a diazoalkane or diazoaralkane. This reaction will be preferably effected in the presence of a Lewis acid such as boron trifluoride. The reaction may be effected in a solvent such as an ether e.g. diethylether, dioxan or tetrahydrofuran, a hydrocarbon e.g. a light petroleum fraction, or a halogenated hydrocarbon e.g. dichloromethane or chloroform. The reaction temperature is preferably low e.g. -15°C to +15°C. This method is capable of yielding ethers in which $R^3$ is an alkyl, alkoxyalkyl or aralkyl group. The ester starting material may if desired be formed from the corresponding free acid by reaction with a diazoalkane or diazoaralkane reagent and allowed to react *in situ* with further reagent together with a Lewis acid e.g. $BF_3$ to effect the desired etherification.

Etherification may also be carried out by reaction of the hydroxyester with a vinyl ether. This method is especially useful for production of tetrahydropyranyl ethers, using dihydropyran as reagent, or 1-alkoxyalkyl ethers such as a 1-ethoxyalkyl ether, using an alkyl vinyl ether as reagent. The reaction is desirably carried out in the presence of a strong acid catalyst, for example a mineral acid such as sulphuric acid, or an organic sulphonic acid such as p-toluene sulphonic acid, in a non-hydroxylic, substantially water-free solvent. Examples of suitable solvents include an ether e.g. diethylether, dioxan or tetrahydrofuran, a hydrocarbon e.g. a light petroleum fraction, or a halogenated hydrocarbon e.g. dichloromethane or chloroform. The

BAD ORIGINAL

reaction temperature is preferably in the range -15°C to +35°C.

Silyl ethers may be prepared using the appropriate silyl halide e.g. trimethyl silyl chloride or _t_-butyldimethylsilyl chloride.

Compounds of formula (I) wherein $R^1$ carries an acylated hydroxyl group $-O\overset{Y}{\overset{\|}{C}}R^c$ wherein $R^c$ and Y are as defined above may be prepared by reaction of an ester of formula (I) wherein $R^1$ carries a free hydroxy group or a corresponding free acid with a compound of formula $R^c.\overset{Y}{\overset{\|}{C}}.X$ (wherein X represents a readily displaceable substituent or, when Y is oxygen, optionally, a hydroxy group). The reaction will desirably be effected under mild conditions in order to prevent rupture of the bicyclic nucleus. The use of neutral or mild acidic or basic conditions, therefore, at temperatures between -70°C and +35°C is preferred.

Thus, an ester of formula (I) in which $R^1$ carries a hydroxy group, or a corresponding free acid, may be reacted with a reactive derivative of a carboxylic acid e.g. a halide or anhydride, for example, an acid chloride. In this case reaction may be effected using either the free antibiotic carboxylic acid or, more preferably, an ester thereof, desirably under mild basic conditions e.g. in the presence of a pyridine base in a solvent such as an ether, e.g. tetrahydrofuran or dioxan, or ester, e.g. ethyl acetate,

or halogenated hydrocarbon, e.g. methylene chloride, or substituted amide, e.g. dimethylacetamide. Where a carboxylic acid is used as acylating agent the reaction is preferably carried out in the presence of a coupling agent such as dicylohexylcarbodiimide.

A mono-N-substituted carbamate may be prepared using an isocyanate or isothiocyanate of formula $R^eNCY$, in which $R^e$ and Y are as defined above. The reaction will preferably be carried out by allowing an ester of formula (I) in which $R^1$ carries a hydroxy group to react with an isocyanate or isothiocyanate, optionally in the presence of a mild organic base e.g. pyridine, to yield the acylated derivative of the compound of formula (I).

Where it is desired to prepare compounds in which $R^d$ and $R^e$ are both hydrogen atoms, these may be prepared from mono-N-carbamates (prepared as described above) in which $R^f$ is a group which can be readily replaced by a hydrogen atom as defined above. These groups $R^f$ may be replaced by hydrogen by mild acid or base hydrolysis using, for example, aqueous sodium bicarbonate or dilute aqueous hydrochloric acid.

The carbamates may also be prepared by reaction of an ester of formula (I) in which $R^1$ carries a hydroxy group with a carbamoyl halide of the formula $R^dR^eNCYX$ (where Y is as defined above, $R^d$ and $R^e$ are as defined above other than hydrogen and X is a halogen atom e.g. a chlorine atom) preferably in the presence of a weak base as hydrogen halide acceptor.

The carbamoyl halide may be prepared by reaction of a carbonyl dihalide such as phosgene with a primary

or secondary amine. Alternatively, an ester of formula (I) in which $R^1$ carries a hydroxy group may be reacted with a carbonyl dihalide followed by reaction with a primary or secondary amine. These reactions are most useful in the preparation of disubstituted carbamates.

The compounds of general formula (II) (used as starting materials) may be prepared by a variety of methods. Thus they may, for example, be prepared by reaction sequences involving clavulanic acid and its derivatives or penicillins as starting materials or they may be prepared by total synthesis.

One method of preparing compounds of general formula (II) wherein $R^5$ represents a halogen atom involves reacting a compound of formula (III)

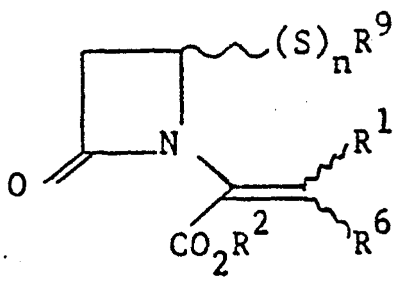

(III)

(wherein $R^1$, $R^2$ and $R^6$ are as hereinbefore defined and: either n is 0 and $R^9$ is a group of formula $-SR^{10}$, $-\overset{O}{\overset{\uparrow}{S}}-R^{10}$ or $-\overset{O}{\overset{\uparrow}{\underset{\underset{O}{\downarrow}}{S}}}-R^{10}$ where $R^{10}$ is an aliphatic, araliphatic, aromatic or heterocyclic group;
or n is 1 and $R^9$ is an acyl group or a group of formula $-SR^{10}$ where $R^{10}$ is as defined above;
with the proviso that when n is 1, $R^1$ is a methyl group) with an electrophilic halogenating agent.

The halogenating agent may, for example, be molecular halogen or an N-halo amide or imide. The N-halo amide or N-halo imide may include a cyclic system,

the amide or imide linkage forming part of the cyclic system; examples of such N-halo amides include N-bromo-caprolactam and examples of such N-halo imides include the 1,3-dibromo-5,5-di-(lower alkyl) hydantoins (e.g. 1,3-dibromo-5,5-dimethyl-hydantoin); N-bromosuccinimide; N-chlorosuccinimide; N-bromophthalimide etc. Other useful N-halo amides include N-halo loweralkanoamides e.g. N-bromoacetamide.

The reaction will preferably be carried out in an inert solvent, e.g. a halogenated hydrocarbon, for example methylene chloride.

Molecular halogen, e.g. chlorine or bromine, may, for convenience, be added in solution, e.g. in a halogenated hydrocarbon, such as carbon tetrachloride.

Suitable reaction temperatures are from -150°C to 25°C. The use of low temperature e.g. below about 0°C, has been found advantageous in assisting the control of the reaction.

Where molecular halogen is used, it may be desirable to carry out the reaction in the presence of an acid binding agent in order to remove any hydrogen halide as it is formed and thus prevent side reactions. Suitable acid binding agents include amides e.g. carboxylic acid amides such as acetamide, metal carbonates e.g. calcium carbonate, oxiranes e.g. propylene oxide, or molecular sieves.

The acid binding agent, when used, will desirably be present in a quantity which ensures as rapid removal of hydrogen halide as possible.

When $R^9$ represents a group $-SR^{10}$, $-\overset{O}{\overset{\uparrow}{S}}-R^{10}$ or $-\underset{O}{\underset{\downarrow}{\overset{O}{\overset{\uparrow}{S}}}}-R^{10}$ in which $R^{10}$ is an aliphatic group, $R^{10}$ may be saturated or unsaturated and thus may, for example, be an alkyl or alkenyl group which may contain 1-6 carbon atoms e.g. $C_{1-4}$ alkyl such as methyl or butyl, or a cycloalkyl group, which may have 3-7, preferably 5 or 6 carbon atoms. Such alkyl groups may, optionally, carry one or more etherified hydroxy groups.

When $R^{10}$ is an araliphatic or aromatic group, it will desirably be an aralkyl group which may have 1-6 carbon atoms in the alkyl portion, or an aryl group, the rings in such aryl and aralkyl groups, preferably being monocyclic, e.g. phenyl; and optionally carrying substituents such as $C_{1-4}$ alkyl groups e.g. methyl groups.

When $R^{10}$ is a heterocyclic group, it will desirably contain a carbon-attached 5-7 membered heterocyclic ring containing one or more heteroatoms such as nitrogen, sulphur or oxygen, which may be aromatic e.g. pyridyl and/or carry one or more alkyl groups, for example containing 1-6 carbon atoms e.g. a methyl group or may have a bicyclic structure, an example of such a group being a benzothiazol-2-yl group.

Where $R^9$ is an acyl group, this may, for example, be an alkanoyl or aralkanoyl group containing from 1 to 20, preferably from 1 to 7, carbon atoms. Especially preferred is an acetyl group.

Specific groups $-(S)_nR^9$ which may be mentioned

include, for example, pyrid-2-yldithio; $C_{1-5}$ alkanoylthio e.g. acetylthio; methylsulphinyl; p-tolylthio; benzothiazol-2-yldithio; $C_{1-4}$ alkylthio e.g. n-butylthio, methylthio and ethylthio; benzylthio; and $C_{2-5}$ alk-1-enylthio groups.

Compounds of formula (III) may, for example, be prepared from compounds of formula (IV),

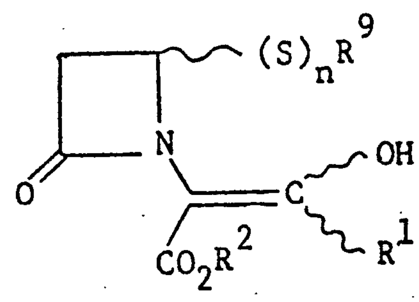

(IV)

(wherein $R^1$, $R^2$, $R^9$ and n are as hereinbefore defined) by reaction with one or more reagents serving to replace the enolic hydroxy group by, or convert the enolic hydroxyl group into, a leaving group e.g. a halogen atom or an acyloxy or ether group.

Compounds of formula (III) in which $R^6$ represents a halogen atom may, for example, be prepared by treating a compound of formula (IV) with dimethylformamide and phosphorus trichloride at a temperature of from -20° to +40°C.

Compounds of formula (III) in which $R^6$ is an acyloxy group may be prepared by reacting a compound of formula (IV) with an appropriate acylating agent, e.g. an acyl halide or anhydride. Such an acyl halide will desirably be a phosphinyl or sulphonyl halide, the phosphinyl and sulphonyl portions being as defined above. Thus, in this case, the compound of formula (IV) may be reacted

with an alkane sulphonyl halide or aryl sulphonyl halide, for example methanesulphonyl halide, p-toluene sulphonyl halide or a dialkoxyphosphinyl chloride e.g. dimethoxyphosphinyl chloride. Reaction is normally carried out in a solvent, e.g. an ether such as diethyl ether or tetrahydrofuran or a halogenated hydrocarbon such as methylene chloride. Reaction temperatures are generally from -30 to +30°C, preferably from -15 to +20°C.

The reaction will normally be effected in the presence of an acid binding agent, e.g. a tertiary amine such as triethylamine, pyridine or collidine or an oxirane such as propylene oxide.

Compounds of formula (III) in which $R^6$ is an ether group may, for example, be prepared from compounds of formula (IV) by reaction with a diazoalkane, e.g. diazomethane, optionally in the presence of a Lewis acid catalyst such as boron trifluoride etherate. The reaction may be effected in a solvent such as an ether e.g. diethylether, dioxan or tetrahydrofuran, a hydrocarbon e.g. light petroleum fraction. or a halogenated hydrocarbon e.g. dichloromethane or chloroform. The reaction temperature is preferably low, e.g. -15° to +15°C.

Alternatively a compound of formula (II) where $R^6$ is as defined above and $R^5$ represents a halogen atom may be prepared from a compound corresponding to formula (II) but where $R^6$ is hydroxyl, by reaction with one or more reagents serving to replace the hydroxyl group by or convert it into a leaving group, e.g. a halogen atom or an acyloxy or ether group. Such reagents may be of the type previously described in relation to compounds of formula (IV). For acylation an acid binding agent will

normally be present, e.g. an oxirane such as propylene oxide or a base; where a base is used, this is preferably a weak base such as pyridine.

The compounds corresponding to formula (II) in which $R^5$ is halogen but in which $R^6$ is hydroxyl may be prepared from compounds of formula (IV) by reaction with an electrophilic halogenating agent e.g. of the type described above in relation to the compounds of formula (III).

The compounds of formula (IV) wherein n is 0, $R^9$ represents a group of formula $-SR^{10}$ as defined above and $R^1$ represents an ethyl group optionally substituted in the β-position by a hydroxyl, etherified hydroxyl or acylated hydroxyl group may be readily prepared from the compounds of formula (V)

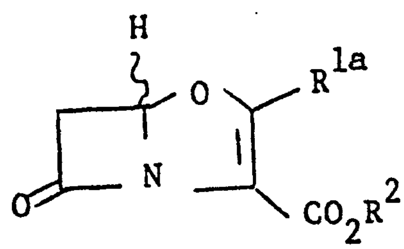

(V)

(wherein $R^2$ is as defined above and $R^{1a}$ represents an ethyl group optionally substituted in the β-position by a hydroxyl, etherified hydroxyl or acylated hydroxyl group) by reaction thereof with a thiol $R^{10}SH$, wherein $R^{10}$ is as defined above.

The thiol $R^{10}SH$ will preferably be present in excess as compared with the starting material of formula (V).

The reaction will desirably be carried out in an aprotic organic solvent, e.g. an ether solvent such as tetrahydrofuran although the thiol may itself serve as the

medium for the reaction. The reaction will desirably be carried out at or above ambient temperature, e.g. at from +20° to +80°C.

The compounds of formula (V) may be prepared from compounds of formula (VI)

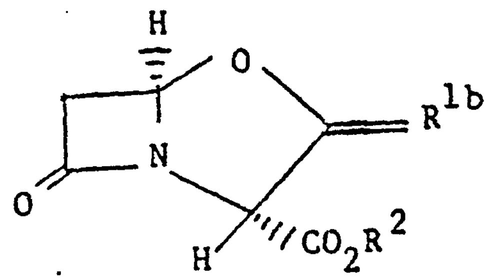

(VI)

(wherein $R^{1b}$ is an ethylidene group optionally substituted in the β-position by a hydroxyl, etherified hydroxyl or acylated hydroxyl group and $R^2$ has the above meanings) as described in Belgian Patent No. 858515.

Compounds of formula (II) wherein $R^5$ represents a group of formula $-\overset{\oplus}{N}R^xR^yR^z$ may be prepared by reaction of compounds of formula (VII)

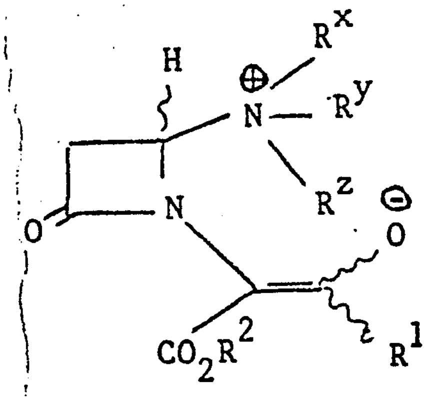

(VII)

(where $R^1$, $R^2$, $R^x$, $R^y$ and $R^z$ have the above meanings) by reaction with an appropriate reagent serving to introduce the group $R^6$. The introduction of the group $R^6$ may be effected by the procedures described above for the formation of compounds of formula (III). In particular, the compound of formula (VII) may be reacted with a sulphonylating agent e.g. a methanesulphonylating agent such as methanesulphonyl chloride. to form a product of formula (II) in which $R^6$ is methanesulphonyloxy. In general, as mentioned above, an anion $A^{\ominus}$, derived from the reagent used for introduction of the group $R^6$, e.g. a halide ion such as a chloride ion will be associated with the compound of formula (II) thus formed. The reaction may be effected in the presence of a base for example a tertiary base such as pyridine. The latter may serve as solvent or a further solvent may be present, e.g. a halogenated hydrocarbon such as methylene chloride or 1,2-dichloroethane.

If desired such a compound of formula (VII) may be used in the presence of pyridine to prepare a compound of formula (II) in situ which compound may then be directly cyclised to a compound of formula (I) by addition of triethylamine and passing in hydrogen sulphide. Heating, e.g. to about 70°C completes the reaction. Where a compound of formula (IIa) is prepared from a compound of formula (VII) without isolation of the compound of formula (II), the compound of formula (IIa) may be partly in the form of an acid addition salt with an acid HA (where A is as defined above) such as HCl.

Compounds of formula (VII) may, for example, be prepared by reaction of a compound corresponding to formula (II)(in which $R^5$ is halogen but in which $R^6$ is a hydroxyl group)with a tertiary base $NR^xR^yR^z$ in the presence of a solvent such as ethyl acetate at temperatures of from 0 to 30°C, preferably at ambient temperature.

Compounds of formula (VII) in which $R^1$ represents an ethyl group optionally substituted in the β-position by a hydroxyl, etherified hydroxyl or acylated hydroxyl group may alternatively be prepared by reaction of a compound of formula (VI) with a base $NR^xR^yR^z$ as described in Belgian Patent 858516.

Clavulanic acid (i.e. the compound of formula (VI) wherein $R^{1b}$ is 2-hydroxyethylidene and $R^2$ is hydrogen) is well known from the literature. The esters thereof are described in, for example, German OLS 2657081 and South African Patent Specification No. 76/1953. Compounds of general formula (VI) wherein $R^{1b}$ represents an ethylidene group are described in Belgian Patent Specification No. .

849,474. Compounds of general formula (VI) wherein $R^{1b}$ represents an etherified 2-hydroxyethylidene group are described in German OLS 2657048 and South African Patent Specification No. 76/5560. Compounds of general formula (VI) wherein $R^{1b}$ represents a 2-acyloxyethylidene group are described in South African Patent Application No. 75/6473 and Belgian Patent Specification No. 847046. The compounds of general formula (VI), the preparation of which is not specifically described in the above publications, may be prepared by methods analogous thereto.

It should be noted that in the above reaction sequences and those described hereinafter the group $R^1$ may be modified between the various reaction stages set out, although remaining within the stated definition. Thus, for example, it will be appreciated that where $R^1$ carries a hydroxyl group then it may be necessary to protect this group during certain stages of the reaction sequence . By way of example, it is possible to start with a clavulanic acid ester of formula (VI) in which $R^{1b}$ carries a hydroxyl group and either at that stage or at a later stage, etherify or acylate the hydroxyl group in order to produce eventually a compound of formula (I) in which $R^1$ carries an etherified or acylated hydroxyl group. Similarly, in producing a compound of formula (I) in which $R^1$ carries a hydroxyl group, it may be convenient to conduct certain reaction stages with the hydroxy group in the form of a protected hydroxyl group, e.g. an etherified or esterified hydroxyl group, and subsequently deprotect to yield the free hydroxyl group. The $R^1$ group

may be modified between the various reaction stages by methods analogous to those described above for the modification of $R^1$ in the compounds of formula (I).

The compounds of formula (IV) wherein $R^1$ represents a methyl group may be obtained from compounds of formula (VIII),

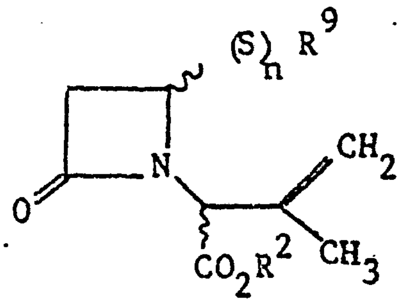

(VIII)

(wherein n, $R^2$ and $R^9$ are as defined above) by means of ozonolysis followed by treatment with a mild reducing agent. Ozonolysis is preferably effected with ozone in the presence of an organic solvent such as for example dichloromethane and/or methanol and at low temperatures e.g. about -78°C. The mild reducing agent may, for example, comprise aqueous sodium metabisulphite or dimethylsulphide.

Compounds of formula (VIII) in which n is 1 and $R^9$ represents a group of formula $-SR^{10}$ may themselves be obtained, if desired, by reaction of a compound of formula (IX),

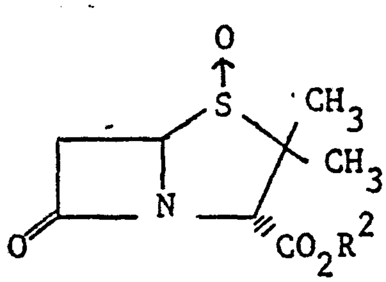

(IX)

(wherein $R^2$ is as defined above) with a thiol $R^{10}SH$ (wherein $R^{10}$ is as defined a... Advantageously the reaction is effected in the presence of an organic solvent e.g. toluene and at elevated temperatures e.g. the boiling point of the reaction mixture.

Compounds of formula (VIII) or indeed compounds of formula (III)or (IV) themselves where n is 0 and $R^9$ represents a group of formula $-SR^{10}$ may be obtained by reaction of the corresponding compound of formula (VIII), (III) or (IV) where n is 1 and $R^9$ represents a group of formula $-SR^{10}$ with a desulphurising agent. The desulphurising agent may, for example, be a trivalent phosphorus compound of formula $P R^{11} R^{12} R^{13}$ where $R^{11}$ and $R^{12}$ are hydrocarbyl, hydrocarbyloxy or hydrocarbylamino groups or $R^{11}$ and $R^{12}$ may together with the phosphorus atom to which they are attached form a ring and $R^{13}$ is as defined for $R^{11}$ and $R^{12}$ or represents a hydroxyl group. Particular reagents which may be used include di- and tri- alkyl phosphites, preferably the latter e.g. trimethyl phosphite, and tri-substituted phosphines. Reduction of a disulphide to a monosulphide may thus be carried out either before or after the conversion of the compound of formula (VIII) into the compound of formula (IV).

Compounds of formula (VIII) wherein n is 1 and $R^9$ represents an acyl group may be obtained from a compound of formula (IX) as defined above by treatment with a trivalent phosphorus compound in the presence of an appropriate acylating agent such as, for example, an anhydride or mixed anhydride. The triva nt phosphorus compound may, for example, be a tri-lower lkyl phosphite e.g.

trimethyl phosphite. The reaction is preferably effected in an inert solvent e.g. a hydrocarbon such as benzene or toluene, an ester such as ethyl acetate or an excess of the acylating agent, where liquid. Compounds of formula (VIII) wherein n is 0 and $R^9$ represents a group of formula $-\underset{\underset{O}{\downarrow}}{\overset{\overset{O}{\uparrow}}{S}}-R^{10}$ or $-\overset{\overset{O}{\uparrow}}{S}-R^{10}$ may be obtained by appropriate oxidation of the corresponding monosulphide (i.e. compound of formula (VIII) wherein n is 0 and $R^9$ represents a group of formula $-S-R^{10}$) for example using a peracid such as e.g. peracetic acid, monoperphthalic acid, m-chloroperbenzoic acid or metaperiodic acid, preferably in the presence of an organic solvent such as acetic acid or dichloromethane. Preferred temperatures are not greater than 10°C e.g. about 0°C. Where the monoxide is desired, then it is preferred to avoid using excess oxidising agent, in order to minimise sulphone formation. Alternatively, oxidation to form the sulphoxide or sulphone may be effected at a later stage in the reaction sequence. Further, as will be appreciated, ozonolysis of a compound of formula (VIII) wherein n is 0 and $R^9$ represents a group of formula $-SR^{10}$ may lead to the formation of some compound of formula (IV) wherein n is 0 and $R^9$ represents a group of formula $-\overset{\overset{O}{\uparrow}}{S}-R^{10}$ as well as the compound of formula (IV) wherein n is 0 and $R^9$ represents a group of formula $-S-R^{10}$, particularly if the ozonolysis is carried out over prolonged periods of time. However, both

compounds of formula (IV) may be processed through to the desired compound of formula (I) as described hereinabove.

Compounds of formula (IX) may be prepar example, by oxidation of a compound of formula

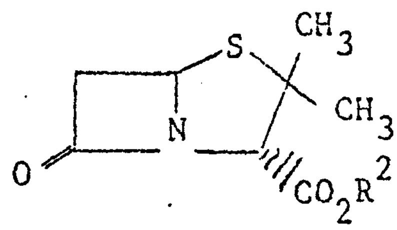 (X)

(wherein $R^1$ and $R^2$ are as defined above) for example by the peracid oxidation described above. In the present case it is preferred to avoid using excess oxidising agent, to minimise sulphone formation.

The compounds of formula (X) may in turn be prepared from penicillanic acid of formula (XI)

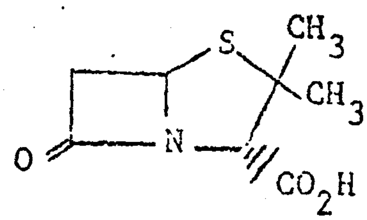 (XI).

Introduction of a carboxyl blocking group $R^2$ may be effected according to methods known <u>per se</u> e.g. esterification such as described above.

An alternative method of obtaining the compounds of formula (IV) wherein n is 0 and $R^9$ represents a group $-SR^{10}$, $-\overset{\overset{O}{\uparrow}}{S}-R^{10}$ or $-\underset{\underset{O}{\downarrow}}{\overset{\overset{O}{\uparrow}}{S}}-R^{10}$ is from a compound of formula (XII),

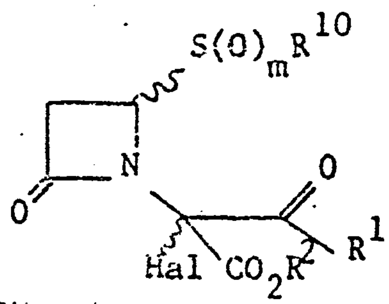

(XII)

(wherein $R^1$, $R^2$ and $R^{10}$ are as defined above, m is 0, 1 or 2 and Hal represents a halogen atom e.g. a chlorine or bromine atom) by reduction with magnesium/iodine or magnesium iodide, preferably in the presence of an anhydrous organic solvent e.g. tetrahydrofuran. A proton source such as, for example, acetic acid is preferably present or added subsequently.

A compound of formula (XII) may, if desired be directly converted into a compound of formula (III) wherein $R^6$ is a phosphinyloxy group by heating with a trivalent phosphorus compound e.g. a trialkyl phosphite, preferably in the presence of an inert solvent such as benzene.

Compounds of formula (XII) may themselves be obtained, if desired, by reaction of a compound of formula (XIII)

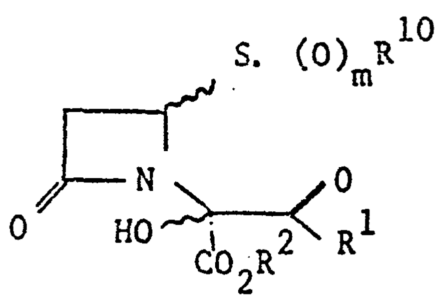

(XIII)

(wherein $R^1$, $R^2$, $R^{10}$ and m are as defined above), with an appropriate halogenating agent, for example, a phosphorus halide, oxalyl chloride or thionyl chloride, preferably in the presence of a base e.g. pyridine. The reaction is preferably effected in the presence of an inert anhydrous organic solvent e.g. dichloromethane.

Compounds of formula (XIII) may be prepared, for example, by reaction of a compound of formula (XIV),

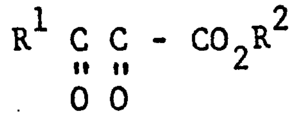

(XIV)

(wherein $R^1$ and $R^2$ are as defined above) or a solvated, e.g. hydrated, form thereof with a compound of formula (XV),

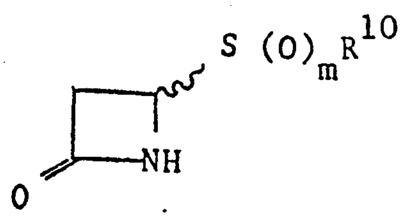

(XV)

(wherein $R^{10}$ and m are as defined above), preferably in the presence of an inert solvent such as benzene and preferably at the boiling point of the reaction mixture. Advantageously the reaction is effected under conditions allowing for azeotropic distillation.

Compounds of formula (XV) may be obtained, for example, from a compound of formula (XVI),

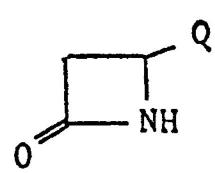

(XVI)

(wherein Q represents a leaving group preferably an acylated hydroxy group such as exemplified hereinbefore in connection with $R^6$) by reaction with a metal salt of an appropriate compound of formula $H\ S(O)_m R^{10}$.

Compounds of general formulae (XII), (XIII) and (XV) wherein m is 0 may, if desired, be converted into their corresponding sulphoxides or sulphones (i.e. m = 1 or 2) by oxidation, for example, according to the peracid technique described above. Alternatively, as mentioned above, oxidation from sulphide to sulphoxide or sulphone, may take place at a later stage in the reaction sequence.

Compounds of formula (XIV) may, for example, be obtained from a compound of formula (XVII),

$$R^1\ \underset{\overset{\shortparallel}{O}}{C}\ \underset{\overset{\shortparallel}{NOH}}{C}\ CO_2\ R^2 \qquad (XVII)$$

(wherein $R^1$ and $R^2$ are as defined above) by treatment with an oxidising agent such as nitrogen dioxide preferably in the presence of an organic solvent such as ether.

Compounds of formula (XVII) may be formed, for example, via the diazonium salt by reaction of a compound of formula (XVIII),

$$R^1\ CO\ CH_2\ CO_2\ R^2 \qquad (XVIII)$$

(wherein $R^1$ and $R^2$ are as defined above) with an aqueous acidic solution of an alkali metal nitrite, e.g. sodium nitrite in the presence of aqueous acetic acid.

According to an alternative embodiment a compound of formula (IV) wherein n is 0 and $R^9$ represents a group

$-SR^{10}$, $-\overset{O}{\overset{\uparrow}{S}}-R^{10}$ or $-\underset{O}{\underset{\downarrow}{\overset{O}{\overset{\uparrow}{S}}}}-R^{10}$ may be obtained by reaction of a compound of formula (XIX),

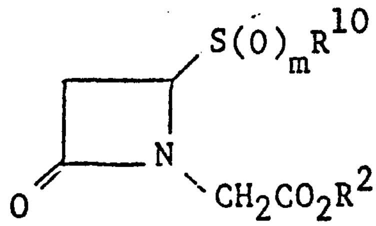

(XIX)

(wherein $R^2$, $R^{10}$ and m are as defined above) with a compound of formula (XX),

$$R^1CO\ Hal'' \qquad (XX)$$

(wherein $R^1$ is as defined above and Hal" represents a halogen, e.g. chlorine, atom) in the presence of a strong base, e.g. lithium di(trimethylsilyl)amide, preferably in the presence of a solvent such as tetrahydrofuran.

Compounds of formula (XIX) may be formed, for example, from a compound of formula (XV) as hereinbefore defined by reaction with a compound of formula (XXI)

$$Hal''CH_2CO_2R^2 \qquad (XXI)$$

(wherein $R^2$ is as defined above and Hal" is as defined above e.g. a bromine atom) in the presence of a base , e.g. sodium hydride, preferably in the presence of an anhydrous organic solvent such as dimethylformamide.

Compounds of general formula (IV) wherein $R^1$ represents a group $-CHR^{14}R^{15}$ (wherein $R^{14}$ represents a hydrogen atom or an optionally substituted alkyl group and $R^{15}$ represents a hydroxymethyl group or a 1-hydroxyethyl group), n is 0 and $R^9$ represents a methylthio group are described in South African Patent Specification No. 77/0435.

Compounds of general formulae (IX) and (X) are known from the literature as are the compounds of formula (XI) and compounds of general formulae (XIV), (XVII), (XVIII), (XX) and (XXI).

The compounds of general formulae (XV), (XVI) and (XIX) are described in Belgian Patent Specifications Nos. 772,940; 755,940 and 772,990 and South African Patent Specification No. 77/0435. The compounds of formula II, IIa, III and IV and compounds corresponding to formula II in which $R^5$ is halogen but wherein $R^6$ is hydroxyl not mentioned above as having been previously described are believed to be novel compounds and thus according to a further feature of the invention there are provided compounds of the general formula (XXII)

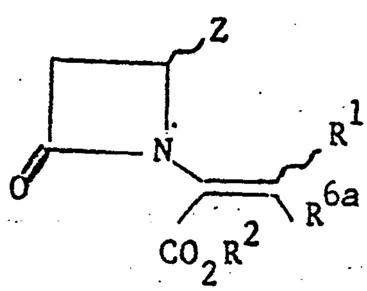

XXII

wherein $R^1$ is as hereinbefore defined; $R^2$ represents a carboxyl esterifying group ; and either Z represents a halogen atom or a group $-(S)_nR^9$ (in which either n is 0 and $R^9$ represents a group $-SR^{10}$,

$$-\overset{O}{\overset{\uparrow}{S}}-R^{10} \quad \text{or} \quad -\overset{O}{\overset{\uparrow}{\underset{\overset{\downarrow}{O}}{S}}}-R^{10}$$

where $R^{10}$ is an aliphatic, araliphatic, aromatic or heterocyclic group; or n is 1 and $R^9$ represents an acyl group or a group - $SR^{10}$ where $R^{10}$ is as defined above) and $R^{6a}$ represents a hydroxyl group or a leaving group;
or Z represents a group of formula

$$-\overset{\oplus}{N}R^xR^yR^zA^{\ominus}$$

(where $R^x$, $R^y$, $R^z$ and $A^{\ominus}$ are as hereinbefore defined) and $R^{6a}$ represents a leaving group;
or Z represents a group $\overset{\oplus}{N}R^xR^yR^z$ (where $R^x$, $R^y$, and $R^z$ are as hereinbefore defined) and $R^{6a}$ represents $S^{\ominus}$:
with the

proviso that when Z represents a methylthio group and $R^{6a}$ represents a free hydroxy group, $R^1$ does not represent a group $-CHR^{14}R^{15}$ where $R^{14}$ represents a hydrogen atom or an optionally substituted alkyl group and $R^{15}$ represents a hydroxymethyl group or a 1-hydroxyethyl group; and with the further proviso that when Z represents a group $-(S)_nR^9$ in which n is 1, $R^1$ represents a methyl group.

Processes for the preparation of the compounds embraced by formula XXII (such processes being as set out hereinbefore) constitute yet further features of the present invention.

The following non-limiting Examples serve to illustrate compounds of the present invention and processes for their preparation.

In the examples, certain bicyclic compounds are named with reference to "penam", the conventional name given to the heterocycle of formula (XXIII),

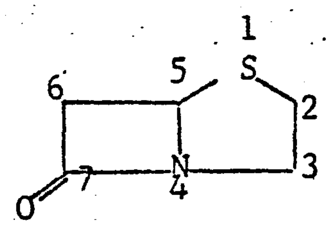

(XXIII)

or "clavam", the name given to the heterocycle of formula (XXIV)

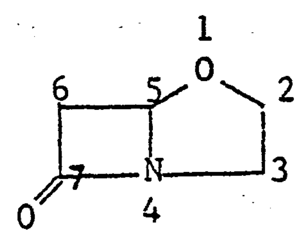

(XXIV)

All temperatures are quoted in °C.
Melting points were determined on a Kofler block or in open ended capilliaries or on a Metler apparatus and are uncorrected.
DMSO is dimethylsulphoxide.
Dichloromethane, toluene and tetrahydrofuran (THF) were dried by passage through basic alumina and stored over molecular sieves (Type 4A).
N,N-dimethylformamide (DMF) was passed through acidic alumina and stored over sieves.
Organic solutions were dried over anhydrous sodium or magnesium sulphate.
Thin layer (tlc) and preparative layer chromatography were conducted on Merck $GF_{254}$ Kieselgel plates, and column chromatography on (A) Merck Kieselgel (0.05-0.2 mm). (B) Crosfield "Sorbsil" silica (60-200 mesh) and (C) May & Baker MFC grade silica; the spots were detected by irradiation with ultra-violet light at 254 nm and/or development with iodine vapour.

Preparation 1

(4RS)-4-p-Tolylthioazetidin-2-one

Sodium hydride (80% suspension in oil, 5.157 g, 0.17 mole) was carefully added portionwise to a solution of p-thiocresol (17.76 g. 0.155 mole) in methanol (80 ml). The solution was then cooled to 5° and a solution of 2-acetoxyazetidin-4-one (18.133 g. 0.14 mole) in methanol (50 ml) was added. After 10 minutes a suspension formed which was poured into a stirred mixture of 2N hydrochloric acid (200 ml) and ethyl acetate (1 l.). The organic phase was separated, washed with brine, and dried, and evaporated. The residue was triturated with dichloromethane: petrol (1:10; 150 ml) and the solid collected, washed once with dichloromethane: petrol (1:20; 150 ml) and dried in vacuo to give the title compound (20.7 g). A similar sample had m.p. 102 to 103° and $\lambda_{max}$ (EtOH) 222.5 nm (ε 10,650) and 254 nm (ε 4,950.)

Preparation 2

p-Nitrobenzyl 2-hydroxyimino-3-oxobutanoate

A solution of sodium nitrite (7.81 g, 113 mmole) in water (30 ml) was slowly added to a stirred and cooled (0°) solution of p-nitrobenzyl 3-oxobutanoate (15.342 g, 64.7 mmole) in glacial acetic acid (30 ml). After 20 minutes the suspension was diluted with water. and extracted with dichloromethane (3 x 500 ml). The combined organic extracts were successively washed with aqueous sodium bicarbonate, water and brine. The organic solution was dried and evaporated to give the title compound (17.05 g). Crystallisation from ether gave an analytical sample with $\lambda_{max}$ ($CHCl_3$) 266.5 nm (ε 10,900).

Preparation 3

p-Nitrobenzyl 2,3-dioxobutanoate

Nitrogen dioxide was bubbled into a solution of the product of Preparation 2 (17.05 g, 64 mmole) in ether (450 ml) at 0° for 5 hours. Concentration of the solution in vacuo at <20° afforded an oil, which was purified by chromatography (B) (ether:dichloromethane - 0:100 to 50:50). Appropriate fractions were evaporated to give the title compound (9.296 g). A similar sample had $\lambda_{max}$ ($CHCl_3$) 265 nm ($E^{1\%}_{1cm}$ 383), inflection at 297.5 nm ($E^{1\%}_{1cm}$ 98).

Preparation 4

p-Nitrobenzyl (2RS,4'RS)-2-hydroxy-3-oxo-2-(2'-oxo-4'-p-tolylthioazetidin-1'-yl)butanoate

A solution of the product of Preparation 1 (0.848 g, 4.4 mmole) and the product of Preparation 3 (0.848 g, 3.1 mmole) in benzene (50 ml) was heated under reflux for 4 hours. Over this period benzene was gradually removed until the final volume of the solution was ca. 10 ml. This was stripped of solvent, the residue redissolved in dichloromethane, and the solution purified by chromatography (B) (dichloromethane). Evaporation in vacuo of the appropriate fractions gave the title compound (0.764 g). A sample prepared by a similar method had $\lambda_{max}$ ($CHCl_3$) 259.5 nm ($E^{1\%}_{1cm}$ 314), inflection at 288.5 nm ($E^{1\%}_{1cm}$ 122).

Preparation 5

p-Nitrobenzyl (2RS,4'RS)-2-chloro-3-oxo-2-(2'-oxo-4'-p-tolylthioazetidin-1'-yl)butanoate

A solution of the product of Preparation 4 (0.200 g,

0.45 mmole) in dry dichloromethane (3 ml) was treated with a solution of thionyl chloride (0.065 ml, 0.9 mmole) in dry dichloromethane (1.3 ml), followed by a solution of pyridine (0.07 ml, 0.9 mmole) in dichloromethane (1.4 ml). After 5 minutes the reaction mixture was washed successively with water and brine, and the organic phase dried and evaporated to give the title compound (0.204 g); $\nu_{max}$ ($CHBr_3$) values include 1778 $cm^{-1}$ (β-lactam).

Preparation 6

p-Nitrobenzyl 4-methyl-3-oxopentanoate

Ethyl 4-methyl-3-oxopentanoate (2.787 g, 17.6 mmole) and p-nitrobenzyl alcohol (4.046 g, 26.4 mmole) were vigorously stirred at 160 to 170° for 2½ hours. The product was purified by chromatography(B)(dichloromethane). Appropriate fractions gave the title compound (3.181 g) $\lambda_{max}$ ($CHCl_3$) 265 nm (ε 11,200).

Preparation 7

p-Nitrobenzyl 2-hydroxyimino-4-methyl-3-oxopentanoate

A solution of sodium nitrite (20.5 g, 0.3 mmole) in water (80 ml) was slowly added to a stirred and cooled (ca. 0°) solution of the product of Preparation 6 (39.4 g, 0.15 mmole) in glacial acetic acid (200 ml). After 15 minutes water was added and the mixture was extracted with dichloromethane (1 l., 3 x 500 ml). The combined organic extracts were washed successively with aqueous sodium bicarbonate, water and brine. The organic solution was dried and evaporated to give the title compound (40.05 g). A similar sample was purified by preparative layer chromatography (dichloromethane: ether = 9:1) to afford the title compound as a gum, which

crystallised on refrigeration, m.p. 55 to 60°, $\lambda_{max}$ ($CHCl_3$) 267 nm (ε 10,650).

Preparation 8

p-Nitrobenzyl 4-methyl-2,3-dioxopentanoate

Nitrogen dioxide was bubbled into a cooled (0°) solution of the product of Preparation 7 (3.15 g, 10.7 mmole) in ether (70 ml) for 5 hours. Concentration of the solution *in vacuo* at 20° afforded an oil which was purified by chromatography (B) (dichloromethane). Appropriate fractions were combined and evaporated to give the title compound (1.034 g); $\lambda_{max}$ ($CHCl_3$) 264 nm (ε 9,820).

Preparation 9

p-Nitrobenzyl (2RS,4'RS)-2-hydroxy-4-methyl-3-oxo-2-(2'-oxo-4'-p-tolylthioazetidin-1'-yl)pentanoate

A solution of the product of Preparation 1 (0.565 g, 2.93 mmole) and the product of Preparation 8 (0.892 g, 2.98 mmole) in benzene was heated under reflux for 3 hours during which time benzene was gradually removed to give a final volume of *ca.* 5 ml. Evaporation of this solution *in vacuo* afforded a gum which was purified by chromatography (B) (ether:dichloromethane = 1:99). The appropriate fractions were combined and evaporated to give the title compound (0.831 g); $\lambda_{max}$ (EtOH) 259 nm ($E^{1\%}_{1cm}$ 301).

Preparation 10

p-Nitrobenzyl (2RS,4'RS)-2-chloro-4-methyl-3-oxo-2-(2'-oxo-4'-p-tolylthioazetidin-1'-yl)pentanoate

A solution of the product of Preparation 9 (10.57 g,

22.4 mmole) in dry dichloromethane (250 ml) was treated with thionyl chloride (3.38 ml, 47 mmole) followed by pyridine (3.42 ml, 43 mmole). After 10 minutes the reaction mixture was washed successively with water, and brine, and the dried organic phase was then evaporated to yield title compound (10.82 g).

Preparation 11

p-Nitrobenzyl 3-oxohexanoate

Ethyl 3-oxohexanoate (20 g, 0.1264 mole) and p-nitrobenzyl alcohol (29 g, 0.1896 mole) were heated together at 170° with vigorous stirring, and the progress of the reaction was monitored by n.m.r. spectroscopy. After 5 hours, the mixture was cooled to ca. 23°, and the resulting oil purified by chromatography (B) (dichloromethane). Appropriate fractions were combined and the solvent evaporated off to give the title ester (25.49 g), $\lambda_{max}$ (EtOH) 263.5 nm (ε9,700).

Preparation 12

p-Nitorbenzyl 2-hydroxyimino-3-oxohexanoate

A cooled (0°) solution of the product of Preparation 11 (25.34 g, 9.55 mmole) in glacial acetic acid (50 ml) was treated, over 5 minutes with a solution of sodium nitrite (13.18 g, 19.1 mmole) in water (50 ml). The resulting suspension was stirred for a further 1 hour and the mixture was extracted with dichloromethane (3 x 100 ml). The organic extracts were combined, covered with water (150 ml) and sodium bicarbonate added until effervescence ceased. The organic phase was separated and washed with water and brine (150 ml of each), and

dried, and the solvent was removed in vacuo to give the title oxime (26.192 g), $\lambda_{max}$ (EtOH) 235 nm ($E^{1\%}_{1cm}$ 373) and 265.5 nm ($E^{1\%}_{1cm}$ 388).

Preparation 13

p-Nitrobenzyl 2,3-dioxohexanoate

A cooled (0°) solution of the product of Preparation 12 (26.192 g) in ether (250 ml) was treated with nitrogen dioxide. After 5 hours the solvent was removed to give an oil. Remaining nitrogen dioxide was removed by addition and evaporation of dichloromethane (100 ml). The residual gum was purified by chromatography (B) (0 to 1.5% ether in dichloromethane), and appropriate fractions were combined and the solvent removed to give the title ester (7.418 g) $\lambda_{max}$ (EtOH) 265 nm ($E^{1\%}_{1cm}$ 73).

Preparation 14

p-Nitrobenzyl (2RS,4'RS)-2-hydroxy-3-oxo-2-[2'-oxo-4'-p-tolythioazetidin-1'-yl]-hexanoate

A solution of the product of Preparation 13 (7.25 g, 26 mmole) and the product of Preparation 1, (5.03 g, 26 mmole) in dry benzene (40 ml) was heated to reflux with azeotropic removal of water. After 5 hours the mixture was allowed to cool and the solvent removed. The residual gum was purified by chromatography (B) (0 to 1% ether in dichloromethane) and appropriate fractions were combined and the solvent removed to give the title compound (4.008 g) $\lambda_{max}$ (EtOH) 260.5 nm ($E^{1\%}_{1cm}$ 321).

Preparation 15

p-Nitrobenzyl (2RS,4'RS)-2-chloro-3-oxo-2-[2'-oxo-4'-p-tolylthioazetidin-1'-yl]hexanoate

A stirred solution of the product of Preparation 14

(3.677 g, 7.782 mmole) in dry dichloromethane (100 ml) at 0° was treated with pyridine (1.19 ml, 14.79 mmole) and thionyl chloride (1.18 ml, 16.34 mmole). After 20 minutes (the temperature was allowed to rise to ca. 23°), water (75 ml) was added and the mixture stirred for a further 10 minutes. The organic layer was separated, washed with brine (60 ml), and dried, and the solvent removed to give the title compound (3.444 g), $\nu_{max}$ ($CHBr_3$) values include 1778 $cm^{-1}$ (β-lactam).

Preparation 16

p-Nitrobenzyl penicillanate

Penicillanic acid (58.5g, 0.29 mole) in N,N-dimethylformamide (110ml) was treated with sodium bicarbonate (24.4g, 0.29 mole) and p-nitrobenzyl bromide (59.7g, 0.28 mole). The mixture was stirred at 25° for 48 hours and then partitioned between ethyl acetate (2 l.) and water (2 l.). The aqueous layer was separated off and further extracted with ethyl acetate (1 l.). The organic layers were washed with water (2 x 1 l.), dried and evaporated. The resulting oil crystallised, and the mixture was diluted with ether and then filtered. The solid was washed with ether and dried to give the title ester (37.5g), m.p. 132°, $[\alpha]_D$+205°(c 1.1, $CHCl_3$).

Preparation 17

p-Nitrobenzyl (1S)-penicillanate, 1-oxide

The product of Preparation 16 (35g, 0.104 mole) in dichloromethane (110ml) at -10° was stirred and treated with peracetic acid (40%, 25ml, 1.2 mole-equivalent) added dropwise over ½-hour. The solution was diluted with more dichloromethane (500ml), and then washed with aqueous sodium

metabisulphite and subsequently with aqueous sodium bicarbonate. It was then dried and concentrated to give a thick slurry of white crystals. This was diluted with methanol and refrigerated. The solid was filtered off, washed with methanol and dried to give the title sulphoxide (34.9 g), m.p. 166°, $[a]_D$+ 200° (c 1.07, $CHCl_3$).

Preparation 18

p-Nitrobenzyl (2R,2'R)-2-(2'-benzothiazol-2-yldithio-4'-oxoazetidin-1'-yl)-3-methylbut-3-enoate

The product of Preparation 17 (34.5 g, 0.1098 mole) and 2-mercaptobenzothiazole (16.39 g, 0.098 mole) were heated to reflux in toluene (240 ml) for 4 hours. The solution was cooled and evaporated to give a slurry of crystals. This was refrigerated; then the solid was filtered off and dried to give the title ester (31.5 g), m.p. 122°, $[a]_D$-258° (c 1.02, $CHCl_3$).

Preparation 19

p-Nitrobenzyl 3-oxoheptanoate

A mixture of ethyl 3-oxoheptanoate (8.57 g, 49.8 mmole) and p-nitrobenzyl alcohol (11.48 g, 75 mmole) was heated to 160° to 170° for 6½ hours. A solution of the cooled mixture in dichloromethane (20 ml) was purified by chromatography (A) (dichloromethane). Combination of the appropriate fractions gave the title ester (8.82 g). This product was used in the following example without further purification. Combination of less polar fractions from the column gave an oil (2.38 g) which was further purified by chromatography (A) (2% ethyl acetate in toluene to give a second crop (1.1 g), $\lambda_{max}$ (EtOH) 263.5nm(ε11,400),

$\nu_{max}(CHBr_3)$ 1740 ($CO_2R$) and 1710 (RCO-)$cm^{-1}$.

Preparation 20

p-Nitrobenzyl 2-hydroxyimino-3-oxoheptanoate

A solution of sodium nitrite (4.498 g, 65.2 mmole) in water (20 ml) was added dropwise over 5 minutes to a stirred and cooled (0°) solution of the product of Preparation 19 (8.66 g, 31.3 mmole) in glacial acetic acid (20 ml). After stirring at 0° for 30 minutes more sodium nitrite (2.25 g) in water (10 ml) was added to the suspension and stirring was continued for a further 1 hour. Dichloromethane (100 ml) and water (50 ml) were added and the aqueous phase was separated and extracted with dichloromethane (25 ml). The combined organic extracts were covered with water (100 ml) and solid sodium bicarbonate was added with stirring until effervescence ceased. The organic phase was separated and washed with water and brine (50 ml of each) and dried and evaporated to a yellow gum (9.61 g) which was essentially the title compound.

A portion (0.200 g) was purified by preparative layer chromatography (toluene:ethyl acetate = 5:1 development) to give a purer sample (0.13 g) as a pale-yellow oil, $\lambda_{max}$ (EtOH) 235 nm ($E^{1\%}_{1cm}$ 371, ε 11,440) and 265 nm ($E^{1\%}_{1cm}$ 363, ε 11,200), $\nu_{max}$ ($CHBr_3$) 1745 ($CO_2R$) and 1690 (C=O) $cm^{-1}$.

Preparation 21

p-Nitrobenzyl 2,3-dioxoheptanoate

Nitrogen dioxide was bubbled into a cooled (0°)

solution of the product of Preparation 20 (9.03 g, 29.3 mmole) in ether (40 ml) for 2½ hours. The solvent was evaporated off and residual nitrogen dioxide was removed by addition and evaporation of dichloromethane (3 x 10 ml). The residue was purified by column chromatography (A) (dichloromethane and dichloromethane:ether = 10:1). Combination of the appropriate fractions gave the title compound (3.21 g) as a gum. This product was further purified by column chromatography (A) (1% ether in dichloromethane) to give a pure sample as a yellow gum $\lambda_{max}$ (EtOH) 266.5 nm ($E^{1\%}_{1cm}$ 293, ε9,120) $\nu_{max}$ ($CHBr_3$) 1746 ($CO_2R$) and 1730 (RCO-)$cm^{-1}$.

Preparation 22

p-Nitrobenzyl (2RS,4'RS)-2-hydroxy-3-oxo-2-(2'-oxo-4'-p-tolylthioazetidin-1'-yl)-heptanoate

A solution of the product of Preparation 21 (12.05 g, 41.1 mmole) and (4RS)-4-p-tolylthioazetidin-2-one (7.222 g, 37.36 mmole) in benzene (50 ml) was heated under reflux for 6 hours during which time benzene was gradually removed to give a final volume of ca 20 ml. More benzene (40 ml) was added, the mixture refluxed for a further 4 hours and the solvent evaporated off. The residue was purified by column chromatography (B) (1% ether in dichloromethane). The appropriate fractions were combined and evaporated to give the title compound (11.635 g) as a yellow oil, $\lambda_{max}$ (EtOH) 259 nm ($E^{1\%}_{1cm}$ 276), $\nu_{max}$ ($CHBr_3$) 1754 (β-lactam), 1732 ($CO_2R$) and 1705 (RCO-)$cm^{-1}$.

Preparation 23

p-Nitrobenzyl (4'RS)-2-(2'-oxo-4'-p-tolylthioazetidin-1'-yl) acetate

Sodium hydride (80%, 0.330 g, 11 mmole) was added to a cooled (-20°) and stirred solution of the product of Preparation 1 (1.93 g, 10 mmole) and p-nitrobenzyl bromoacetate (2.74 g, 10 mmole) in dry DMF (40 ml). The solution was allowed to warm to ca 23° over 1 hour and then poured into a stirred mixture of ethyl acetate (200 ml) and 2N hydrochloric acid (200 ml). The aqueous phase was separated and extracted with ethyl acetate (200 ml). The combined organic solutions were washed with water (2 x 100 ml), and brine (100 ml), dried, and evaporated to a gum.

This gum was purified by chromatography (B) (0 to 2% ether in dichloromethane) and appropriate fractions were combined and evaporated in vacuo to give the title compound (2.63 g).

A sample purified by preparative layer chromatography (dichloromethane:ether (20:1)) gave $\lambda_{max}$ (EtOH) 258 nm ($E^{1\%}_{1cm}$ 350).

Example 1

p-Nitrobenzyl(5RS)-2-methylpen-2-em-3-carboxylate

A cooled (0°) solution of triethylamine (1.36 ml, 9.8 mmole) in dry tetrahydrofuran (30 ml) was treated with hydrogen sulphide for 15 minutes. The resulting solution was added, dropwise to a cooled (-15°) and stirred solution of the product of Example 94 (2.053 g, 4.9 mmole) in dry tetrahydrofuran (30 ml). After 15 minutes nitrogen was first passed through the solution and ether (600 ml) and 0.1M-pH7 phosphate buffer (500 ml) were then added. The organic phase was separated, washed with water, and brine, then dried and evaporated. The residue (1.5 g) in dichloromethane was subjected to chromatography (B) (dichloromethane) to give the title compound (0.677 g). Crystallisation of a similar sample from ether gave analytical material with m.p. 125° (decomp.) and $\lambda_{max}$ (EtOH) 266nm (ε 12,235) and 312.5 (ε 9,130).

Example 2

p-Nitrobenzyl (5RS)-2-methylpen-2-em-3-carboxylate

A cooled (0°) solution of triethylamine (0.08 ml, 0.51 mmole) in dry tetrahydrofuran (5 ml) was treated with hydrogen sulphide for 10 minutes and the resulting solution was then added dropwise to a cooled (-15°) and stirred solution of the product of Example 95 (0.129 g, 0.29 mmole) in dry tetrahydrofuran (3 ml). The reaction mixture was kept at -15° for 30 minutes and then allowed to warm to 0° over 30 minutes, after which time it was flushed

with nitrogen. Ether (60 ml) and 0.1M-pH 7 phosphate buffer (50 ml) were then added and the organic phase was separated, washed with brine and dried. Removal of solvent gave a gum which was subjected to chromatography (B) (dichloromethane). Appropriate fractions were combined and evaporated to give a solid (0.039 g) which was triturated with ether to give the title compound (0.030 g) m.p. 128° (decomp.). The UV (EtOH) spectrum was similar to that given in Example 1.

Example 3

p-Nitrobenzyl (5RS)-2-isopropylpen-2-em-3-carboxylate

Hydrogen sulphide was passed for 20 minutes into a cooled (0°) solution of triethylamine (0.135 ml, 0.98 mmole) in dry dichloromethane (5 ml). The resulting solution was then added, dropwise to a cooled (-15°) and stirred solution of the product of Example 96 (0.218 g, 0.49 mmole) in dry dichloromethane (5 ml). After 1 hour at -15° the reaction mixture was allowed to warm to *ca.* 20° over 2 hours. The above mixture was stored for 14 hours at *ca.* 5° and then treated with more hydrogen sulphide in triethylamine (0.135 ml, 0.98 mmole). The mixture was allowed to warm to *ca.* 20° over 1 hour, and flushed with nitrogen, and washed with 0.1N hydrochloric acid, and brine, and dried, and evaporated to dryness *in vacuo*. The residue was purified by chromatography (B) (dichloromethane). Appropriate fractions were combined and evaporated to a solid (0.076 g). Trituration of this material with ether gave the title compound (0.030 g), m.p. 110 to 125°, $\lambda_{max}$ (EtOH) 264 nm ($\varepsilon$ 11,390) and 313nm ($\varepsilon$ 8,320).

Example 4

p-Nitrobenzyl (5RS)2-propylpen-2-em-3-carboxylate

A solution of triethylamine (0.09 ml, 0.62 mmole) in dry tetrahydrofuran (10 ml) at 0° was saturated with hydrogen sulphide and then added to a solution of the product of Example 97 (0.138 g, 0.31 mmole) in dry tetrahydrofuran (10 ml) at -20°, under nitrogen. After stirring for an hour, nitrogen was bubbled through the solution for 10 minutes to remove the excess of hydrogen sulphide, then ether (15 ml) was added and the mixture was poured into 0.1M - pH 7 phosphate buffer (50 ml). The organic layer was separated, and washed with water, and brine (20 ml of each), and dried. and the solvent evaporated off. The residual yellow gum was purified by preparative layer chromatography to give a gum, (0.037 g), which was identified as the title ester $\nu_{max}$ ($CHBr_3$) 1785 (β-lactam), 1706 ($CO_2R$), 1576 (C=C) and 1520 and 1346 $cm^{-1}$ ($NO_2$).

Example 5

p-Nitrobenzyl (5ξ)-2-methylpen-2-em-3-carboxylate

Triethylamine (10.9 ml, 78.8 mmole) in purified tetrahydrofuran (200 ml) at 5° was stirred under nitrogen and a stream of hydrogen sulphide was passed through for 20 minutes. The product of Example 98 (16.5 g, 39.4 mmole) in purified tetrahydrofuran (100 ml) was added, and the mixture was stirred for 1/4-hour. Excess hydrogen sulphide was removed at reduced pressure; the solution was then diluted with ether (2 l.) and washed with water (4 x500ml), 0.05 N hydrochloric acid (500 ml), and brine (2 xl l.). It

was then dried and evaporated until crystallisation began. The mixture was cooled and filtered; the solid was washed with ether and dried to give the title ester (2.14 g), m.p. 139°. Further concentration of the mother liquors gave a second crop, 2.08 g, $[\alpha]_D$-13° (c 1.1, $CHCl_3$).

Example 6

Sodium (5ξ)-2-methylpen-2-em-3-carboxylate

The product of Example 5 (1.00 g, 3.1 mmole), $[a]_D$-13° ($CHCl_3$) in ethyl acetate (80 ml) with 20% palladium on charcoal (2.0 g) was vigorously shaken under an atmosphere of hydrogen for 3 hours. The catalyst was filtered off and washed with ethyl acetate (2 x 15 ml). The combined filtrate and washings were treated with sodium 2-ethylhexanoate (2.0 mmole) in ethyl acetate (18ml); the resulting suspension was diluted with ether (150 ml, sodium dried) and then cooled to 5° for 1½ hours. The product was filtered off, washed with dry ether and dried in vacuo over phosphorus pentoxide to give the title sodium salt (278 mg), $[\alpha]_D^{24}$-21° (c 0.87, dimethyl sulphoxide).

Example 7

p-Nitrobenzyl (5RS)-2-[2-(1-ethoxyethoxy)ethyl]pen-2-em-3-carboxylate

A stirred suspension of 4-(1-ethoxyethoxy)-1-(p-nitrobenzyloxycarbonyl)-1-(2-oxo-4-triethylammonioazetidin-1-yl)but-1-en-2-olate (30 g) in 1,2-dichloroethane (1 l) at ambient temperature was treated with pyridine (22 ml) followed by mesyl chloride (13 ml). The mixture became homogeneous within 10 minutes and was stirred for a further 35 minutes. The resulting solution was cooled to 0° and treated with a fast stream of hydrogen sulphide gas while triethylamine (50 ml) was added rapidly. The resulting

suspension was diluted with 1,2-dichloroethane (300 ml) and heated rapidly to reflux temperature for ca. 2 minutes while being purged with a fast stream of nitrogen. The stream of nitrogen was maintained while the reaction mixture was cooled in ice to room temperature. The mixture was washed successively with 0.5 N hydrochloric acid (2 x 500 ml). The organic solution was dried and concentrated to ca. 600 ml. Chromatographic silica gel (ca. 50 g) was added and the mixture evaporated to give a powder. The powder was added to the top of a column of silica gel made up in ethyl acetate-petroleum spirit (1:9) and the column then was eluted with ethyl acetate-petroleum spirit (1:9 to 1:1). Appropriate fractions were combined and evaporated to give a solid residue. The residue was broken-up, washed with petroleum spirit and dried *in vacuo* to afford the title ester (18.48 g), $\lambda_{max}$ (EtOH) 316 nm ($\varepsilon$ 8,300).

Example 8

p-Nitrobenzyl (5RS)-2-(2-hydroxyethyl)pen-2-em-3-carboxylate

A solution of the product of Example 7 (18.2 g) in tetrahydrofuran (750 ml) was treated with pH 0.91 aqueous buffer [300 ml, prepared from N sodium acetate, N hydrochloric acid and water (5:8:12] and stood at ambient temperature for 5 hours. The mixture was partitioned between ethyl acetate and brine and the separated organic layer washed successively with 0.5 N aqueous sodium hydrogen carbonate, water and brine. The resulting solution was dried over sodium sulphate and concentrated to give a slurry of crystals. The slurry was diluted with ether and the crystals collected, washed with ether and dried *in vacuo* to

give the <u>title ester</u> (7.38 g), $\lambda_{max}$ (EtOH) 316.5 nm ($\varepsilon$ 9,250).

Example 9

p-Nitrobenzyl (5RS)-2-(2-t-butyldimethylsilyloxyethyl)-pen-2-em-3-carboxylate

A solution of the product of Example 120 (0.254 g) in dry DMF (1 ml) was diluted with ethyl acetate (50 ml) and heated gently to boiling for 5 minutes. The cooled reaction mixture was washed with water (x 2), then dried and evaporated to low volume. The solution was added to a rapidly stirred mixture of ether (16 ml) and petroleum ether (40-60°) (8 ml). The resulting suspension was filtered and the filtrate was evaporated to give the <u>title ester</u> (0.06 g), $\nu_{max}$ ($CHBr_3$) 1784 ($\beta$-lactam) and 1710 $cm^{-1}$ ($CO_2R$).

Example 10

p-Nitrobenzyl (5RS)-2-(2-hydroxyethyl)pen-2-em-3-carboxylate-1-oxide

A solution of 3-chloroperoxybenzoic acid (0.13 g) in dry dichloromethane (1 ml) was added dropwise over 10 minutes to a stirred solution of the product of Example 8 (0.95 g) in dry dichloromethane (4 ml) at 0°. After 15 minutes the mixture was diluted with dichloromethane and washed with water, 0.5N aqueous $NaHCO_3$ and saturated brine. The organic solution was dried and evaporated to low volume to afford a slurry of crystals which was collected and dried *in vacuo* to give the <u>title ester</u> (0.033 g), $\lambda_{max}$ ($CHCl_3$) 265.5 nm ($\varepsilon$ 12,570), $\nu_{max}$ ($CHBr_3$) 1810 $cm^{-1}$ ($\beta$-lactam).

Example 11

Potassium (5RS)-2-(2-hydroxyethyl)pen-2-em-3-carboxylate

A solution of the product of Example 8 (0.678 g) in ethyl acetate (188 ml) was shaken and hydrogenated over 10% palladium on charcoal (Pd-C) (5.42 g) at room temperature and 1 atmosphere pressure. After 35 minutes the mixture was filtered through kieselguhr and the pad was washed with ethyl acetate. The filtrate was treated with a solution of potassium 2-ethylhexanoate (0.14 g) in ethyl acetate (5 ml), then concentrated to ca. 10 ml and diluted with diethyl ether. The resulting solid was collected and dried in vacuo to give the title salt (0.13 g), $\lambda_{max}$ ($H_2O$) 301.5 nm (ε 3,750) $\nu_{max}$ (Nujol) 1750 $cm^{-1}$ (β-lactam).

Example 12

p-Nitrobenzyl (5RS)-2-(2-acetoxyethyl)pen-2-em-3-carboxylate

Acetyl chloride (0.38 ml) was added to a stirred solution of the product of Example 8 (0.934 g) in ethyl acetate (65 ml), maintained at 5°, and containing dry pyridine (1.43 ml). The reaction mixture was allowed to warm to ambient temperature and was stirred for 65 minutes. The resulting mixture was diluted with ethyl acetate and then washed successively with 0.5N aqueous HCl, 0.5N aqueous $NaHCO_3$, and saturated brine. The organic phase was dried and then evaporated to dryness. Trituration with ether afforded a crystalline solid which was collected and dried in vacuo to afford the title ester (0.838 g), $\lambda_{max}$ (EtOH) 315.5 nm (ε 8,300), $\nu_{max}$ ($CHBr_3$) 1786 $cm^{-1}$ (β-lactam).

Example 13

Potassium (5RS)-2-(2-acetoxyethyl)pen-2-em-3-carboxylate

A solution of the product of Example 12 (0.812 g) in ethyl acetate (100 ml) containing 10% Pd-C (6.5 g) was shaken and hydrogenated at room temperature and at 1 atmosphere pressure. After 1 hour the mixture was filtered through kieselguhr and the pad was washed with ethyl acetate (150 ml). The filtrate was treated with a solution of potassium 2-ethylhexanoate (0.126 g) in ethyl acetate (10 ml) and the resulting fine suspension was evaporated to low volume (ca. 7 ml) and, with stirring, was diluted with diethyl ether. The solid was collected and dried in vacuo to afford the title salt (0.178 g), $\lambda_{max}$ (pH6 buffer) 301 nm($\varepsilon$4,440), $\nu_{max}$ (Nujol) 1755 $cm^{-1}$ ($\beta$-lactam).

Example 14

p-Nitrobenzyl (5RS)-2-(2-phenylacetoxyethyl)pen-2-em-3-carboxylate

Phenylacetyl chloride (0.8 ml) was adde. to a stirred, ice-cooled solution of the product of Exampl. 8 (1.05 g) in dry THF (21 ml) containing dry pyridine (1.62 ml). The temperature was allowed to rise to +23° and the mixture was stirred for a further 15 minutes and then partitioned between ethyl acetate and 0.5N aqueous HCl The organic phase was washed successively with 0.5N aqueous HCl, 0.5N aqueous $NaHCO_3$, and saturated brine and then dried. Silica gel was added to the filtered organic solution and the slurry was evaporated to dryness. The impregnated silica gel was placed on top of a dry silica gel column and eluted with ether:petroleum ether (40-60°)

(3:1). Appropriate fractions were combined and evaporated to afford an oil (1.135 g) which crystallised from ethyl acetate/ether. The crystals were collected and dried *in vacuo* to afford the *title ester* (0.55 g), m.p. 107.2-109.6°. $\lambda_{max}$ (EtOH) 317 nm (ε 9,200).

Example 15

(5RS)-2-(2-Phenylacetoxyethyl)pen-2-em-3-carboxylic acid

A solution of the product of Example 14 (0.56 g) in ethyl acetate (80 ml) containing 10% Pd-C (4.48 g) was shaken and hydrogenated at room temperature and at 1 atomosphere pressure. After 40 minutes the mixture was filtered through kieselguhr and the pad was washed with ethyl acetate. The filtrate was extracted with 0.1M aqueous pH7 phosphate buffer (2 x 50 ml) and the combined aqueous extracts were cooled to 3°, then saturated with NaCl, and, with rapid stirring, were acidified to pH 1 with 0.5N aqueous HCl under ethyl acetate. The organic phase was washed with saturated brine, then dried and evaporated to low volume. The solution was added to a rapidly stirred mixture of petroleum ether (40-60°): ether (2:1, 20 ml) and the resulting suspension was evaporated to low volume and diluted with ether. The solid was collected and dried *in vacuo* to afford the *title acid* (0.042 g), $\lambda_{max}$ ($CHCl_3$) 314 nm (ε 5,100), $\nu_{max}$ ($CHBr_3$) 1798 $cm^{-1}$ (β-lactam).

Example 16

p-Nitrobenzyl (5RS)-2-(2-trichloroacetylcarbamoyloxyethyl)-pen-2-em-3-carboxylate

Trichloroacetylisocyanate (0.12 ml) was added to a

stirred suspension/solution of product of Example 8 (0.35 g) in ethyl acetate (20 ml) at 5°. After 20 minutes the solution was diluted with ethyl acetate and washed with 0.5N aqueous $NaHCO_3$, water (x 2), and saturated brine. The dried organic phase was evaporated to low volume, silica gel was added, and the mixture was evaporated to dryness. The impregnated silica gel was placed on top of a dry silica gel column and eluted with ether:petroleum ether (40-60°) (1:1) followed by ether. Appropiate fractions were combined and evaporated to give the title ester (0.33 g), $\nu_{max}$ ($CHBr_3$) 3390 (NH), 1798 (β-lactam). 1732 ($COCCl_3$), 1710 and 1482 $cm^{-1}$ (OCONH).

Example 17

p-Nitrobenzyl (5RS)-2-(2-carbamoyloxyethyl)pen-2-em-3-carboxylate

Trichloroacetyl isocyanate (1.79 ml) was added to a stirred solution/suspension of product of Example 8 (4.20g) in dry ethyl acetate (240 ml) maintained at 0°. After 30 minutes the mixture was diluted with ethyl acetate and washed successively with 0.5N aqueous $NaHCO_3$, water, and saturated brine, and the organic phase was then dried. Evaporation gave an oil which was dissolved in methanol (180 ml) and treated with saturated aqueous $NaHCO_3$ (21 ml). The resulting mixture was allowed to stand at ambient temperature for 55 minutes and then partitioned between ethyl acetate (450 ml) and saturated brine (450 ml). The organic phase was washed with saturated brine (3 x 300 ml), then dried , and evaporated to low volume.

The resulting slurry of crystals was collected and dried *in vacuo* to afford the title ester (3.09 g), m.p. 135-137°, $\lambda_{max}$ (EtOH) 317 nm (ε 9,200).

Example 18

(5RS)-2-(2-Carbamoyloxyethyl)pen-2-em-3-carboxylic acid

A solution of the product of Example 17 (0.71 g) in ethyl acetate (130 ml) containing 10% Pd-C (5.68 g) was shaken and hydrogenated at room temperature and at 1 atmosphere pressure. After 51 minutes the mixture was filtered through kieselguhr and the pad was washed with ethyl acetate. The filtrate was extracted with 0.1M aqueous pH7 phosphate buffer (2 x 30 ml) and the combined extracts were cooled to 3°, then saturated with NaCl, and with rapid stirring, were acidified to pH1 with 0.5N aqueous HCl under ethyl acetate. The organic phase was washed with saturated brine, then dried, and concentrated to low volume. The solution was added to a rapidly stirred mixture of petroleum ether (40-60°):ether (2:1, 20 ml) and the resulting suspension was concentrated to low volume and diluted with ether. The solid was collected and dried *in vacuo* to afford the title acid (0.033 g), $\lambda_{max}$ ($CHCl_3$) 313.5 nm (ε 5,000), $\nu_{max}$ ($CHBr_3$) 1792 $cm^{-1}$ (β-lactam).

Example 19

Potassium (5RS)-2-(2-carbamoyloxyethyl)pen-2-em-3-carboxylate

A solution of the product of Example 17 (1.00 g) in ethyl acetate:ethanol (1:1; 92 ml) containing 10% Pd-C

(4.0 g) was shaken and hydrogenated at room temperature and 1 atmosphere pressure. After 100 minutes the mixture was filtered through kieselguhr and the pad was washed with ethyl acetate:ethanol (1:1). The combined filtrates were treated with a solution of potassium 2-ethylhexanoate (0.232 g) in ethyl acetate (7 ml), then concentrated to low volume, and diluted with diethyl ether. The supernatant was decanted from the resulting solid, and the latter was washed with diethyl ether (3 times), then dried _in vacuo_ to afford the _title salt_ (0.43 g), $\lambda_{max}$ (pH6 buffer) 301 nm (ε 4,950), $\nu_{max}$ (Nujol) 1765 $cm^{-1}$ (β-lactam).

Example 20

p-Nitrobenzyl (5RS)-2-(2-N-methylcarbamoyloxyethyl)pen-2-em-3-carboxylate

A stirred solution/suspension of the product of Example 8 (1.05 g) in ethyl acetate (40 ml) was cooled to +5° and treated with bis-(tri-_n_-butyltin)-oxide (0.25 ml) followed by methyl isocyanate (0.35 ml). The mixture was allowed to warm to ambient temperature and after 30 minutes further portions of bis-(tri-_n_-butyltin)oxide (1.0 ml) followed by methyl isocyanate (1.0 ml) were added. After 10 minutes the resulting solution was diluted with ethyl acetate and washed with saturated brine (x 3). The organic phase was dried and then evaporated to low volume. Addition of a little diethyl ether gave a slurry of crystals which was collected and dried _in vacuo_ to afford the _title ester_ (1.15 g), m.p. 135-138°, $\lambda_{max}$ ($CHCl_3$) 317 nm (ε 8,760).

Example 21

Potassium (5RS)-2-(2-N-methylcarbamoyloxyethyl)pen-2-em-3-carboxylate

A solution of the product of Example 20 (1.0 g) in ethyl acetate: ethanol (1:1; 88 ml) containing 10% Pd-C (4.0 g) was shaken and hydrogenated at room temperature and at 1 atmosphere pressure. After 35 minutes the mixture was filtered through kieselguhr and the pad was washed with a little ethyl acetate and ethanol. The combined filtrates were evaporated to ca. 50 ml and then treated with a solution of potassium 2-ethylhexanoate (0.313 g) in ethyl acetate (15 ml). The solution was diluted with diethyl ether and the resulting solid was collected and dried in vacuo to afford the title salt (0.353 g), $\lambda_{max}$ 300 nm ($\varepsilon$ 5,090), $\nu_{max}$ (Nujol) 1758 $cm^{-1}$ ($\beta$-lactam).

Example 22

p-Nitrobenzyl (5RS)-2-(2-N-phenylcarbamoyloxyethyl)pen-2-em-3-carboxylate

Bis-(tri-n-butyltin)oxide (2.38 ml) was added to a stirred solution/suspension of the product of Example 8 (1.75 g) in dry ethyl acetate (20 ml) at ambient temperature. Phenyl isocyanate (1.02 ml) was added dropwise to the resulting solution and after 2 minutes the mixture was diluted with ethyl acetate and washed with 0.5N aqueous HCl followed by saturated brine. The organic phase was dried and evaporated to low volume. The resulting slurry of crystals was collected and dried in vacuo to afford the title ester (1.46 g), m.p. 151-152°, $\lambda_{max}$ ($CHCl_3$) 317.5 nm ($\varepsilon$ 9,500).

Example 23

Potassium (5RS)-2-(2-N-phenylcarbamoyloxyethyl)pen-2-em-3-carboxylate

A solution of the product of Example 22 (1.425 g) in ethyl acetate:ethanol (1:1; 126 ml) containing 10% Pd-C (5.70 g) was shaken and hydrogenated at room temperature and at 1 atmosphere pressure. After 45 minutes the mixture was filtered through kieselguhr and the pad was washed with ethyl acetate:ethanol (2:1; 75 ml). The combined filtrates were treated with a solution of potassium 2-ethylhexanoate (0.388g) in ethyl acetate (15 ml), then concentrated to ca. 100 ml, and diluted with diethyl ether. The resulting solid was collected, washed with diethyl ether, and then dried in vacuo to afford the title salt (0.472 g), $\lambda_{max}$ (pH 6 buffer) 302 nm (ε 5,200), $\nu_{max}$ (Nujol) 1770 $cm^{-1}$ (β-lactam).

Example 24

Methyl (5RS)-2-ethylpen-2-em-3-carboxylate

Methanesulphonyl chloride (4.77 ml) was added to a stirred, ice-cooled solution of 1-methoxycarbonyl-1-(2-oxo-4-triethylammonioazetidin-1-yl)but-1-en-2-olate (6.13 g) in dry 1,2-dichloroethane (112 ml) containing dry pyridine (8.31 ml). After 20 minutes hydrogen sulphide was passed through the stirred solution under nitrogen, and an excess of triethylamine was added in portions over 10 minutes. The resulting mixture was diluted with dry 1,2-dichloroethane (100 ml) and heated to boiling for 5 minutes. The mixture was

cooled to room temperature and washed successively with 0.5 N aqueous HCl (2x), 0.5 N aqueous $NaHCO_3$ (2x), water (2x), and saturated brine, and then dried. The solution was evaporated to low volume and fractionated on a silica gel column eluting with ethyl acetate-petroleum ether (40-60$^o$) mixtures. Appropriate fractions were combined and concentrated to low volume to give a slurry of crystals which was collected and dried *in vacuo* to afford the *title ester* (0.98 g), $\lambda_{max}$ (EtOH) 313 nm ($\varepsilon$ 7,400), $\nu_{max}$ (Nujol) 1765 $cm^{-1}$ (β-lactam).

Example 25

p-Nitrobenzyl (5RS)-2-n-butylpen-2-em-3-carboxylate

A cooled (0$^o$) solution of triethylamine (4.9 ml, 35.52 mmole) in dry dichloromethane (30 ml) was saturated with hydrogen sulphide and then added to a cooled (0$^o$) solution of the product of Example 105 (5.46 g, 11.84 mmole) in dry dichloromethane (80 ml).

After stirring for 20 minutes at 0 to *ca* 21$^o$, N hydrochloric acid (80 ml) was added and stirring was continued for a further 5 minutes.

The organic layer was washed with water and brine (50 ml of each) and stirred for 5 minutes with anhydrous magnesium sulphate and charcoal. The mixture was filtered through kieselguhr and the solvent was removed to give an orange-yellow gum (4.318 g). This product was divided into five portions each of which

were purified by column chromatography (C) (1% ether in dichloromethane). Appropriate fractions from each portion were combined to give the title compound (1.263 g) $\lambda_{max}$ ($CHCl_3$) 267.5 nm ($E^{1\%}_{1cm}$ 343) and an inflexion at 294 nm ($E^{1\%}_{1cm}$ 226).

Example 26

(5RS)-2-n-Butylpen-2-em-3-carboxylic acid

The product of Example 25 (0.205 g) was dissolved in ethyl acetate (20 ml) and 20% Pd-C (0.41 g) was added. The stirred suspension was treated with hydrogen (2 atmospheres pressure) at 23° for 2$^3$/4 hours.

The mixture was filtered through kieselguhr and the filtrate was added to a solution of sodium bicarbonate (0.095 g) in water and stirred vigorously for several minutes. The organic layer was separated and the aqueous layer extracted with further sodium bicarbonate (0.047 g) in water. The aqueous extracts were combined, covered with ethyl acetate (50 ml) and acidified to pH 2.5 by addition of phosphoric acid. The aqueous layer was separated and extracted with further ethyl acetate (25 ml). The organic phases were combined and washed with brine (30 ml) and dried and evaporated to give the title compound (0.021 g) as a yellow solid. τ ($CDCl_3$) values include 4.36 (m, 5H) and 7.16 (m, $\underline{CH_2}$ $(CH_2)_2CH_3$).

Example 27

p-Nitrobenzyl (5RS)-2-methoxymethylpen-2-em-3-carboxylate

Triethylamine (0.087 ml, 0.62 mmole) in dry N,N-dimethylformamide (3 ml) was added, under nitrogen, to a solution of hydrogen sulphide (0.465 mmole) in N,N-dimethylformamide (0.26 ml) and the mixture was cooled to $-42^{o}$.

A cooled ($-42^{o}$) solution of the product of Example 106 (0.14 g, 0.31 mmole) in dry N,N-dimethylformamide (3 ml) was added and the reaction mixture was stirred for 23 minutes.

Excess hydrogen sulphide was removed and the mixture was poured into ethyl acetate and brine. The organic phase was evaporated and washed five times with brine, then dried (sodium sulphate) and evaporated to dryness.

Dichloromethane was added to the residue and removed in vacuo to give the title compound (0.094 g).

This was further purified by chromatography (B) (deactived by 5% water) (dichloromethane: ethyl acetate 20:1) and appropriate fractions were collected and evaporated to low volume.

Trituration with ether afforded the title compound as white crystals (0.027 g), m.p. 91 to $95^{o}$, $\nu_{max}$ ($CHBr_3$) 1790 (β-lactam) and 1705 ($CO_2R$)$cm^{-1}$.

Example 28

(5RS)-2-Propylpen-2-em-3-carboxylic acid

The product of Example 4 (0.500 g, 1.435 mmole)

was dissolved in ethyl acetate (45 ml) and 20% Pd-C (1 g) was added. The suspension was stirred at ca 25° and hydrogen was passed for 3 hours. The suspension was filtered and the filtrate poured into a stirred solution of sodium bicarbonate (0.24 g, 2.87 mmole) in water (50 ml). After 2 to 3 minutes the organic phase was separated and extracted with sodium bicarbonate (0.12 g, 1.435 mmole) in water (25 ml). The combined aqueous extracts were covered with ethyl acetate (50 ml) and acidified to ca pH 2.5 with phosphoric acid.

The organic layer was separated and the aqueous layer extracted with ethyl acetate (25 ml) and the combined organic extracts were dried and the solvent was removed *in vacuo* to give an orange gum (0.142 g). This gum was combined with the residue (0.044 g) from a similar experiment performed on $\frac{2}{5}$ the scale and the mixture was triturated with ether (5 ml) to give the title acid (0.056 g), as a pale yellow solid. M.p. 110° (decomp), $\lambda_{max}$ (EtOH) 262.5 nm (ε 4,415) and 301.5 nm (ε 4,930), $\nu_{max}$ ($CHBr_3$) 1708 and 1670 ($CO_2H$) and 1784 (β-lactam) $cm^{-1}$.

Evaporation of the mother liquor provided a second crop (0.057 g) (from ether:petroleum ether).

Example 29

Sodium (5RS)-2-isopropylpen-2-em-3-carboxylate

A stirred solution of the product of Example 3

(0.418 g, 1.2 mmole) in dry ethyl acetate (25 ml) was hydrogenated over 20% Pd-C (0.836 g) for 2 3/4 hours.

The mixture was flushed with nitrogen, filtered and the filter bed was washed with dry ethyl acetate (2 x 5 ml). The filtrate and washings were then treated with a solution of sodium D,L-2-ethyl hexanoate (ca 0.11 mmole/ml) in dry ethyl acetate (6.55 ml). This solution was first evaporated to ca 10 ml then diluted with dry ether (100 ml) and again concentrated to ca 5 ml. Slow addition of petrol (40 ml) to the concentrate gave a precipitate which, after refrigeration for several hours was collected, washed with petrol (2 x 5 ml) and dried in vacuo over phosphorus pentoxide to give the crude product (0.102 g).

A portion (0.068 g) of this material was triturated thrice with di-isopropyl ether and then dried in vacuo over phosphorus pentoxide to give the title compound (0.04 g) as a solid, $\lambda_{max}$ (EtOH) 253 nm ($E^{1\%}_{1cm}$ 325) and 300.5 nm ($E^{1\%}_{1cm}$ 151), $\nu_{max}$ (Nujol) 1770 (β-lactam) and 1606 $cm^{-1}$ ($CO_2$-).

Example 30

p-Nitrobenzyl (5RS)-2-ethylpen-2-em-3-carboxylate

A solution of the product of Example 107 (0.864 g) in dry DMF (5 ml) was added slowly, under nitrogen, to a stirred solution of 3-azabicyclo[3,2,2]nonane (0.520 g) in dry DMF (10 ml), saturated with hydrogen sulphide, at 0°. After 5 mins. the mixture was poured, under nitrogen, into ether (250 ml) and 0.5 N aqueous

HCl (40 ml). The organic phase was washed (x4) with saturated brine and then dried. Evaporation to low volume afforded a slurry of crystals which was collected and dried in vacuo to give the title ester (0.350 g), m.p. 107-112$^o$, $\lambda_{max}$ (EtOH) 313.5 nm ($\varepsilon$ 9,400).

Example 31

p-Nitrobenzyl (5RS)-2-ethylpen-2-em-3-carboxylate

A solution of the product of Example 107 (2.23 g) in dry THF (13 ml) was added dropwise, under nitrogen, to a stirred solution of triethylamine (1.44 ml) in dry THF (29 ml), maintained at 0$^o$ and through which hydrogen sulphide was being bubbled. After 20 minutes the mixture was poured into ether (400 ml) and water (150 ml). The organic phase was washed (twice) with water and then dried. Evaporation to low volume afforded a slurry of crystals which was collected and dried in vacuo to give the title ester (1.16 g) whose physical and spectroscopic properties resembled those as described in Example 30.

Example 32

Potassium (5RS)-2-ethylpen-2-em-3-carboxylate

A solution of the product of Example 30 (1.05 g) in ethyl acetate (150 ml) was shaken and hydrogenated with 10% Pd-C (8.4 g). After 3 hours the mixture was filtered through kieselguhr which was washed (twice) with ethyl acetate. The filtrate was treated with a solution of potassium 2-ethylhexanoate (0.60 g) in ethyl acetate (25 ml) and the resulting suspension was

evaporated to low volume and then diluted with ether. The solid was collected and dried in vacuo to afford the title salt (0.280 g), m.p. 134-139°, $\lambda_{max}$ ($H_2O$) 300 nm (ε 5,000).

Example 33

Methyl (5RS)-2-ethylpen-2-em-3-carboxylate

A solution of the product of Example 30 (0.51 g) in ethyl acetate (75 ml) was shaken and hydrogenated with 10% Pd-C (4.06 g). After 2.5 hours the mixture was filtered through kieselguhr which was washed (twice) with ethyl acetate. The filtrate was cooled to 5° and then treated with ethereal diazomethane until a faint yellow colour persisted. After 2 minutes a fast stream of nitrogen was bubbled through the solution for 10 minutes. The reaction mixture was washed with 0.5 N aqueous HCl followed by saturated brine and then dried. Evaporation to low volume afforded a slurry of crystals which was collected and dried *in vacuo* to give the *title ester* (0.102 g), m.p. 88.1°, whose spectroscopic properties resembled those as described in Example 24.

Example 34

p-Nitrobenzyl (5RS)-2-ethylpen-2-em-3-carboxylate-1-oxide

A solution of m-chloroperbenzoic acid (0.065 g) in dry dichloromethane (2 ml) was added dropwise to a stirred solution of product of Example 30 (0.100 g) in dry dichloromethane (2 ml) at 0°. After 25 minutes the mixture was diluted with ethyl acetate and washed successively with aqueous $NaHCO_3$, water, and saturated brine. The organic solution was dried and then evaporated to an oil. Trituration with ether afforded a solid which was collected and dried *in vacuo* to give

the title ester (0.048 g), m.p. 135-137.5°, $\lambda_{max}$ (EtOH) 264.5 nm (ε 12,700).

Example 35

(5RS)-2-Ethylpen-2-em-3-carboxylic acid

A solution of the product of Example 30 (3.31 g) in dry ethyl acetate (150 ml) containing 10% Pd-C (26.45 g) was shaken and hydrogenated at room temperature and 1 atmosphere pressure. After 15 minutes the mixture was filtered through kieselguhr and the pad was washed with ethyl acetate. The combined filtrates contained the title acid (0.99 g) and an aliquot was evaporated to afford the title acid (0.10 g) as a solid which was collected and dried in vacuo, $\lambda_{max}$ (EtOH) 304 nm (ε 3,900), $\nu_{max}$ ($CHBr_3$) 1786 $cm^{-1}$ (β-lactam).

Example 36

Potassium (5RS)-2-ethylpen-2-em-3-carboxylate-1-oxide

Potassium periodate (0.690 g) was added to a stirred solution of the product of Example 32 (0.475 g) in water (10 ml) at ambient temperature. After 3½ hours the mixture was freeze dried and the residue was chromatographed on an XAD-2 resin column eluting with water. Appropriate fractions were combined and freeze-dried to afford the title salt (0.171 g), $\nu_{max}$ (Nujol) 1780 (β-lactam), 1618 $cm^{-1}$ ($CO_2^-$).

Example 37

Methyl (5RS)-2-ethylpen-2-em-3-carboxylate-1-oxide

A solution of 3-chloroperoxybenzoic acid (0.303 g) in dry dichloromethane (5 ml) was added to a stirred, ice-cooled solution of the product of Example 33 (0.320 g) in dry dichloromethane (5 ml). After 15 minutes the mixture was diluted with dichloromethane and washed with 0.5 N aqueous $NaHCO_3$ followed by saturated brine. The organic phase was dried and evaporated to low volume. Dilution with diethyl ether afforded a slurry of crystals which was collected and dried in vacuo to give the title ester (0.19 g), $\lambda_{max}$ ($CHCl_3$) 290 nm (ε 4,100), $\nu_{max}$ ($CHBr_3$) 1808 $cm^{-1}$ (β-lactam).

Example 38

p-Nitrobenzyl (5RS)-2-[2-(tetrahydropyran-2-yloxy)-ethyl]pen-2-em-3-carboxylate

A solution of the product of Example 110 (0.360 g) in dry THF (3 ml) was added dropwise, under nitrogen, to a stirred solution of triethylamine (0.19 ml) in dry THF (5 ml), maintained at 0° and through which hydrogen sulphide was being passed. After 15 minutes the reaction mixture was poured into ether (300 ml) and saturated brine (100 ml). The aqueous phase was further extracted with ether and the combined extracts were washed with saturated brine and then dried. Evaporation afforded the title ester, $\nu_{max}$ ($CHBr_3$)

1788 (β-lactam), 1710 ($CO_2R$), 1520 and 1346 $cm^{-1}$ ($NO_2$).

Example 39

p-Nitrobenzyl (5RS)-2-[2-(tetrahydropyran-2-yloxy)ethyl]-pen-2-em-3-carboxylate

Methanesulphonyl chloride (0.058 g) was added to a stirred solution of 1-(p-nitrobenzyloxycarbonyl)-1-(2-oxo-4-triethylammonioazetidin-1-yl)-4-(tetrahydropyran-2-yloxy)but-1-en-2-olate (0.105 g) in dry pyridine (1 ml). After 2 hours triethylamine (0.050 g) was added to the reaction mixture through which was passed a steady stream of hydrogen sulphide. The solution was diluted with ethyl acetate (20 ml) and then heated gently to 65° for 15 minutes. The organic solution was diluted with ethyl acetate (100 ml) and washed (x3) with water. The organic phase was dried and then evaporated to afford the title ester (0.050 g) whose spectroscopic properties resembled those as described in Example 38.

Example 40

p-Nitrobenzyl (5RS)-2-[2-(tetrahydropyran-2-yloxy)-ethyl]pen-2-em-3-carboxylate

A suspension of the product of Example 123 (0.108 g) in ethyl acetate (30 ml) was heated gently to reflux for 5 minutes. The resulting solution was evaporated to afford the title ester (0.087 g), whose spectroscopic properties resembled those as described in Example 38.

0000636

Example 41

p-Nitrobenzyl (5RS)-2-ethylpen-2-em-3-carboxylate

Methanesulphonyl chloride (0.126 g) was added to a stirred solution of 1-(p-nitrobenzyloxycarbonyl)-1-(2-oxo-4-triethylammonioazetidin-1-yl)but-1-en-2-olate (0.419 g) in dry pyridine (1.5 ml). After 4 hours triethylamine (0.202 g) was added to the reaction mixture through which was passed a steady stream of hydrogen sulphide. After 15 minutes the solution was diluted with ethyl acetate (50 ml) and heated gently to 70$^{o}$ for 10 minutes. The organic solution was washed with 0.1 N aqueous HCl followed by saturated brine, and then dried. Evaporation afforded the title ester (0.050 g) whose spectroscopic properties resembled those as described in Example 30.

Example 42

p-Nitrobenzyl (5RS)-2-ethylpen-2-em-3-carboxylate

A suspension of the product of Example 121 (0.127 g) in ethyl acetate (30 ml) was heated gently to reflux for 5 minutes. The organic solution was evaporated to afford the title ester (0.02 g) whose spectroscopic properties resembled those as described in Example 30.

Example 43

Potassium (5RS)-2-[2-(tetrahydropyran-2-yloxy)ethyl]-pen-2-em-3-carboxylate

Zinc powder (26.0 g) was added in portions to a

stirred solution of the product of Example 38 (2.17 g) in 25% aqueous THF (100 ml) at 0° and maintained at pH 4.3 by the addition of 1 N aqueous HCl. After 4.25 hours the mixture was filtered through kieselguhr. The filtrate was saturated with NaCl, covered with ethyl acetate (1.1L) and, with stirring, acidified with 2N aqueous HCl. The organic phase was concentrated to ca 400 ml and then extracted with pH 7 aqueous buffer (2 x 700 ml). The combined aqueous extracts, saturated with NaCl, were covered with ethyl acetate (1L) and, with stirring, were acidified with 2 N aqueous HCl. The aqueous phase was re-extracted with ethyl acetate (300 ml) and the combined organic solutions were dried, then concentrated to 116 ml, and treated with a solution of potassium 2-ethylhexanoate (0.416 g) in ethyl acetate (15 ml). The resulting suspension was concentrated to ca. 30 ml and was then diluted with ether. The precipitated solid was collected and dried *in vacuo* to afford the title salt (0.56 g), m.p. 122-126°, $\lambda_{max}$ 300 nm ($\varepsilon$ 3,500).

Example 44

p-Nitrobenzyl (5RS)-2-(2-methoxyethyl)pen-2-em-3-carboxylate

A suspension of the product of Example 125 (2.33 g) and $Na_2CO_3$ (0.13 g) in ethyl acetate (50 ml) was heated gently to reflux for

3 minutes and then filtered. The filtrate was added slowly to a stirred mixture of ether; petroleum ether (40-60°) (2:1, 100 ml) and the mixture refiltered. Evaporation gave an oil (1.38 g) which was chromatographed on a column of silica gel eluting with ether. Appropriate fractions were combined and evaporated to low volume (*ca*. 8 ml) and then diluted with ethyl acetate (2 ml). The resulting slurry of crystals was collected and dried *in vacuo* to afford the *title ester* (0.216 g), m.p. 73.5-75.0°, $\lambda_{max}$ (EtOH) 316 nm ($\varepsilon$ 9,000).

Example 45

Sodium (5RS)-2-(2-methoxyethyl)pen-2-em-3-carboxylate

A solution of the product of Example 44 (0.49 g) in ethyl acetate (30 ml) was shaken and hydrogenated with 10% Pd-C (3.93 g). After 3 hours the mixture was filtered through kieselguhr and the pad was washed (twice) with ethyl acetate (2 x 10 ml). The combined organic solutions were treated with a solution of sodium 2-ethylhexanoate (0.08 g) in ethyl acetate (5 ml) and then evaporated to low volume (*ca*. 5 ml). Dilution with ether afforded a deliquescent solid which was collected, then redissolved in water (25 ml), and freeze-dried to afford the *title salt* (0.025 g), $\lambda_{max}$ ($H_2O$) 301 nm ($\varepsilon$ 3,300), $\nu_{max}$ ($D_2O$) 1750 $cm^{-1}$ ($\beta$-lactam).

Example 46

p-Nitrobenzyl-2-[2-(1-ethoxyethoxy)ethyl]pen-2-em-3-carboxylate

A suspension of the product of Example 126 (5.23 g) and $Na_2CO_3$ (0.253 g) in ethyl acetate (100 ml) was heated gently to reflux for 5 minutes. The mixture was filtered and the filtrate was concentrated to ca 5 ml and then added to a stirred mixture of ether: petroleum ether (40-60$^o$): ethyl acetate (6:3:1; 100 ml). The mixture was quickly refiltered and the filtrate was evaporated to give an oil (2.47 g). A portion of the crude product (0.30 g) was chromatographed on a column of silica gel eluting with ether-petroleum ether (40-60$^o$) (3:1). Appropriate fractions were combined and evaporated to give an oil (0.167 g). Trituration with ether gave a solid which was collected and dried *in vacuo* to afford the title ester (0.065 g), m.p. 77.8°, whose spectroscopic properties resembled those as described in Example 7.

Example 47

Potassium (5RS)-2-[2-(1-ethoxyethoxy)ethyl]pen-2-em-3-carboxylate

A solution of the product of Example 46 (0.49 g) in ethyl acetate (70 ml) was shaken and hydrogenated over 10% Pd-C (3.92 g). After 90 minutes the mixture was filtered through kieselguhr and the pad was washed with ethyl acetate (150 ml). The organic solution was concentrated to ca 20 ml, then treated with a solution of potassium 2-ethylhexanoate (0.128 g)

in ethyl acetate (5 ml), and reconcentrated to ca 3 ml. Addition of petroleum ether (40-60°) to the stirred solution gave a solid which was collected and dried in vacuo to afford the title salt (0.08 g), m.p. 113-1°, $\lambda_{max}$ ($H_2O$) 301.5 nm ($\varepsilon$ 5,200).

Example 48

p-Nitrobenzyl 2-(2-hydroxyethyl)pen-2-em-3-carboxylate

A solution of the product of Example 46 (0.33 g) in acetic acid:water:THF (2.5:3.0:5.0 ml) was stood at room temperature for 24 hours and was then poured into ethyl acetate (100 ml) and water (50 ml). The organic phase was washed with 0.5 N aqueous $NaHCO_3$ followed by water and then dried. Evaporation gave an oil which was chromatographed on a silica gel column eluting with ether-petroleum ether (40-60°) (3:1) followed by ether. Appropriate fractions were combined and evaporated to an oil (0.08 g) which crystallised from ethyl acetate/ether to afford the title ester (0.04 g), m.p. 146.2°, whose spectroscoptic properties resembled those as described in Example 8.

Example 49

p-Nitrobenzyl 2-(2-hydroxyethyl)pen-2-em-3-carboxylate

A solution of the product of Example 46 (0.45 g) in methanol (10 ml) containing 0.1 N aqueous HCl (2 ml) was stood at room temperature for 90 minutes and then partitioned between ethyl acetate (75 ml) and water (40 ml). The organic phase was washed with

saturated aqueous $NaHCO_3$ followed by water, then dried, and concentrated to 5 ml. Dilution with ether afforded a slurry of crystals which was collected and dried in vacuo to give the title ester (0.08 g), whose physical and spectroscopic properties resembled those as described in Example 8.

Example 50

p-Nitrobenzyl (5RS)-2-methylpen-2-em-3-carboxylate

A solution of triethylamine (0.4 ml, 3.0 mmole) in tetrahydrofuran (15 ml) at $-15^{o}$ was saturated with hydrogen sulphide and then added dropwise to a stirred solution of the product of Example 99 (0.600 g, 1.43 mmole) in tetrahydrofuran (10 ml) at $-15^{o}$. After 15 minutes the suspension was treated with ether (30 ml) and 0.2 M pH7 phosphate buffer (20 ml). The upper phase was separated off, washed with brine, dried and concentrated until crystallisation began. The suspension was cooled to $-10^{o}$ and then filtered. The crystals were washed with ether and dried to give the title ester (0.185 g) $[\alpha]_D^{22} +2^{o}$ (c 1.00, $CHCl_3$).

Example 51

p-Nitrobenzyl (5RS)-2-acetoxymethylpen-2-em-3-carboxylate

A solution of triethylamine (0.205 ml) in dry DMF (5 ml) was stirred at $-20^{o}$ under nitrogen and hydrogen sulphide (1.8 M solution in DMF, 0.61 ml) was added, followed over 2 minutes, by a solution of

the product of Example 113 (0.35 g) in DMF (5 ml). The solution was stirred for a further 30 minutes at -20° then poured into a stirred mixture of N.HCl and ethyl acetate (60 ml of each) and the organic phase was separated, washed with water and brine (20 ml of each), dried, and evaporated to a foam (245 mg). This foam was purified by column chromatography (C) (10 g) using 1% ether in dichloromethane as eluant. Collection of the appropriate fractions and evaporation to dryness gave the title compound (45 mg) as a gum, $\nu_{max}$ ($CHBr_3$) 1790 (β-lactam), 1712 ($CO_2R$) and 1522 and 1346 $cm^{-1}$ ($NO_2$).

Example 52

Potassium (5RS)-2-methoxymethylpen-2-em-3-carboxylate

A solution of the product of Example 27 (1.148 g) in ethyl acetate (22 ml) and ethanol (22 ml) was added to a slurry of 20% Pd-C (2.286 g) in ethyl acetate (14 ml). The mixture was hydrogenated for ca. 3½ hours.

The reaction mixture was filtered through kieselguhr and the filtrate was treated with a solution of potassium ethyl hexanoate in ethyl acetate (0.69 M, 2.4 ml).

The mixture was reduced to ca. 20 ml and ether was added dropwise to assist precipitation.

The resultant suspension was cooled and ether was added (x2).

Filtration of the mixture, washing of the filter-bed with ether, and drying in vacuo afforded the title compound (0.351 g), $\lambda_{max}$ (EtOH) 259 nm (ε 3,600) and 299 nm (ε 4,050) and $\nu_{max}$ (Nujol) 1750 $cm^{-1}$ (β-lactam).

Example 53

Diphenylmethyl (5RS)-2-(2-hydroxyethyl)pen-2-em-3-carboxylate

A solution of the product of Example 8 (1.05 g) in ethyl acetate: ethanol (1:3.5; 320 ml) containing 10% Pd-C (4.2 g) was shaken and hydrogenated at room temperature and at 1 atmosphere pressure. After 25 minutes the mixture was filtered through kieselguhr and the pad was washed with ethyl acetate. The filtrate was evaporated to ca. 110 ml and then treated with a solution of diphenyldiazomethane in ethyl acetate (0.24 M, 8.33 ml). The solution was allowed to stand for 16 hours and then evaporated to low volume. Silica gel (5 g) was added and the mixture evaporated to dryness. The powder was transferred to the top of a dry silica gel column and eluted with ethyl acetate-petroleum ether (40-60$^{o}$) mixtures. Appropriate fractions were combined and evaporated to leave an oil (0.325 g) which crystallised from ethyl acetate/ether to afford the title ester (0.185 g), m.p. 103.5-107.0$^{o}$, $\lambda_{max}$ (EtOH) 317.5 nm ($\varepsilon$ 8,660).

Example 54

p-Nitrobenzyl (5RS)-2-(2-N-methylthiocarbamoyloxyethyl)-pen-2-em-3-carboxylate

Bis(tri-n-butyltin)oxide (2.1 ml) was added to a solution/suspension of the product of Example 8 (1.75 g) in ethyl acetate (67 ml) containing N-methylisothiocyanate (2.80 g). After 48 hours the solution was diluted with ethyl acetate, then washed with saturated brine and dried. The filtered solution was evaporated to low

volume, silica gel (10 g) was added and the mixture was evaporated to dryness. The powder was transferred to the top of a dry silica gel column and eluted with ethyl acetate-petroleum ether (40-60°) mixtures. Appropriate fractions were combined and evaporated to give an oil (0.834 g) which crystallised from ethyl acetate/ether to afford the title ester (0.628 g), $\lambda_{max}$ ($CHCl_3$) 315 nm (ε 7,960), $\nu_{max}$ ($CHBr_3$), 1790 $cm^{-1}$ (β-lactam).

Example 55

Potassium (5RS)-2-(2-N-methylthiocarbamoyloxyethyl)pen-2-em-3-carboxylate

A solution of the product of Example 54 (0.600 g) in ethyl acetate: ethanol (1:1, 50 ml) containing 10% Pd-C (2.4 g) was shaken and hydrogenated at room temperature and 1 atmosphere pressure. After 40 minutes the mixture was filtered through kieselguhr and the pad was washed with ethyl acetate followed by ethanol. The filtrate was treated with a solution of potassium 2-ethylhexanoate in ethyl acetate (0.69 M, 1.23 ml) and the resulting solution was concentrated to low volume and, with stirring, was diluted with ether to afford a solid which was collected and dried *in vacuo* to give the title salt (0.100 g), $\lambda_{max}$ 301.5 nm (ε 4,050), $\nu_{max}$ (Nujol) 1766 and 1752 $cm^{-1}$ (β-lactam).

Example 56

p-Nitrobenzyl (5RS)-2-[2-(2-chloroacetyl)oxyethyl]pen-2-em-3-carboxylate

Chloroacetyl chloride (1.13 g) was added to a

stirred suspension/solution of the product of Example 8 (1.75 g) in ethyl acetate (100 ml) maintained at 5° and containing dry pyridine (2.7 ml). The reaction mixture was allowed to warm to room temperature and after 1 hour was diluted with ethyl acetate and washed successively with 0.5 N aqueous HCl, 0.5 N aqueous $NaHCO_3$, water and saturated brine, and then dried. Evaporation gave a solid (2.0 g) which was recrystallised from ethyl acetate/ether to afford the title ester (1.50 g), m.p. 95.0-97.0°, $\lambda_{max}$ ($CHCl_3$) 318 nm (ε 9,400).

Example 57

Potassium (5RS)-2-[2-(2-chloroacetyl)oxyethyl]pen-2-em-3-carboxylate

A solution of the product of Example 56 (1.05 g) in ethyl acetate: ethanol (1:1, 100 ml) containing 10% Pd-C (4.2 g) was shaken and hydrogenated at room temperature and 1 atmosphere pressure. After 28 minutes the mixture was filtered through kieselguhr, the pad was washed with ethyl acetate followed by ethanol, and the filtrate was evaporated to low volume and treated with a solution of potassium 2-ethylhexanoate in ethyl acetate (0.69 M, 2.17 ml). Ether was added dropwise to the stirred solution and the resulting solid was collected and dried in vacuo to afford the title salt (0.417 g), $\lambda_{max}$ (pH 6 buffer) 302 nm (ε 4,600), $\nu_{max}$ (Nujol) 1758cm$^{-1}$ (β-lactam).

Example 58

p-Nitrobenzyl (5RS)-2-(2-benzoyloxyethyl)pen-2-em-3-carboxylate

Benzoyl chloride (1.16 ml) was added to a stirred solution/suspension of the product of Example 8 (1.75 g) in ethyl acetate (100 ml) maintained at 5$^{o}$ and containing dry pyridine (2.7 ml). The reaction mixture was allowed to warm to room temperature and after 22 hours was diluted with ethyl acetate and washed successively with 0.5 N aqueous HCl, water, and saturated brine and then dried. Silica gel (10 g) was added to the filtered organic solution and the mixture was evaporated to dryness. The impregnated silica gel was transferred to the top of a dry silica gel column and eluted with ethyl acetate-petroleum ether (40-60$^{o}$) mixtures. Appropriate fractions were combined and evaporated to give an oil (1.21 g) which crystallised from ethyl acetate/ether to afford the title ester (0.841 g), m.p. 119.5-121.5$^{o}$, $\lambda_{max}$ ($CHCl_3$) 318 nm ($\varepsilon$ 9,680).

Example 59

Potassium (5RS)-2-(2-benzoyloxyethyl)pen-2-em-3-carboxylate

A solution of the product of Example 58 (0.750 g) in ethyl acetate: ethanol (1:1, 60 ml) containing 10% Pd-C (3.0 g) was shaken and hydrogenated at room temperature and 1 atmosphere pressure. After 35 minutes the mixture was filtered through kieselguhr and the pad was washed with ethyl acetate followed by ethanol. The filtrate was evaporated to low volume

and then treated with a solution of potassium 2-ethylhexanoate in ethyl acetate (0.69 M, 1.5 ml). Dropwise addition of ether to the stirred solution gave a solid which was collected and dried *in vacuo* to afford the title salt (0.223 g), $\lambda_{max}$ (pH 6 buffer) 302.5 nm ($\varepsilon$ 5,250), $\nu_{max}$ (Nujol) 1758 $cm^{-1}$ ($\beta$-lactam).

Example 60

Potassium (5RS)-2-(2-hydroxyethyl)pen-2-em-3-carboxylate-1-oxide

A solution/suspension of the product of Example 10 (0.50 g) in ethyl acetate: ethanol (1:1, 150 ml) containing 10% Pd-C (0.75 g) was shaken and hydrogenated at ambient temperature and 1 atmosphere pressure. After 38 minutes the mixture was filtered through kieselguhr and the pad was washed with ethyl acetate: ethanol (1:1). The filtrate was treated with a solution of potassium 2-ethylhexanoate (0.162 g) in ethyl acetate (10 ml) and the mixture was evaporated to low volume. Dropwise addition of ether to the stirred solution gave a solid. The supernatant was decanted off and the solid was washed with ether (twice) and then dried *in vacuo* to afford the title salt (0.260 g), $\lambda_{max}$ (pH 6 buffer) 270 nm ($\varepsilon$ 3,400), $\nu_{max}$ (Nujol) 1786 $cm^{-1}$ ($\beta$-lactam).

Example 61

Acetoxymethyl (5RS)-2-(2-carbamoyloxyethyl)pen-2-em-3-carboxylate

A solution of the product of Example 19 (0.426 g) in dry DMF (5 ml) was treated with an excess of acetoxymethyl iodide dissolved in ether (10 ml). After

45 minutes the mixture was partitioned between ethyl acetate and saturated brine. The organic phase was washed with water followed by saturated brine and then dried. Evaporation gave an oil (0.226 g) which crystallised from ethyl acetate-acetone to afford the title ester (0.072 g), m.p. 148-150°, $\lambda_{max}$ ($CHCl_3$) 321 nm (ε7,400).

Example 62

p-Nitrobenzyl (5RS)-2-(2-carbamoyloxyethyl)pen-2-em-3-carboxylate-1-oxide

A solution of 3-chloroperoxybenzoic acid (1.083 g) in dry dichloromethane (26 ml) was added dropwise to a stirred solution/suspension of the product of Example 17 (1.882 g) in dry dichloromethane (33 ml) at 0°. After 5 minutes the resulting solution was diluted with dichloromethane and washed with saturated brine and 0.5 N aqueous $NaHCO_3$. The aqueous phase was extracted with dichloromethane and the combined organic solutions were washed with saturated brine and then dried. Evaporation gave a foam (1.92 g) which crystallised from ethyl acetate to afford the title sulphoxide (1.15 g), m.p. 110-113°, $\lambda_{max}$ ($CHCl_3$) 283 nm (ε 10,500).

Example 63

Potassium (5RS)-2-(2-carbamoyloxyethyl)pen-2-em-3-carboxylate-1-oxide

A solution of the product of Example 62 (1.035 g) in ethyl acetate:ethanol (1:1, 300 ml) containing 10% Pd-C (1.553 g) was shaken and hydrogenated at room

temperature and 1 atmosphere pressure. After 35 minutes the mixture was filtered through kieselguhr and the pad was washed with ethyl acetate:ethanol (1:1). The filtrate was treated with a solution of potassium 2-ethylhexanoate (0.323 g) in ethyl acetate (30 ml) and the resulting fine suspension was evaporated to low volume (ca. 40 ml) and, with stirring, was diluted with ether. The supernatant was decanted from the resulting solid and the latter was washed with ether (twice) and then dried in vacuo to afford the title salt (0.636 g), $\lambda_{max}$ (pH 6 buffer) 265 nm ($\varepsilon$ 3,700), $\nu_{max}$ (Nujol) 1782 $cm^{-1}$ ($\beta$-lactam).

Example 64

p-Nitrobenzyl (5RS)-2-ethylpen-2-em-3-carboxylate

A solution of the product of Example 114 (0.09 g) in dry THF (0.6 ml) was added dropwise, under nitrogen, to a stirred solution of triethylamine (0.11 ml) in dry THF (5 ml) maintained at 0° and through which hydrogen sulphide was being passed. After 20 minutes the mixture was partitioned between ether and water. The organic phase was washed with 0.5 N aqueous HCl followed by water and then dried. Evaporation gave an oil which was chromatographed on a silica gel plate eluting with ether:petroleum ether (40-60°) (2:1). The major band was isolated, then eluted with acetone, and the organic solution evaporated to give the title ester (0.01 g) whose spectroscopic properties resembled those as described in Example 30.

Example 65

p-Nitrobenzyl (5RS)-2-ethylpen-2-em-3-carboxylate

A solution of the product of Example 111 (0.055 g) in dry THF (0.4 ml) was added dropwise, under nitrogen, to a stirred solution of triethylamine (0.08 ml) in dry THF (4 ml) maintained at $0^{o}$ and through which hydrogen sulphide was being bubbled. After 20 minutes the mixture was poured into ether (100 ml) and water (50 ml). The organic phase was washed with water, 0.5 N aqueous HCl, water, and saturated brine and then dried. Evaporation gave an oil which was chromatographed on a silica gel plate eluting with ether: petroleum ether ($40-60^{o}$) (2:1). The major band was isolated, then eluted with acetone, and the organic solution was evaporated to give the title ester (0.009 g) whose spectroscopic properties resembled those as described in Example 30.

Example 66

p-Nitrobenzyl (4'RS)-3-hydroxy-2-(2'-oxo-4'-p-tolylthio-azetidin-1'-yl)but-2-enoate

Magnesium powder (3.438 g, 0.143 g atom) and iodine (2.01 g, 7.9 mmole) were vigorously stirred together in dry tetrahydrofuran (130 ml) and glacial acetic acid (0.6 ml) for 10 minutes. A solution of the product of Preparation 5 (6.545 g, 14 mmole) in tetrahydrofuran (130 ml) and glacial acetic acid(1.1 ml) was added over 7 minutes, and after a further 5 minutes the suspension was filtered through cotton wool. The filtrate was evaporated to a foam, which was partitioned between dichloromethane (1 l.) and 20% aqueous sodium metabisulphite (1 l.). The organic phase was separated and washed successively with water and brine, and evaporation of the dried organic phase gave the title compound (5.92 g). A purer sample obtained by preparative layer chromatography (dichloromethane: ether (9:1), had $\lambda_{max}$($CHCl_3$) 261 nm ($E^{1\%}_{1cm}$ 389), inflection at 268 nm ($E^{1\%}_{1cm}$ 370).

Example 67

p-Nitrobenzyl (4'RS)-3-hydroxy-2-[2'-oxo-4'-p-tolylthio-azetidin-1'-yl]hex-2-enoate

A suspension of magnesium (1.68 g, 0.0691 g atom) in dry tetrahydrofuran (100 ml) containing acetic acid (ca. 5 ml) was stirred at 0° and iodine (0.877 g, 3.455 mmole) was added. The mixture was stirred for 10 minutes at 0 to ca. 23° and then a solution of the product of Preparation 15 (3.394g)in tetrahydrofuran(40ml) was added. Stirring was continued for a further 20

minutes (at ca. 23°), and the reaction mixture was filtered through kieselguhr. The filtrate was evaporated to dryness in vacuo, and the residue partitioned between dichloromethane and aqueous sodium metabisulphite (150 ml of each). The organic layer was separated, washed with water, and brine (100 ml of each) and dried, and the solvent removed to give the title compound (2.750 g), $\nu_{max}$($CHBr_3$) values include 1758 $cm^{-1}$ (β-lactam).

Example 68

p-Nitrobenzyl (2'R)-2-(2'-benzothiazol-2-yldithio-4'-oxoazetidin-1'-yl)-3-hydroxybut-2-enoate

The product of Preparation 18 (22.7 g, 45.2 mmole) in dichloromethane (200 ml) at -78° was stirred and a stream of ozonised oxygen was passed through for 2 hours. The solution was then purged with oxygen for 20 minutes, warmed to -10° and stirred vigorously with aqueous sodium metabisulphite for 10 minutes. The organic layer was separated, dried and evaporated to a foam. This was chromatographed (B) (0 to 15% ethyl acetate in toluene) and appropriate fractions were combined and evaporated to give the title ester (10.42g), $[\alpha]_D^{21}$ -78° (c 1.07, $CHCl_3$).

Example 69

p-Nitrobenzyl (2'ξ)-2-(2'-benzothiazol-2-ylthio-4'-oxoazetidin-1'-yl)-3-hydroxybut-2-enoate

The product of Example 68 (2.01g, 4.0 mmole) in dichloromethane (20ml) at -15° was stirred and treated with trimethyl phosphite (0.48ml, 4.0 mmole) added dropwise over

5 minutes. After 15 minutes the solution was evaporated and the residue was chromatographed (B) (0 to 12% ethyl acetate in toluene). Evaporation of fractions containing the main product gave the title ester (1.16g), $[\alpha]_D^{27}$ +7° (c 0.97, $CHCl_3$).

Example 70

p-Nitrobenzyl (4'RS)-3-hydroxy-4-methoxy-2-(2'-oxo-4'-p-tolythioazetidin-1'-yl)but-2-enoate

n-Butyl lithium in hexane (1.3 molar solution, 3.5 ml, 4.55 mmole) was added dropwise under nitrogen to a cooled (-70°) solution of hexamethyldisilazane (0.97 ml, 4.55 mmole) in dry tetrahydrofuran (15 ml).

A solution of the product of Preparation 23 (1.005 g, 2.6 mmole) in dry tetrahydrofuran (15 ml) was added to the above cooled and stirred solution over 1 minute.

Stirring was continued for a further 3 minutes at -70° and then methoxyacetyl chloride (0.21 ml, 0.254 g, 2.34 mmole) was added dropwise.

After 22 minutes the reaction mixture was poured into ethyl acetate and 2N hydrochloric acid.

The organic layer was washed with aqueous sodium bicarbonate and twice with brine and dried (sodium sulphate) and evaporated to dryness to give the title compound (1.126 g). τ ($CDCl_3$) values include 4.90 (m, 4'-H) and 6.59 (s, $CH_2OCH_3$).

Example 71

p-Nitrobenzyl 3-hydroxy-2-(4-methylthio-2-oxoazetidin-1-yl) pent-2-enoate

A solution of p-nitrobenzyl 2-ethylclav-2-em-3-carboxylate (14.59 g) in dry THF (40 ml) was treated with a solution of methanethiol in THF (3.23 M, 85 ml) and was stood at 19° for 7 days. The solution was evaporated to an oil which was chromatographed on a column of silica gel eluting with ether-petroleum ether (40 - 60°) (2:1). Appropriate fractions were combined and evaporated to leave the title ester (9.36 g), m.p. 73.1°, $\lambda_{max}$ (EtOH) 274 nm (ε 22,350).

Example 72

p-Nitrobenzyl 2-(4-ethylthio-2-oxoazetidin-1-yl)-3-hydroxy pent-2-enoate

A solution of p-nitrobenzyl 2-ethylclav-2-em-3-carboxylate (12.60 g) in dry THF (95 ml), containing ethanethiol (37 ml), was stood at 30° for 6 days. The solution was evaporated to an oil which was chromatographed on a silica gel column eluting with ether-petroleum ether (40-60°) (2:1). Appropriate fractions were combined and evaporated to afford the title ester (9.68 g), m.p. 66.5°, $\lambda_{max}$(EtOH) 275 nm (ε 21,700).

Example 73

p-Nitrobenzyl 2-(4-n-butylthio-2-oxoazetidin-1-yl)-3-hydroxy-pent-2-enoate

A solution of p-nitrobenzyl 2-ethylclav-2-em-3-carboxylate (0.318 g) in dry THF (5 ml) containing n-butanethiol (0.54 ml) was heated to 75° for 18 hr. The reaction mixture was allowed to cool and was then evaporated to leave the title ester (0.39 g), $\lambda_{max}$ (EtOH) 272 nm ($\varepsilon$ 26,700), $\nu_{max}$ ($CHBr_3$) 1760 $cm^{-1}$ ($\beta$-lactam).

Example 74

p-Nitrobenzyl 2-(4-n-butylthio-2-oxoazetidin-1-yl)-3-hydroxy-5-(tetrahydropyran-2-yloxy)pent-2-enoate

A solution of p-nitrobenzyl 2-[2-(tetrahydropyran-2-yloxy)ethyl]clav-2-em-3-carboxylate (2.52 g) in dry THF (30 ml), containing n-butanethiol (3.86 ml), was heated to 75° for 4.5 hours. The reaction mixture was allowed to cool and then evaporated to afford the title ester (3.05g), $\nu_{max}$($CHBr_3$) 1758 ($\beta$-lactam), 1722 and 1656 ($CO_2R$), 1520 and 1345 $cm^{-1}$ ($NO_2$).

Example 75

p-Nitrobenzyl 2-(4-n-butylthio-2-oxoazetidin-1-yl)-5-(1-ethoxyethoxy)-3-hydroxypent-2-enoate

A solution of p-nitrobenzyl 2-[2-(1-ethoxyethoxy)-ethyl]clav-2-em-3-carboxylate (3.0 g) in dry THF (25 ml) containing n-butanethiol (3.94 ml) was heated under reflux for 17 hours; then concentrated to an oil to which toluene was added and the mixture evaporated to afford the title ester (3.62g), $\nu_{max}$ ($CHBr_3$) 1758 ($\beta$-lactam), 1720 and 1658 ($CO_2R$), 1520 and 1344 $cm^{-1}$($NO_2$).

Example 76

p-Nitrobenzyl (4'RS)-3-methanesulphonyloxy-2-(2'-oxo-4'-p-tolylthioazetidin-1'-yl)but-2-enoate

A solution of the product of Example 66 (0.384 g, 0.9 mmole) in dry dichloromethane (30 ml) was treated with methanesulphonyl chloride (0.1 ml, 1.35 mmole), followed by triethylamine (0.18 ml, 1.35 mmole). After 10 minutes the solution was washed successively with 0.5 N hydrochloric acid, water and brine, and the organic phase dried, and evaporated to give the title compound (0.460 g). A similar sample had $\lambda_{max}$(EtOH) 260 nm ($E^{1\%}_{1cm}$ 338).

Example 77

p-Nitrobenzyl (4'RS)-3-dimethoxyphospinyloxy-2-(2'-oxo-4'-p-toly-thioazetidin-1'-yl)but-2-enoate

A solution of the product of Preparation 5 (0.343 g, 0.74 mmole) and trimethylphosphite (0.46 ml, 3.7 mmole) in dry benzene (15 ml) was refluxed for 1 hour, after which time a further aliquot of trimethylphosphite (0.46 ml) was added, and the reflux continued for 15 hours. The solution was evaporated to dryness and the residue purified by preparative layer chromatography (dichloromethane:ether= 9:1) to give the title compound (0.217 g). A sample prepared in a similar manner had $\lambda_{max}$ ($CHCl_3$) 260 nm ($E^{1\%}_{1cm}$ 334).

Example 78

p-Nitrobenzyl (4'RS)3-methanesulphonyloxy-2-[2'-oxo-4'-p-tolylthioazetidin-1'-yl]hex-2-enoate

A solution of triethylamine (1.24 ml, 8.895 mmole) in dry dichloromethane (20 ml) was added dropwise, over 5 minutes, to a stirred solution of the compound of Example 67 (2.705 g) and methanesulphonyl chloride(0.69ml), 8.895 mmole) in dry dichloromethane (100 ml) at 0°. Stirring was continued for 20 minutes (at 0 to ca. 23° ), and 2N hydrochloric acid (100 ml) was added. After stirring for 5 minutes, the organic layer was separated, washed with water, and brine (100 ml of each), and dried, and the solvent removed to give the title compound(3.105g), $\nu_{max}$ ($CHBr_3$) values include 1762 $cm^{-1}$ (β-lactam).

Example 79

p-Nitrobenzyl (2'R)-2-(2'-benzothiazol-2-yldithio-4'-oxoazetidin-1'-yl)-3-methanesulphonyloxybut-2-enoate

The product of Example 68 (2.01 g, 4.0 mmole) in dichloromethane (20 ml) at -15° was stirred and treated with methanesulphonyl chloride (0.46 ml, 6.0 mmole) and triethylamine (0.46 ml, 3.3 mmole). The solution was stirred at -15° for 20 minutes, warmed to 25° over 40 minutes, then cooled to -15° and treated with more triethylamine (0.23 ml, 1.65 mmole) and methanesulphonyl chloride (0.23 ml, 3.0 mmole). After 20 minutes the solution was warmed to 0°, washed with dilute sulphuric acid, dried and evaporated. The residue was chromatographed (B) (0 to 15% ethyl acetate in toluene) and

appropriate fractions were combined and evaporated to give the <u>title ester</u> (1.46 g), m.p. 65°, $[\alpha]_D^{21}$ - 63° (<u>c</u> 1.02, $CHCl_3$).

<u>Example 80</u>

<u>p-Nitrobenzyl (2'ξ)-2-(2'-benzothiazol-2-ylthio-4'-oxoazetidin-1'-yl)-3-methanesulphonyloxybut-2-enoate</u>

(a) <u>from the disulphide</u>

The product of Example 79 (0.58 g, 1.0 mmole), in dichloromethane (20 ml) at -15° was stirred and treated with trimethyl phosphite (0.13 ml, 1.1 mmole). The solution was allowed to warm to 25° over 1 hour and was then evaporated to an oil. This was chromatographed (B) (0 to 30% ethyl acetate in toluene) and evaporation of appropriate fractions gave the <u>title ester</u> (0.085 g), $[\alpha]_D^{24}$+13° (<u>c</u> 1.03, $CHCl_3$).

(b) <u>from the 3-hydroxybut-2-enoate</u>

The product of Example 69 (1.15 g, 2.3 mmole) in dichloromethane (20 ml) at -15° was stirred and treated with triethylamine (0.30 ml, 2.2 mmole) and then with methanesulphonyl chloride (0.30 ml, 4.0 mmole). The solution was allowed to warm to 25° over 3/4 hour; it was then cooled to -15° and more triethylamine (0.30ml) and methanesulphonyl chloride (0.30 ml) were added. After 10 minutes at -15° the solution was washed with 2N sulphuric acid, then dried and evaporated to an oil. This was chromatographed (B) (0 to 15% ethyl acetate in toluene) and evaporation of appropriate fractions gave the <u>title ester</u> (0.36 g) $[\alpha]_D$+12° (<u>c</u> 0.99, $CHCl_3$).

Example 81

p-Nitrobenzyl 2-(4-n-butylthio-2-oxoazetidin-1-yl)-3-trifluoromethanesulphonyloxypent-2-enoate

Trifluoromethanesulphonic anhydride (0.355 ml) was added dropwise to a stirred solution of the product of Example 73 (0.817 g) in dry dichloromethane (18 ml) containing triethylamine (0.345 ml) at 0°. After 5 minutes the solution was diluted with dichloromethane (30 ml) and washed with 0.5N aqueous HCl followed by water, then dried, and evaporated to afford the title ester (0.98 g), $\nu_{max}$ ($CHBr_3$) 1772 (β-lactam), 1728 ($CO_2R$), 1520 and 1345 $cm^{-1}$ ($NO_2$).

Example 82

p-Nitrobenzyl(4'RS)-4-methoxy-3-methanesulphonyloxy-2-(2'-oxo-4'-p-tolylthioazetidin-1'-yl)-but-2-enoate

A cooled (-5°) stirred solution of the product of Example 70 (0.986 g, 2.15 mmole) in dry dichloromethane (36 ml) was treated with methanesulphonyl chloride (0.27 ml, 3.44 mmole) followed by dropwise addition of triethylamine (0.39 ml, 2.8 mmole). The reaction solution was stirred at ca 0° for 30 minutes then a second portion of triethylamine (0.06 ml, 0.43 mmole) was added dropwise. After 17 minutes the reaction mixture was poured into 2N hydrochloric acid and dichloromethane. The organic phase was washed with aqueous sodium bicarbonate and twice with brine. The aqueous layers were extracted with dichloromethane. The combined organic extracts were dried and evaporated to dryness (1.207 g). The product was purified by

chromatography (B) (deactivated with 5% water) (toluene: ethyl acetate 20:1, 10:1). The appropriate fractions were combined, evaporated to dryness and re-evaporated from dichloromethane to give the title compound (0.623 g). τ ($CDCl_3$) values include 4.6-4.9 (m, $CH_2C_6H_4NO_2$, 4'-H) and 5.5-6.0 (m, $\underline{CH_2}OCH_3$).

## Example 83

p-Nitrobenzyl 3-methanesulphonyloxy-2-(4-methylthio-2-oxoazetidin-1-yl)pent-2-enoate

Methanesulphonyl chloride (0.03 ml) was added dropwise to a stirred solution of the product of Example 71 (0.122 g) in dry dichloromethane (5 ml) containing triethylamine (0.051 g) at 0°. After 20 minutes the reaction mixture was diluted with dichloromethane and washed with 0.5 N aqueous HCl followed by saturated brine. The organic phase was dried and then evaporated to an oil which was chromatographed on silica gel plates eluting with ether. The major band was isolated and stirred with ethyl acetate for 10 min. The mixture was filtered and the filtrate evaporated to afford the title ester (0.08 g) $\lambda_{max}$ 264.5 nm (ε 15,600), $\nu_{max}$ ($CHBr_3$) 1766 $cm^{-1}$ (β-lactam).

## Example 84

p-Nitrobenzyl 2-(4-ethylthio-2-oxoazetidin-1-yl)-3-methanesulphonyloxy-pent-2-enoate

Methanesulphonyl chloride (0.47 ml) was added dropwise to a stirred solution of the product of Example

72 (1.90 g) in dichloromethane (30 ml) containing triethylamine (0.758 g) at 0°. After 30 mins the reaction mixture was diluted with dichloromethane (200 ml) and washed with 0.5 N aqueous HCl followed by saturated brine. The organic phase was dried and then evaporated to an oil which was chromatographed on a column of silica gel eluting with ether-petroleum ether (40 - 60°) (2:1). Appropriate fractions were combined and evaporated to afford the title ester (1.21 g), $\lambda_{max}$(EtOH) 264 nm ($\varepsilon$14,500), $\nu_{max}$($CHBr_3$) 1765cm$^{-1}$ ($\beta$-lactam).

Example 85

p-Nitrobenzyl 2-(4-n-butylthio-2-oxoazetidin-1-yl)-3-methanesulphonyloxy-pent-2-enoate

Methanesulphonyl chloride (4.30 ml) was added dropwise to a stirred solution of the product of Example 73 (20.42 g) in dichloromethane (150 ml) containing triethylamine (10.45 ml) at 0°. After 40 min. the reaction mixture was diluted with dichloromethane (200 ml) and washed with 0.5 N aqueous HCl (300 ml) followed by water (3 x 200 ml). The organic phase was dried and evaporated to an oil (23.36 g). A portion of the crude product (0.48 g) was chromatographed on silica gel plates eluting with ether-petroleum ether (40-60°) (2:1). The major band was isolated and stirred with dichloromethane. Evaporation of the filtered organic solution gave the title ester (0.097 g), $\lambda_{max}$(EtOH)

264 nm (ε 18,150) $\nu_{max}$ ($CHBr_3$) 1766 $cm^{-1}$ (β-lactam).

Example 86

p-Nitrobenzyl 2-(4-n-butylthio-2-oxoazetidin-1-yl)-3-methanesulponyloxy-5-(tetrahydropyran-2-yloxy)pent-2-enoate

Methanesulphonyl chloride (0.51 ml) was added dropwise to a stirred solution of the product of Example 74 (3.05 g) in dry dichloromethane (20 ml) containing triethylamine (1.25 ml), at 0°. After 30 minutes the reaction mixture was diluted with dichloromethane and washed with 0.1N aqueous HCl followed by saturated brine and then dried. Evaporation gave the title ester (3.52 g), $\nu_{max}$($CHBr_3$) 1766 (β-lactam), 1728 ($CO_2R$), 1522 and 1348 $cm^{-1}$ ($NO_2$).

Example 87

p-Nitrobenzyl 3-chloro-2-(4-methylthio-2-oxoazetidin-1-yl)-pent-2-enoate

Phosphorus trichloride (0.18 ml) was added slowly to a stirred solution of the product of Example 71 (0.367g) in dry DMF(5ml) at 0°.The dark coloured solution was stood at 19° for 20 hours and then partitioned between ether (100 ml) and water (30 ml). The aqueous phase was extracted further with ether (3 x 100 ml) and the combined organic extracts were washed with saturated brine and then dried. Evaporation gave an oil which was chromatographed on silica gel plates eluting with ether-petroleum ether (40-60°) (2:1). The appropriate band was isolated and eluted with acetone. Evaporation

afforded the title ester (0.08 g), $\lambda_{max}$ (EtOH) 265 nm ($\varepsilon$ 17,200), $\nu_{max}$ ($CHBr_3$) 1762 $cm^{-1}$ ($\beta$-lactam).

Example 88

p-Nitrobenzyl 3-chloro-2-(4-ethylthio-2-oxoazetidin-1-yl) pent-2-enoate

The title ester (0.028 g) was prepared from the product of Example 72 (0.114 g) and phosphorus trichloride (0.041 g) by the method as described in Example 87; m.p. 60.8°, $\lambda_{max}$ (EtOH) 264 nm ($\varepsilon$ 13,900).

Example 89

p-Nitrobenzyl 2-(4-ethylthio-2-oxoazetidin-1-yl)-3-methoxypent-2-enoate

A solution of the product of Example 72 (0.285 g) in ethyl acetate (10 ml) was treated with an excess of ethereal diazomethane at 0°. After 15 minutes a fast stream of nitrogen was passed for 5 minutes through the reaction mixture which was then evaporated to afford the title ester (0.29 g), $\lambda_{max}$ (EtOH) 266 nm ($\varepsilon$ 16,900), $\nu_{max}$ ($CHBr_3$) 1752 $cm^{-1}$ ($\beta$-lactam).

Example 90

p-Nitrobenzyl 3-methoxy-2-(4-methylthio-2-oxoazetidin-1-yl)-pent-2-enoate

A solution of the product of Example 71 (0.46 g) in ethyl acetate (20 ml) was treated with excess ethereal diazomethane at 0°. After 30 minutes a fast stream of

nitrogen was passed for 5 minutes through the reaction mixture which was then evaporated to give the title ester (0.48 g), $\lambda_{max}$(EtOH) 264.5 nm (ε 17,200), $\nu_{max}$ ($CHBr_3$) 1754 $cm^{-1}$ (β-lactam).

Example 91

p-Nitrobenzyl 2-(4-n-butylthio-2-oxoazetidin-1-yl)-5-(1-ethoxyethoxy)-3-methanesulphonyloxypent-2-enoate

Methanesulphonyl chloride (0.19 ml) was added to a stirred solution of the product of Example 75 (0.99 g) in dry dichloromethane (12 ml) containing triethylamine (0.41 ml) at 0°. After 35 minutes the mixture was allowed to warm to room temperature and stirred for a further 60 minutes. The resulting mixture was diluted with dichloromethane and washed successively with 0.5 N aqueous HCl, water, and brine, then dried and evaporated to an oil which was chromatographed on a dry silica gel column eluting with ether. Appropriate fractions were combined and evaporated to give the title ester (0.472 g), $\lambda_{max}$(EtOH) 265 nm (ε 16,600), $\nu_{max}$($CHBr_3$) 1765 $cm^{-1}$ (β-lactam).

Example 92

p-Nitrobenzyl 2-(4-n-butylthio-2-oxoazetidin-1-yl)-5-(1-ethoxyethoxy)-3-methoxypent-2-enoate

A solution of the product of Example 75 (2.45 g) in ethyl acetate (15 ml) was treated with an excess of ethereal diazomethane at 0°. After 7 minutes a fast

stream of nitrogen was passed for 5 minutes through the reaction mixture which was then evaporated to an oil and chromatographed on a dry silica gel column eluting with ether. Appropriate fractions were combined and evaporated to afford the title ester (0.726 g), $\lambda_{max}$(EtOH) 267 nm ($\varepsilon$19,750) $\nu_{max}$ ($CHBr_3$) 1750 $cm^{-1}$ ($\beta$-lactam).

Example 93

p-Nitrobenzyl 2-(4-n-butylthio-2-oxoazetidin-1-yl)-3-(4-toluenesulphonyloxy)pent-2-enoate

4-Toluenesulphonyl chloride (0.247 g) followed by triethylamine (0.150 g) were added to a stirred, ice-cooled solution of the product of Example 73 (0.408 g) in dry dichloromethane (10 ml). After 10 minutes the solution was allowed to warm to room temperature and stirred for a further 18 hours. The reaction mixture was diluted with dichloromethane and washed successively with 0.5N aqueous HCl, water (twice) and brine, and then dried. Evaporation gave an oil which was chromatographed on a dry silica gel column eluting with ether-petroleum ether (40-60°) (3:1). Appropriate fractions were combined and evaporated to afford the title ester (0.116 g), $\nu_{max}$ ($CHBr_3$) 1760 ($\beta$-lactam), 1700 ($CO_2R$), 1528 and 1350 $cm^{-1}$ ($NO_2$).

Example 94

p-Nitrobenzyl (2'RS)-2-(2'-chloro-4'-oxoazetidin-1'-yl)-3-methanesulphonyloxybut-2-enoate

A 5% solution of chlorine (2.96 mmole) in dry dichloromethane (4.2 ml) was added to a stirred and cooled (ca. 0°) solution of the product of Example 76 (0.500 g, 0.99 mmole) in dry dichloromethane (40 ml). After 15 minutes the solution was evaporated to a small volume (ca. 5 ml) and purified by chromatography (B) (0 to 5% ether in dichloromethane) to give the title compound (0.233 g). A similar sample had $\lambda_{max}$(EtOH) 262 nm ($E^{1\%}_{1\ cm}$ 315).

Example 95

p-Nitrobenzyl (2'RS)-2-(2'-chloro-4'-oxoazetidin-1'-yl)-3-(dimethoxyphosphinyloxy)but-2-enoate

A solution of chlorine in dry dichloromethane (5%, 0.55 ml, 0.39 mmole of chlorine) was added to a stirred and cooled (ca. 0°) solution of the product of Example 77 (0.69g, 0.13 mmole) in dry dichloromethane (5 ml). After 5 minutes the solution was evaporated to dryness in vacuo and the residue was purified by preparative layer chromatography (dichloromethane: ether = 9:1). The appropriate band was cut out and extracted with ethyl acetate. Evaporation of the extract afforded the title compound (0.039 g) $\lambda_{max}$(EtOH) 259 nm ($E^{1\%}_{1\ cm}$ 323).

Example 96

p-Nitrobenzyl(2'RS)-2-(2'-chloro-4'-oxoazetidin-1'-yl)-4-methyl-3-methanesulphonyloxypent-2-enoate

(a) Magnesium powder (5.369 g, 0.224 g atom), iodine (2.839 g, 11.2 mmole) and glacial acetic acid (5 ml) were vigorously stirred together in dry tetrahydrofuran (200 ml) for 15 minutes. A solution of the product of Preparation 10 (10.82 g, 22 mmole) and glacial acetic acid (2 ml) in dry tetrahydrofuran (100 ml) was added slowly and the suspension was stirred for 80 minutes and then filtered through cotton wool. The filtrate was evaporated to a foam which was partitioned between dichloromethane (1.5 l.) and 10% aqueous sodium metabisulphite (1 l.). The organic phase was separated and washed successively with water, and brine, and evaporation of the dried organic solution gave p-nitrobenzyl (4'RS)-3-hydroxy 4-methyl-2-(2'-oxo-4'-p-tolylthioazetidin-1'-yl)pent-2-enoate (9.588 g).

(b) A solution of the product of Example 96 (a) (4.463 g, 9.78 mmole) in dry dichloromethane (100 ml) was treated with methanesulphonyl chloride (1.41 ml, 19.5 mmole) followed by triethylamine (2.03 ml, 19.5 mmole). After 40 minutes at 20° the solution was washed successively with N hydrochloric acid, water, and brine, and the organic phase dried and evaporated to give p-nitrobenzyl (4'RS)-4-methyl-3-methanesulphonyloxy-2-

(2'-oxo-4'-p-tolylthioazetidin-1'-yl)pent-2-enoate (4.971 g).

(c) A 5% solution of chlorine in dichloromethane (42 ml, 29.6 mmole of chlorine) was added to a stirred and cooled (ca.0°) solution of the product of Example 96 (b) (4.971 g, 9.3 mmole) in dry dichloromethane (100 ml). After 15 minutes the solution was concentrated (ca. 5 ml) and then subjected to chromatography (B) (0 to 50% ether in dichloromethane). Appropriate fractions were combined and evaporated *in vacuo* to give the title compound (1.121 g). A similar sample had $\lambda_{max}$(EtOH) 261 nm ($E^{1\%}_{1\ cm}$ 285).

Example 97

p-Nitrobenzyl (2'RS)3-methanesulphonyloxy-[2'-chloro-4'-oxoazetidin-1'-yl]hex-2-enoate

A solution of the product of Example 78 (3.060 g) in dry dichloromethane (120 ml) was stirred at 0°; chlorine (16.2 ml of 5% solution in dichloromethane, 11.45 mmole) was added and the solution kept at 0° for 20 minutes. Nitrogen was passed through the solution for 10 minutes to remove the excess of chlorine, and the solvent was evaporated off. The residual oil was purified by chromatography (B) (0 to 2% ethyl acetate in dichloromethane) and appropriate fractions were combined and the solvent removed to give the title compound (0.916 g) $\lambda_{max}$(EtOH) 260 nm ($E^{1\%}_{1cm}$ 160).

Example 98

p-Nitrobenzyl (2'ξ)-2-(2'-chloro-4'-oxoazetidin-1'-yl)-3-methanesulphonyloxybut-2-enoate

The product of Example 79 (8.25 g, 14.2 mmole) in dichloromethane (50 ml) at -15° was stirred and treated with a solution of chlorine in carbon tetrachloride (0.8M, 10 ml, 8 mmole chlorine) added dropwise. More chlorine solution (10 ml each time) was added after ½-hour and 1 hour. After 1 hour the suspension was filtered. The filtrate was evaporated to an oil which was chromatographed (B) (0 to 15% ethyl acetate in toluene). Appropriate fractions were combined and evaporated to small volume. The crystalline product was filtered off, washed with toluene and dried to give the title ester (1.81 g), m.p. 102 to 105°(decomp), $\lambda_{max}$(EtOH) 262 nm ($\varepsilon$ 16,200).

Example 99

p-Nitrobenzyl (2'ξ)-2-(2'-chloro-4'-oxoazetidin-1'-yl)-3-methanesulphonyloxybut-2-enoate

The product of Example 80 (0.92 g, 1.67 mmole) in dichloromethane (20 ml) at -15° was stirred and treated with a solution of chlorine in carbon tetrachloride (1.7 ml, 1.1M, *ca.* 1.8 mmole chlorine). After 1 hour at -15° more chlorine solution (1.7 ml) was added and the solution was allowed to warm to 25° over ½-hour. It was then evaporated to a slurry and ethyl acetate

(20 ml) was added. The suspension was stirred until the solid was uniform and then filtered. The filtrate was concentrated to give a second crop of solid which was also filtered off. The resulting filtrate was evaporated to a gum (750 mg). This material was purified by chromatography (B) (0 to 15% ethyl acetate in toluene) to give the title ester (0.500 g), $[\alpha]_D$-1° (c 1.02, $CHCl_3$).

Example 100

Triethyl- {1-[4-(1-ethoxyethoxy)-2-methanesulphonyloxy-1-(p-nitrobenzyloxycarbonyl)but-1-enyl]-2-oxoazetidin-4-yl} ammonium chloride

Methanesulphonyl chloride (0.15 ml) was added to a stirred solution of 4-(1-ethoxyethoxy)-1-(p-nitrobenzyloxycarbonyl)-1-(2-oxo-4-triethylammonioazetidin-1-yl)but-1-en-2-olate (0.495 g) in dry dichloromethane (9 ml) at 0°. After 15 minutes the reaction mixture was allowed to warm to ambient temperature, stirred for a further 45 minutes, and then added, with stirring, to ether (200 ml). The resulting solid was collected, then washed with ether, and dried in vacuo to afford the title salt (0.542 g), $\nu_{max}$(Nujol) 1798 (β-lactam), 1730 ($CO_2R$), 1375 $cm^{-1}$ ($OSO_2R$).

Example 101

Triethyl-{1-[4-(1-ethoxyethoxy)-1-(p-nitrobenzyloxycarbonyl)-2-(p-toluenesulphonyloxy)but-1-enyl]-2-oxo-azetidin-4-yl} ammonium chloride

p-Toluenesulphonyl chloride (0.223 g) was added to a stirred solution of 4-(1-ethoxyethoxy)-1-(p-nitrobenzyloxycarbonyl)-1-(2-oxo-4-triethylammonioazetidin-1-yl)but-1-en-2-olate (0.495 g) in dichloromethane (9 ml) containing pyridine ( 1 drop) at 0°. After 20 minutes the solution was allowed to warm to room temperature, stirred for a further 45 minutes, and then added, with stirring, to ether (200 ml). The supernatant was decanted from the resulting precipitate and the latter was then agitated with more ether (50 ml). The solid was collected, washed with ether, and dried *in vacuo* to afford the *title salt* (0.564 g), $\nu_{max}(CHBr_3)$ 1800 (β-lactam), 1728 ($CO_2R$), 1360 $cm^{-1}$ ($OSO_2R$).

Example 102

p-Nitrobenzyl 2-(4-chloro-2-oxoazetidin-1-yl)-3-trifluoro-methanesulphonyloxypent-2-enoate

A stirred solution of the product of Example 115 (0.500 g) in dichloromethane (11 ml) at -78° was treated with trifluoromethanesulphonic anhydride (0.25 ml) followed by triethylamine (0.26 ml). The resulting solution was stirred in an ice-bath for 5 minutes, then diluted with ether (100 ml) and washed with 0.5N aqueous

HCl followed by water. Evaporation of the dried organic phase gave the title ester (0.714 g), $\lambda_{max}$(EtOH) 260 nm ($\varepsilon$ 13,800), $\nu_{max}$($CHBr_3$) 1790 $cm^{-1}$ ($\beta$-lactam).

Example 103

p-Nitrobenzyl 2-(4-chloro-2-oxoazetidin-1-yl)-3-trifluoromethanesulphonyloxypent-2-enoate

A solution of chlorine in carbon tetrachloride (1.086M, 3.52 ml) was added dropwise to a stirred solution of the product of Example 81 (0.925 g) in dichloromethane (10 ml) containing acetamide (0.505 g) at -78°. After 17 minutes, the mixture was warmed to ca.0° under a fast stream of nitrogen then filtered and the filtrate concentrated to ca. 3 ml. The resulting slurry was filtered and the filtrate evaporated to an oil (1.147 g). The crude product was re-dissolved in dichloromethane and applied to a dry column of silica gel which was eluted with ethyl acetate: petroleum ether (40-60°) (1:1). Appropiate fractions were combined and evaporated to give the title ester (0.282 g) whose spectroscopic properties were similar to those as described in Example 102.

Example 104

p-Nitrobenzyl (2'RS)-2-(2'-chloro-4'-oxoazetidin-1'-yl)-3-methanesulphonyloxyhept-2-enoate

(a) A solution of the product of Preparation 22 (14.11 g, 0.029 mole) in dry dichloromethane (250 ml) at 0° was treated with thionyl chloride (4.4 ml, 0.061 mole)

followed by pyridine (4.44 ml, 0.055 mole) and the mixture stirred for 30 minutes at 0° to ca 23°. Water (200 ml) was added and the mixture stirred for a further 10 minutes. The organic phase was separated and washed with brine (100 ml) and dried and the solvent evaporated off to give p-nitrobenzyl (2RS,4'RS)-2-chloro-3-oxo-2-(2'-oxo-4'-p-tolylthioazetidin-1'-yl)heptanoate as an orange-brown oil which was used without further purification in the following reaction.

(b) Iodine (3.68 g, 0.015 mole) was added to a stirred and cooled (0°) suspension of magnesium powder (7.06 g, 0.29 g atom) in dry tetrahydrofuran (200 ml). Glacial acetic acid (10 ml) was added, followed 10 minutes later by dropwise addition (over 10 minutes) of a solution of the product from (a) above in dry tetrahydrofuran (50 ml). The mixture was stirred for 30 minutes and allowed to warm to ca 23°. The mixture was filtered through Celite and the filtrate was poured into a stirred mixture of ethyl acetate and aqueous sodium metabisulphite (500 ml of each). The aqueous phase was separated and extracted with ethyl acetate (250 ml) and the organic solutions were combined and washed successively with aqueous sodium bicarbonate and brine (400 ml of each) and the solvent was evaporated off. Dichloromethane (220 ml) was added and the solution dried to give a clear orange solution which was used in the following stage.

(c) The above solution was cooled to ca 0° and treated with methanesulphonyl chloride (3.4 ml, 0.0435 mmole) and then a solution of triethylamine (6.05 ml, 0.0435 mole) in dry dichloromethane (30 ml) was added, with stirring, over 5 minutes. After 40 minutes the mixture was cooled to 0° and more methanesulphonyl chloride (3.4 ml) and triethylamine (6.05 ml) were added. After 20 minutes 2N hydrochloric acid (200 ml) was added and the mixture was stirred for a further 5 minutes. The organic layer was separated and washed with water and brine (200 ml) and dried to give a brown solution containing p-nitrobenzyl (4'RS)-3-methanesulphonyloxy-2-(2'-oxo-4'-p-tolylthioazetidin-1'-yl)-heptenoate.

(d) The above solution was cooled to 0° and treated with chlorine (5% solution of chlorine in dichloromethane, 0.058 mmole, 82 ml). After 40 minutes, nitrogen was passed and the solvent was removed to give a brown oil. This oil was purified by column chromatography (B) (0 to 4% of ether in dichloromethane). Combination of the appropriate fractions gave the title compound (2.850 g) as a brown gum, $\lambda_{max}$(EtOH) 259 nm ($E_{1cm}^{1\%}$ 287), $\nu_{max}$ ($CHBr_3$) 1780 ($\beta$-lactam) and 1722 ($CO_2R$) $cm^{-1}$.

Example 105

p-Nitrobenzyl (2'RS)-2-(2'-chloro-4'-oxoazetidin-1'-yl) 3-methanesulphonyloxyhept-2-enoate

(a) A solution of n-butyl lithium in hexane (1.31 M, 33.7 ml, 43.8 mmole) was added to a stirred and cooled (-70°) solution of hexamethyldisilazane (9.2 ml, 43.8 mmole) in dry tetrahydrofuran (130 ml) under nitrogen. After 3 minutes a solution of the product of Preparation 23 (9.66g) in dry tetrahydrofuran (80ml) was added followed, 2 minutes later, by valeryl chloride (2.6 ml, 22.5 mmole). The reaction mixture was stirred at -78° for 20 minutes then poured into a mixture of ethyl acetate and N hydrochloric acid (750 ml of each) and stirred for a further 5 minutes. The aqueous phase was separated and extracted with ethyl acetate (150 ml) and the combined organic phases were washed with water and brine (200 ml of each) and evaporated to an orange-brown oil. This oil was purified by column chromatography (A) (dichloromethane). Combination of the appropriate fractions gave p-nitrobenzyl (4'RS)-3-hydroxy-2-(2'-oxo-4'-p-tolylthioazetidin-1'-yl) hep-2-enoate (7.29 g), as a pale-orange gum.

(b) A cooled (0°) solution of the above product in dry dichloromethane (100 ml) was successively treated with methanesulphonyl chloride (1.8 ml, 23.25 mmole) and triethylamine (3.2 ml, 23.25 mmole). The reaction mixture was stirred for 20 minutes at 0 to ca 21°; 2N

hydrochloric acid (70 ml) was added and the mixture stirred for a further 5 minutes. The organic phase was separated and washed successively with water and brine (50 ml of each) and dried. The resulting yellow-orange solution was cooled to 0° and treated with chlorine (5% solution in dichloromethane, 31 mmole, 44ml). After stirring for 20 minutes nitrogen was passed and the solvent removed to give an orange gum which was purified by column chromatography (C) (0 to 2% ether in dichloromethane). Combination of the fractions gave the title compound (5.46 g) whose spectroscopic properties were essentially similar to those obtained with the product of Example 104.

Example 106

p-Nitrobenzyl (2'RS)-4-methoxy-3-methanesulphonyloxy-2-(2'-chloro-4'-oxoazetidin-1'-yl)but-2-enoate

A cooled (-5°) solution of chlorine in carbon tetrachloride (1.2 ml, 1.65 mmole of chlorine) was added to a cooled (-10°) and stirred solution of the product of Example 82 (0.608 g, 1.1 mmole) in dry dichloromethane (20 ml). After 24 minutes at -5° to -2° the solution was evaporated to dryness.

The product was dissolved in dichloromethane and the solution was washed with aqueous sodium metabisulphite solution and brine. The washings were repeated and the organic solution was dried (sodium sulphate) and

evaporated to dryness to give the crude product (0.627 g). The product was purified by chromatography (B) (deactivated with 5% water) (dichloromethane: ethyl acetate 20:1). Appropriate fractions were collected and evaporated to dryness.

The product was re-evaporated from dichloromethane to give the title compound (0.31 g). τ ($CDCl_3$) values include 3.90 (dd, J 4 and 2 Hz, 4'-H) and 5.48 (s, $\underline{CH_2}OCH_3$).

Example 107

p-Nitrobenzyl 2-(4-chloro-2-oxoazetidin-1-yl)-3-methanesulphonyloxy-2-enoate

A solution of chlorine in carbon tetrachloride (0.89 M, 2.8 ml) was added dropwise to a stirred solution of the product of Example 84 (0.459 g) in dry dichloromethane (5ml) containing acetamide (0.295 g) at -70°. After 40 minutes a fast stream of nitrogen was passed for 20 minutes through the reaction mixture which was allowed to warm to ambient temperature. The mixture was filtered and the filtrate was evaporated to leave an oil which was fractionated on a silica gel column eluting with ether-petroleum ether (40-60°) (2:1). Appropriate fractions were combined and evaporated to afford the title ester (0.284 g), $\lambda_{max}$ (EtOH) 261.5 nm (ε 13,500), $\nu_{max}$ ($CHBr_3$) 1786 $cm^{-1}$ (β-lactam).

Example 108

p-Nitrobenzyl-2-(4-chloro-2-oxoazetidin-1-yl)-3-methanesulphonyloxypent-2-enoate

Reaction of the product of Example 83 (4.25 g) with a solution of chlorine in carbon tetrachloride (0.90M, 26.6 ml) in the presence of acetamide (2.83 g) by the method as described in Example 107 gave an oil (4.6 g) which was chromatographed on a column of silica gel eluting with ether-petroleum ether (40-60°) (2:1). Appropriate fractions were combined and evaporated to afford the title ester (2.73 g) whose physical and spectroscopic properties resembled those as described in Example 107.

Example 109

p-Nitrobenzyl 2-(4-chloro-2-oxoazetidin-1-yl)-3-methanesulphonyloxypent-2-enoate

Reaction of the product of Example 85 (26.95 g) with a solution of chlorine in carbon tetrachloride (0.90 M, 148 ml) in the presence of acetamide (15.83 g) by the method as described in Example 107 gave an oil which was chromatographed on a column of silica gel eluting with ether-petroleum ether (40-60°) (2:1). Appropriate fractions were combined and evaporated to afford the title ester (15.05 g) whose physical and spectroscopic properties resembled those as described in Example 107.

Example 110

p-Nitrobenzyl 2-(4-chloro-2-oxoazetidin-1-yl)-3-methanesulphonyloxy-5-(tetrahydropyran-2-yloxy)pent-2-enoate

A solution of chlorine in carbon tetrachloride (1.194 M, 12.5 ml) was added dropwise to a stirred solution of the product of Example 86 (3.52 g) in dry dichloromethane (25 ml) containing acetamide (1.77 g) at -70°. After 30 minutes a fast stream of nitrogen was passed for 20 minutes through the reaction mixture which was allowed to warm to room temperature. The mixture was filtered and the filtrate was evaporated to leave an oil which was fractionated on a silica gel column eluting with ether-petroleum ether (40-60°) (2:1) followed by ethyl acetate. Appropriate fractions were combined and evaporated to afford the title ester (0.450 g), $\nu_{max}$($CHBr_3$) 1786 (β-lactam), 1730 ($CO_2R$), 1522 and 1345 $cm^{-1}$ ($NO_2$).

Example 111

p-Nitrobenzyl 3-chloro-2-(4-chloro-2-oxoazetidin-1-yl)pent-2-enoate

A solution of chlorine in carbon tetrachloride (0.79 M, 0.74ml) was added to a stirred solution of the product of Example 88 (0.154 g) in dry dichloromethane (4.5 ml) containing acetamide (0.114 g) at -70°. After 1 hour a fast stream of nitrogen was passed for 10 minutes through the reaction mixture. The mixture was filtered and the filtrate evaporated to leave an oil

containing a little acetamide. The product was re-dissolved in dichloromethane, filtered and then re-evaporated to afford an oil which was fractionated on silica gel plates eluting with ether-petroleum ether (40-60°) (2:1). The major band was isolated and eluted with dichloromethane. Evaporation gave the title ester (0.014 g), $\nu_{max}(CHBr_3)$ 1784 (β-lactam), 1730 ($CO_2R$), 1522 and 1348 $cm^{-1}$ ($NO_2$).

Example 112

p-Nitrobenzyl 2-(4-chloro-2-oxoazetidin-1-yl)-3-methoxypent-2-enoate

A solution of the product of Example 115 (0.07g) in ethyl acetate (5 ml) was treated with an excess of ethereal diazomethane at 0°. After 15 minutes a fast stream of nitrogen was passed for 7 minutes through the solution which was then evaporated to afford the title ester (0.07 g), $\lambda_{max}$(EtOH) 264.5 nm (ε 12,900), $\nu_{max}$ ($CHBr_3$) 1778 $cm^{-1}$ (β-lactam).

Example 113

p-Nitrobenzyl (2'RS)-4-acetoxy-3-methanesulphonyloxy-2-(2'-chloro-4'-oxoazetidin-1'-yl)but-2-enoate

A solution of n-butyllithium in hexane (11.3 ml of a 1.3 molar solution) was added, under nitrogen, to a cooled (-78°) and stirred solution of hexamethyldisilazane (3.07 ml) in dry tetrahydrofuran (80 ml). After 5 minutes a solution of the product of Preparation 23 (3.24 g) in THF (30 ml) was added, dropwise, over

5 minutes. After a further 2 minutes acetoxyacetyl chloride (1.144 g) was added and the solution stirred at -78° for 30 minutes. The reaction solution was poured into a stirred mixture of ethyl acetate and N-HCl (300 ml of each). The organic phase was separated, washed with brine (100 ml) and dried and the solvent evaporated off. The residue was purified by column chromatography (A) (120 g) using a gradient of 1 to 5% ether in dichloromethane as eluant. Combination of the appropriate fractions gave p-nitrobenzyl (4'RS)-4-acetoxy-3-hydroxy-2-(2'-oxo-4'-p-tolylthioazetidin-1'-yl)but-2-enoate (0.83 g) as a gum.

A cooled (0°) solution of this gum (0.83 g) in dry dichloromethane (40 ml) was successively treated with methanesulphonyl chloride (0.27 ml) and triethylamine (0.49 ml). After 20 minutes the solution was washed with 2N-HCl (40 ml) and brine (20 ml) and dried. The cooled (0°) solution was treated with chlorine (5% solution in dichloromethane, 4.9 ml). After 10 minutes the solvent was removed and the residue purified by column chromatography (C) (25 g) using 1% ether in dichloromethane as eluant. Combination of the appropriate fractions gave the title compound (0.34 g), $\lambda_{max}$(EtOH) 261.5 nm ($E^{1\%}_{1cm}$ 295), $\nu_{max}$(CHBr$_3$) 1788 cm$^{-1}$ (β-lactam).

Example 114

p-Nitrobenzyl 2-(4-bromo-2-oxoazetidin-1-yl)-3-methanesulphonyloxypent-2-enoate

A solution of bromine in dichloromethane (1.0M, 1.5 ml) was added over 12 minutes to a stirred solution of the product of Example 85 (0.486 g) in dry dichloromethane (8 ml) containing acetamide (0.236 g) at -78°. After 27 minutes a fast stream of nitrogen was passed for 10 minutes at -50°. The mixture was filtered and the filtrate was evaporated to an oil, which was chromatographed on a dry silica gel column eluting with ether: petroleum ether (40-60°) (4:1). Appropriate fractions were combined and evaporated to afford the title ester (0.108 g) $\nu_{max}(CHBr_3)$ 1780 (β-lactam), 1727 ($CO_2R$), 1520 and 1347 $cm^{-1}$ ($NO_2$).

Example 115

p-Nitrobenzyl 2-(4-chloro-2-oxoazetidin-1-yl)-3-hydroxypent-2-enoate

A solution of chlorine in carbon tetrachloride (0.79 M, 1.2 ml) was added dropwise to a stirred solution of the product of Example 72 (0.230 g) in dry dichloromethane (3 ml) containing acetamide (0.19 g) at -70°. After 30 minutes a fast stream of nitrogen was passed for 5 minutes through the reaction mixture. The mixture was filtered and the filtrate evaporated to an oil containing a little acetamide. The residue was re-dissolved in

dichloromethane and refiltered. Evaporation of the filtrate gave the title ester (0.22 g), $\lambda_{max}$(EtOH) 266 nm ($\varepsilon$ 15,100), $\nu_{max}$($CHBr_3$) 1780 $cm^{-1}$ ($\beta$-lactam).

Example 116

p-Nitrobenzyl 2-(4-chloro-2-oxoazetidin-1-yl)-3-hydroxypent-2-enoate

A solution of chlorine in carbon tetrachloride (0.79M, 0.48 ml) was added dropwise to a stirred solution of the product of Example 73 (0.05 g) in dry dichloromethane (2 ml) containing acetamide (0.04 g) at -78°. After 30 minutes a fast stream of nitrogen was passed for 5 minutes through the reaction mixture which was allowed to warm to room temperature. The mixture was filtered and the filtrate was evaporated to afford the title ester (0.05 g) whose spectral properties resembled those as described in Example 115.

Example 117

p-Nitrobenzyl 2-(4-chloro-2-oxoazetidin-1-yl)-3-hydroxypent-2-enoate

Reaction of the product of Example 71 (0.055 g) with a solution of chlorine in carbon tetrachloride (0.79M, 0.29 ml) in the presence of acetamide (0.045 g) by the method as described in Example 116 gave the title ester (0.05 g) whose spectroscopic properties resembled those as described in Example 115.

Example 118

p-Nitrobenzyl 2-(4-chloro-2-oxoazetidin-1-yl)-3-hydroxpent-2-enoate

A dry solution of chlorine in carbon tetrachloride (0.79 M, 0.38 ml) was added dropwise to a stirred solution of the product of Example 71 (0.05 g) in dry dichloromethane (0.7 ml) containing molecular sieves (Type 4A; 0.50 g) at -78°. After 15 minutes a fast stream of nitrogen was passed for 2 minutes through the reaction mixture which was allowed to warm to room temperature. The mixture was filtered and the filtrate was evaporated to afford the title ester (0.05 g) whose spectral properties resembled those as described in Example 115.

Example 119

p-Nitrobenzyl 2- (4-chloro-2-oxoazetidin-1-yl)-3-hydroxy-pent-2-enoate

N-Chlorosuccinimide (0.04 g) was added in portions to a stirred solution of the product of Example 72 (0.06 g) in dry dichloromethane (8ml)at room temperature. After 4 hours the mixture was filtered. The filtrate was diluted with dichloromethane (50 ml) then washed with water (15 ml) followed by saturated brine (15 ml), and dried over magnesium sulphate. Evaporation afforded the title ester (0.05 g) whose spectral properties resembled those as described in Example 115.

Example 120

4-(tert-Butyldimethylsilyloxy)-1-(p-nitrobenzyloxycarbonyl)-1-(2-oxo-4-triethylammonioazetidin-1-yl)but-1-en-2-thiolate

Methanesulphonyl chloride (1.28 ml) was added dropwise to a stirred solution of 4-(tert-butyldimethylsilyloxy)-1-(p-nitrobenzyloxycarbonyl)-1-(2-oxo-4-triethylammonioazetidin-1-yl)but-1-en-2-olate(3.02 g) in dry dichloromethane (20 ml) containing dry pyridine (3.8 ml) and maintained at 5°. After 10 minutes the solution was allowed to warm to room temperature and stirred for 5 hours. The solution was cooled to 5° and hydrogen sulphide was passed through the reaction mixture under nitrogen. After 10 minutes the mixture was allowed to warm to ambient temperature and hydrogen sulphide was passed for a further 45 minutes. The resulting mixture was filtered and the filtrate was diluted with dichloromethane and washed with saturated brine containing 0.5N aqueous $NaHCO_3$, followed by saturated brine. The dried organic phase was evaporated to low volume, and then poured into rapidly stirred diethyl ether. The supernatant was decanted and the residual material was stirred with diethyl ether to give a solid which was collected and dried in vacuo to afford the title salt (0.33 g), $\nu_{max}$(Nujol) 1778 (β-lactam), 1660 ($CO_2R$), 1520 and 1344 $cm^{-1}$ ($NO_2$).

Example 121

1-(p-Nitrobenzyloxycarbonyl)-1-(2-oxo-4-triethylammonioazetidin-1-yl)but-1-en-2-thiolate

Methanesulphonyl chloride (0.2 ml) was added to a stirred solution of 1-(p-nitrobenzyloxycarbonyl)-1-(2-oxo-4-triethylammonioazetidin-1-yl)but-1-en-2-olate (0.42 g) in dry dichloromethane (2 ml) containing dry pyridine (0.4 ml) at 5°. After 10 minutes the reaction mixture was allowed to warm to room temperature and treated with 15-crown-5, (0.22 g) followed by sodium sulphide (0.39g, dried over $P_2O_5$). After 15 minutes the mixture was diluted with dichloromethane and washed with saturated brine containing 0.5N aqueous $NaHCO_3$ followed by saturated brine and then dried. The solution was evaporated to low volume and then added slowly to rapidly stirred diethyl ether (100 ml). The resulting solid was collected and dried in vacuo to afford the title salt (0.195 g), $\nu_{max}$ (Nujol) 1780 (β-lactam), 1720 and 1666 ($CO_2R$), 1516 and 1344 $cm^{-1}$ ($NO_2$).

Example 122

1-(p-Nitrobenzyloxycarbonyl)-1-(2-oxo-4-triethylammonioazetidin-1-yl)but-1-en-2-thiolate

A stirred, ice-cooled solution of 1-(p-nitrobenzyloxycarbonyl)-1-(2-oxo-4-triethylammonioazetidin-1-yl)but-1-en-2-olate (0.1 g) in dichloromethane (10 ml) was treated with pyridine (0.1 ml) followed by methanesulphonyl chloride (0.06 ml). After 30 minutes hydrogen sulphide was passed through the stirred solution and triethylamine (0.06 ml) was added. After 15 minutes the solution was treated with an excess of triethylamine and then diluted with ether. The resulting crystals were collected, then washed with ether, and dried *in vacuo* to afford the title salt (0.115 g), whose spectroscopic properties resembled those as described in Example 121.

Example 123

1-(p-Nitrobenzyloxycarbonyl)-1-(2-oxo-4-triethylammonioazetidin-1-yl)-4-(tetrahydropyran-2-yloxy)but-1-en-2-thiolate

Methanesulphonyl chloride (2.35 ml) was added in portions to a stirred solution of 1-(p-nitrobenzyloxycarbonyl)-1-(2-oxo-4-triethylammonioazetidin-1-yl)-4-(tetrahydropyran-2-yloxy)but-1-en-2-olate (5.2 g) in dry dichloromethane (30 ml) containing pyridine (4.0 ml) at 0°. The reaction mixture was allowed to warm to room temperature and after 3 hours hydrogen sulphide was passed

through the stirred solution at 0°. The reaction mixture was allowed to warm to ambient temperature and after 30 minutes was filtered. The filtrate was washed with water followed by saturated brine and then dried. The filtered solution was diluted with ether and the resulting oil was triturated with ether to give a solid which was collected and dried *in vacuo* to afford the *title salt* (0.40 g) $\nu_{max}$(Nujol) 1788 (β-lactam), 1720 and 1670 ($CO_2R$), 1520 and 1344 $cm^{-1}$ ($NO_2$).

Example 124

1-(p-Nitrobenzyloxycarbonyl)-1-(2-oxo-4-triethylammonioazetidin-1-yl)but-1-en-2-thiolate

Methanesulphonyl chloride (0.70 ml) was added dropwise to a stirred solution of 1-(p-nitrobenzyloxycarbonyl)-1-(2-oxo-4-triethylammonioazetidin-1-yl)but-1-en-2-olate(1.257g) in dry dichloromethane(10ml) containing pyridine (1.21 ml) at 0°. The reaction mixture was allowed to warm to room temperature and after 2.5 hours hydrogen sulphide was passed through the stirred solution at 0°. The reaction mixture was allowed to warm to ambient temperature and hydrogen sulphide was passed through the mixture for a further 10 minutes. The mixture was filtered and the filtrate was poured into a mixture of dichloromethane (100 ml) and water (50 ml). The organic phase was further washed with water followed by saturated brine and then dried. The filtered solution was evaporated to low volume (*ca* 20 ml) and then diluted

with ether. The precipitated solid was collected and dried in vacuo to afford the title salt (0.31 g), whose spectroscopic properties resembled those as described in Example 121.

Example 125

4-Methoxy-1-(p-nitrobenzyloxycarbonyl)-1-(2-oxo-4-triethylammonioazetidin-1-yl)but-1-en-2-thiolate

Methanesulphonyl chloride (6.72 ml) was added dropwise to a stirred solution of 4-methoxy-1-(p-nitrobenzyloxycarbonyl)-1-(2-oxo-4-triethylammonioazetidin-1-yl)but-1-en-2-olate (13.0g) in dry dichloromethane (73ml) containing pyridine (11.7ml)at 0°. After 10 minutes the reaction mixture was allowed to warm to room temperature and after a further 3 hours hydrogen sulphide was passed through the stirred solution at 0°. After 20 minutes the reaction mixture was allowed to warm to ambient temperature and hydrogen sulphide was passed for a further 30 minutes. The filtered organic solution was washed with saturated brine, then dried, and evaporated to ca 50ml. Dilution with ether gave an oil which, on trituration with ether, gave a solid which was collected and dried in vacuo to afford the title salt (6.39 g), $\nu_{max}$ (Nujol) 1770 (β-lactam), 1710 and 1670 ($CO_2R$), 1520 and 1342 $cm^{-1}$ ($NO_2$).

Example 126

4-(1-Ethoxyethoxy)-1-(p-nitrobenzyloxycarbonyl)-1-(2-oxo-4-triethylammonioazetidin-1-yl)but-1-en-2-thiolate

Methanesulphonyl chloride (3.83 ml) was added to a stirred solution of 4-(1-ethoxyethoxy)-1-(p-nitrobenzyloxycarbonyl)-1-(2-oxo-4-triethylammonioazetidin-1-yl)but-1-en-2-olate (8.38 g) in dry dichloromethane (110 ml) containing dry pyridine (6.65 ml) at 5°. After 5 minutes the solution was allowed to warm to room temperature and after a further 18 hours hydrogen sulphide was passed through the stirred solution at 0° under nitrogen. After 5 minutes the reaction mixture was allowed to warm to ambient temperature and hydrogen sulphide was passed for a further 15 minutes. The filtered organic solution was washed with saturated brine (2 x 50 ml), then dried and concentrated to ca 10ml. Dilution with ether gave an oil which, on trituration with ether, afforded a solid which was collected and dried in vacuo to give the title salt (5.63 g) $\nu_{max}$ (Nujol) 1780 (β-lactam) 1705 and 1665 ($CO_2R$), 1520 and 1345 $cm^{-1}$ ($NO_2$).

Example 127

Potassium (5RS)-2-(2-hydroxyethyl)pen-2-em-3-carboxylate

A solution of sodium sulphide (0.48 g) in water (10ml) was added to a stirred, ice-cooled solution of the product of Example 8 (0.700g) in THF (24ml) and water (12ml). After 25 minutes 2N aqueous HCl (1ml) was added and the THF was evaporated off. The aqueous solution was saturated with ammonium sulphate and extracted with ethyl acetate (x 3). The aqueous phase was acidified with 2N aqueous HCl and re-extracted with ethyl acetate (x3). These organic extracts were dried, then concentrated to 5 ml, and treated with a solution of potassium 2-ethylhexanoate in ethyl acetate (0.69M, 1 ml). The resulting precipitate was collected, then washed with ethyl acetate followed by ether, and dried *in vacuo* to give a solid (0.16g), whose spectroscopic properties resembled those of the *title salt* as described in Example 11.

The following Examples illustrate how compounds according to the invention maybe formulated into pharmaceutical compositions.

Example A

Formula per tablet

| | |
|---|---|
| Densified potassium (5RS)-2-ethylpen-2-em-3-carboxylate containing 1% magnesium stearate | 302.0 mg |
| Empicol LZ | 4.5 mg |
| Explotab | 9.0 mg |
| Avicel PH 101 to tablet core weight of | 450.0 mg |

Method of Preparation

Blend the antibiotic with magnesium stearate and compress into slugs on a heavy duty tableting machine. Break down the slugs through a series of screens (10, 12, 16 and 20 mesh) on a rotary granulator to produce free-flowing granules. Blend the granules with the rest of the excipients and compress the blend on a tablet machine using normal or deep concave punches (9 - 12 mm diameter).

Film coat the tablet cores using hydroxypropylmethyl cellulose or similar film forming material using either an aqueous or non-aqueous solvent system. A plasticizer and suitable colour may be included in the film coating solution.

Example B

Formula per tablet

| | |
|---|---|
| Potassium (5RS)-2-ethylpen-2-em-3-carboxylate | 299.0 mg |

| | |
|---|---|
| Maize starch | 22.5 mg |
| Explotab | 9.0 mg |
| Empicol LZ | 4.5 mg |
| Magnesium stearate | 4.5 mg |
| Avicel PH 101 to tablet core weight of | 450.0 mg |

Method of Preparation

Add sufficient quantity of a 10% starch paste to the antibiotic to produce a suitable wet mass for granulation. Prepare the granules and dry using a tray or fluid bed dryer. Sift through a sieve (10 or 12 mesh), add the remaining ingredients and compress into tablets as described in Example A.

The tablet cores may be film coated as described in Example A.

Example C

Formula per capsule

| | |
|---|---|
| Densified potassium (5RS)-2-ethylpen-2-em-3-carboxylate containing 1% magnesium stearate | 302.0 mg |
| Explotab | 6.0 mg |
| Aerosil 200 | 2.0 mg |

Method of Preparation

Densify and prepare granules of the antibiotic as described in Example A, blend the granules and excipients and fill the blend into appropriate size hard gelatin capsules (lock fitting type) on an automatic capsule filling machine.

Example D

Dry Powder for Injection

Fill sterile potassium (5RS)-2-(2-carbamoyloxyethyl)-pen-2-em-3-carboxylate aseptically into glass vials under a blanket of sterile nitrogen such that each vial contains an amount equivalent to 500 mg of the antibiotic free acid. Close the vials using rubber discs or plugs held in position by aluminium sealing rings, thereby preventing gaseous exchange or ingress of microorganisms. Constitute the product by dissolving in Water for Injections shortly before administration.

Empicol LZ is sodium lauryl sulphate available from Marchon Ltd.; Explotab is sodium starch glycolate available from Greeff Fine Chemicals Ltd., Croydon, Surrey, England; Avicel PH 101 is microcrystalline cellulose available from FMC Corporation, U.S.A.; Emcompress is dicalcium phosphate dihydrate NF; and Aerosil 200 is finely divided silicon dioxide.

Results of Biological Tests

Biological testing of a number of compounds of the invention has provided the results shown below;- the test compounds are identified by their Example Numbers:

1. Determination of Minimum Inhibitory Concentrations (MIC)

Serial two-fold dilutions of freshly prepared test solutions were made into Oxoid No. 1 Nutrient agar with or without added enrichment and poured into petri diches. Plates were inoculated with a multipoint inoculator with inoculum containing approximately $10^5$ colony forming units of the organisms shown in the following Tables, all of which organisms are clinical isolates. The MIC, quoted in the Tables as µg/ml, was read after 18 hours incubation at 37°C as the lowest concentration which inhibited growth. N.T. means not tested.

| Organism \ Example No: | | 11 | 13 | 18 | 21 | 52 | 57 |
|---|---|---|---|---|---|---|---|
| Staph.aureus | 853E | 0.5 | 0.5 | <0.1 | 0.2 | 0.5 | 0.5 |
| Micrococcus Sp | 1810E | <0.1 | 0.2 | <0.1 | <0.1 | 0.2 | 0.2 |
| E. coli | 851E | 4 | 4 | 2 | 4 | 4 | 8 |
| E. cloacae | 1051E | 8 | 16 | 8 | 31 | 8 | 16 |
| E. cloacae | 1321E | 4 | 2 | 2 | 4 | 2 | 8 |
| K. aerogenes | 1522E | 8 | 8 | 4 | 4 | 4 | 8 |
| S. marcescens | 1324E | 8 | 62 | 4 | 4 | 4 | 8 |
| H. influenzae | 1184E | 8 | N.T | 2 | 4 | 4 | 4 |

| Organism \ Example No: | 36 | 45 | 28 | 26 | 6 |
|---|---|---|---|---|---|
| Staph.aureus 853E | 0.5 | 0.5 | <0.1 | 0.5 | 1 |
| Micrococcus Sp 1810E | 0.1 | <0.1 | <0.1 | N.T | 0.2 |
| E. coli 851E | 8 | 2 | 0.5 | 31 | 8 |
| E. cloacae 1051E | 8 | 8 | 4 | 125 | 16 |
| E. cloacae 1321E | 8 | 2 | 0.5 | 31 | 8 |
| K. aerogenes 1522E | 8 | 4 | 0.5 | 31 | 16 |
| S. marcescens 1324E | 8 | 8 | 1 | 31 | 8 |
| H. influenzae 1184E | 2 | 4 | 1 | 4 | 4 |

2. Toxicity

The compounds of Example 36 and 43 when administered intraperitoneally to mice gave $LD_{50}$ values of greater than 500 mg/kg.

Claims:

1. Compounds of the general formula

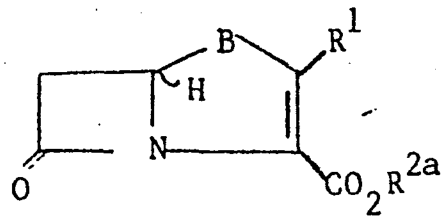

I

(wherein $R^1$ represents an alkyl group containing from 1 to 8 carbon atoms, optionally substituted by a hydroxy, etherified hydroxy or acylated hydroxy group; $R^{2a}$ represents a hydrogen atom or a carboxyl esterifying group; and B represents a sulphur atom or a group $>S \rightarrow O$) and salts thereof.

2. Compounds as claimed in claim 1 wherein $R^1$ represents a group $-CH_2CH_2R^o$ where $R^o$ represents a hydrogen atom or a hydroxy , etherified hydroxy or acylated hydroxy group.

3. Compounds as claimed in claim 1 or claim 2 wherein B represents a sulphur atom.

4. Compounds as claimed in claim 3 wherein $R^1$ represents an ethyl, n-propyl, 2-carbamoyloxyethyl, methoxymethyl or 2-methoxyethyl group.

5. A process for the preparation of compounds of formula I as defined in claim 1 which comprises reacting a compound of formula

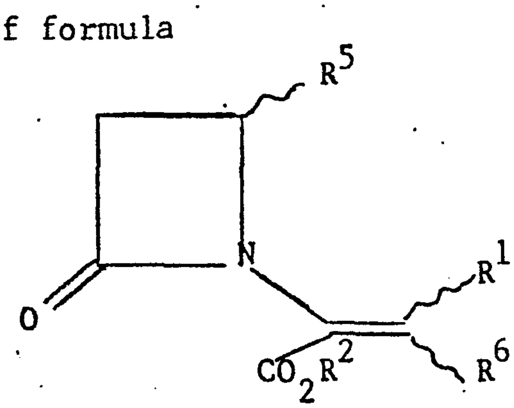

II

[wherein $R^1$ is as defined in claim 1; $R^2$ represents a carboxyl esterifying group; $R^5$ represents a halogen atom or a group of formula

$$-\overset{\oplus}{N}R^xR^yR^z\ A^{\ominus}$$

(in which $R^x$, $R^y$ and $R^z$, which may be the same or different, each represents an aliphatic, araliphatic or aromatic group; or two of $R^x$, $R^y$ and $R^z$ together with the nitrogen atom to which they are attached represent a five-, six- or seven-membered heterocyclic ring optionally containing a further hetero atom and the third of $R^x$, $R^y$ and $R^z$ is an aliphatic, araliphatic or aromatic group; or $R^x$, $R^y$ and $R^z$ together with the nitrogen atom to which they are a attached form a polycyclic heterocyclic ring system or an aromatic heterocyclic ring: and $A^{\ominus}$ is an anion) and $R^6$ represents a leaving group] with hydrogen sulphide or with a hydrosulphide or sulphide salt: if desired, subsequently converting a compound of formula I wherein B represents a sulphur atom initially obtained into a compound of formula I wherein B represents a group $>S\rightarrow O$; and/or if desired, converting a compound of formula I wherein $R^{2a}$ represents a carboxyl esterifying group into a compound of formula I wherein $R^{2a}$ represents a hydrogen atom; and/or if desired converting an appropriate compound of formula I into a salt thereof; and/or if desired converting a compound of formula I in which $R^2$ represents a hydrogen atom, or a salt thereof, into a corresponding ester; and/or if desired modifying the group $R^1$ in the compound obtained.

6. A process as claimed in claim 5 wherein the reaction of the compound of formula II with hydrogen sulphide or

with a hydrosulphide or sulphide salt is effected in the presence of a base.

7. Pharmaceutical compositions comprising a compound of formula I as defined in claim 3 in which $R^{2a}$ represents a hydrogen atom or a metabolically labile carboxyl esterifying group or a salt of such a compound of formula I in association with a pharmaceutical carrier or excipient.

8. Compounds of the general formula

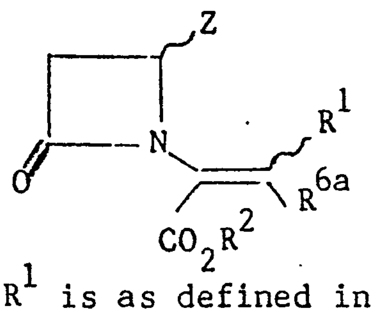

XXII

wherein $R^1$ is as defined in claim 1; $R^2$ represents a carboxyl esterifying group; and either Z represents a halogen atom or a group $-(S)_nR^9$ (in which either n is 0 and $R^9$ represents a group $-SR^{10}$,

$$-\overset{O}{\overset{\uparrow}{S}}-R^{10} \quad \text{or} \quad -\underset{O}{\underset{\downarrow}{\overset{O}{\overset{\uparrow}{S}}}}-R^{10}$$

where $R^{10}$ is an aliphatic, araliphatic, aromatic or heterocyclic group; or n is 1 and $R^9$ represents an acyl group or a group $-SR^{10}$ where $R^{10}$ is as defined above) and $R^{6a}$ represents a hydroxyl group or a leaving group;

or Z represents a group of formula

$$-\overset{\oplus}{N}R^xR^yR^zA^{\ominus}$$

(where $R^x$, $R^y$, $R^z$ and $A^{\ominus}$ are as defined in claim 5) and $R^{6a}$ represents a leaving group;

or Z represents a group $\overset{\oplus}{N}R^xR^yR^z$ (where $R^x$, $R^y$, and $R^z$ are as defined in claim 5) and $R^{6a}$ represents $S^{\ominus}$:

with the proviso that when Z represents a methylthio group and $R^{6a}$ represents a free hydroxy group, $R^1$ does not represent a group $-CHR^{14}R^{15}$ where $R^{14}$ represents a hydrogen atom or an optionally substituted alkyl group and $R^{15}$ represents a hydroxymethyl group or a 1-hydroxyethyl group; and with the further proviso that when Z represents a group $-(S)_nR^9$ in which n is 1, $R^1$ represents a methyl group.

European Patent Office

# EUROPEAN SEARCH REPORT

Application number

| Category | Citation of document with indication, where appropriate, of relevant passages | Relevant to claim |
|---|---|---|
| A | DE - A - 2 655 298 (CIBA-GEIGY)<br>* Claims *<br>& FR - A - 2 342 730 (30 september 1977)<br>& BE - A - 849 118<br>& NL - A - 76 13663 | 1,5-8 |
| A | GB - A - 1 368 074 (BEECHAM)<br>* Claims *<br>& DE - A - 2 205 123<br>& FR - A - 2 124 251<br>& NL - A - 72 01386 | 8 |
| P | DE - A - 2 740 527 (GLAXO)<br>* Claims *<br>& FR - A - 2 364 205<br>& BE - A - 858 516<br>& NL - A - 77 09875 | 8 |

BAD ORIGINAL

CLASSIFICATION OF THE APPLICATION (Int. Cl.)

C 07 D 499/00<br>
C 07 F [illegible]<br>
C 07 D 417/1[illegible]<br>
A 61 K 31/4[illegible]<br>
// C 07 F [illegible]<br>
C 07 C [illegible]<br>
C 07 F 7/10<br>
C 07 C [illegible]/11

TECHNICAL FIELDS SEARCHED (Int.Cl.²)

C 07 D 499/00<br>
C 07 D 205/08<br>
C 07 D 417/12<br>
C 07 F 9/65<br>
A 61 K 31/43

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| | | |